# EUROPEAN PATENT SPECIFICATION

(11) **EP 1 628 989 B1**
(45) Date of publication and mention of the grant of the patent: **20.12.2006**
(21) Application number: 04732108.8
(22) Date of filing: 11.05.2004
(51) Int. Cl.: C07H 17/08, A61K 31/7048

(54) **NOVEL 14 AND 15 MEMBERED-RING COMPOUNDS**
NEUE 14- UND 15-GLIEDRIGE RINGVERBINDUNGEN
NOUVEAUX COMPOSES A CYCLE RAMIFIE EN 14 ET 15

(30) Priority: 13.05.2003 GB 0310962; 31.03.2004 GB 0407391
(43) Date of publication of application: 01.03.2006
(73) Proprietor: GLAXO GROUP LIMITED, Greenford, Middlesex UB6 ONN (GB); Pliva - Istrazivacki Institut d.o.o., 10000 Zagreb (HR)
(72) Inventor: ALIHODZIC, Sulejman, Pliva Istrazivacki Inst. doo, 10000 Zagreb (HR); BERGE, John Michael, GlaxoSmithKline, Harlow, Essex CM19 5AW (GB); JARVEST, Richard Lewis, GlaxoSmithKline, Stevenage, Hertfordshire SG1 2NY (GB)
(74) Representative: Hambleton, Bernadette Angelina
(86) International application number: PCT/EP2004/005083
(87) International publication number: WO 2004/101587

(56) References cited:
- EP-A- 0 895 999
- WO-A-03/042228
- WO-A-20/04039822
- US-B1- 6 300 316

## Description

The present invention relates to novel semi-synthetic macrolides having antimicrobial activity, in particular antibacterial activity. More particularly, the invention relates to 14-and 15-membered macrolides substituted at the 4" position, to processes for their preparation, to compositions containing them and to their use in medicine.

Macrolide antibacterial agents are known to be useful in the treatment or prevention of bacterial infections. However, the emergence of macrolide-resistant bacterial strains has resulted in the need to develop new macrolide compounds. For example, EP 0 895 999 and US 6,300, 316 describe derivatives modified at the 4" position of the macrolide ring having antibacterial activity.

According to the present invention, we have now found novel 14- and 15-membered macrolides substituted at the 4" position which also have antimicrobial activity.

Thus, the present invention provides compounds of general formula (I) 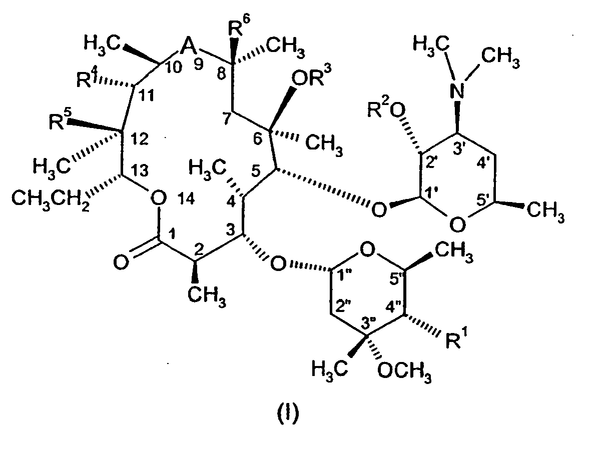 wherein
A is a bivalent radical selected from -C(O)-, -C(O)NH-, -NHC(O)-, -N(R⁷)-CH₂-, -CH₂-N(R⁷)-, -CH(NR⁸R⁹)- and -C(=NR¹⁰)-;
R¹ is -OC(O)(CH₂)_{d}XR¹¹;
R² is hydrogen or a hydroxyl protecting group;
R³ is hydrogen, C₁₋₄alkyl, or C₃₋₆alkenyl optionally substituted by 9 to 10 membered fused bicyclic heteroaryl;
R⁴ is hydroxy, C₃₋₆alkenyloxy optionally substituted by 9 to 10 membered fused bicyclic heteroaryl, or C₁₋₆alkoxy optionally substituted by C₁₋₆alkoxy or -O(CH₂)ₑNR⁷R¹², R⁵ is hydroxy, or
R⁴ and R⁵ taken together with the intervening atoms form a cyclic group having the following structure: 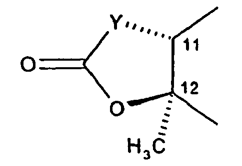 wherein Y is a bivalent radical selected from -CH₂-, -CH(CN)-, -O-, -N(R¹³)- and-CH(SR¹³)-;
R⁶ is hydrogen or fluorine;
R⁷ is hydrogen or C₁₋₆alkyl;
R⁸ and R⁹ are each independently hydrogen, C₁₋₆alkyl, -C(=NR¹⁰)NR¹⁴R¹⁵ or-C(O)R¹⁴, or
R⁸ and R⁹ together form =CH(CR¹⁴R¹⁵R¹⁵)_{f}aryl, =CH(CR¹⁴R¹⁵)_{f}heterocyclyl, =CR¹⁴R¹⁵ or =C(R¹⁴)C(O)OR¹⁴, wherein the alkyl, aryl and heterocyclyl groups are optionally substituted by up to three groups independently selected from R¹⁶;
R¹⁰ is -OR¹⁷, C₁₋₆alkyl, -(CH₂)_{g}aryl, -(CH₂)_{g}heterocyclyl or -(CH₂)ₕO(CH₂)ᵢOR⁷, wherein each R¹⁰ group is optionally substituted by up to three groups independently selected from R¹⁶;
R¹¹ is a heterocyclic group having the following structure: 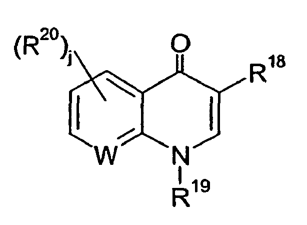 or 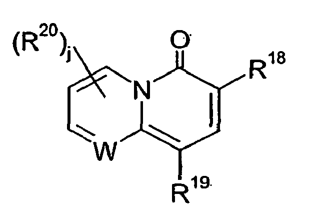 R¹² is hydrogen or C₁₋₆alkyl;
R¹³ is hydrogen or C₁₋₄alkyl optionally substituted by a group selected from optionally substituted phenyl, optionally substituted 5 or 6 membered heteroaryl and optionally substituted 9 to 10 membered fused bicyclic heteroaryl;
R¹⁴ and R¹⁵ are each independently hydrogen or C₁₋₆alkyl;
R¹⁶ is halogen, cyano, nitro, trifluoromethyl, azido, -C(O)R²¹, -C(O)OR²¹, -OC(O)R²¹,-OC(O)OR²¹, -NR²²C(O)R²³, -C(O)NR²²R²³, -NR²²R²³, hydroxy, C₁₋₆alkyl, -S(O)ₖC₁₋₆alkyl, C₁₋₆alkoxy, -(CH₂)ₘaryl or -(CH₂)ₘheteroaryl, wherein the alkoxy group is optionally substituted by up to three groups independently selected from -NR¹⁴R¹⁵, halogen and -OR¹⁴, and the aryl and heteroaryl groups are optionally substituted by up to five groups independently selected from halogen, cyano, nitro, trifluoromethyl, azido,-C(O)R²⁴, -C(O)OR²⁴, -OC(O)OR²⁴, -NR²⁵C(O)R²⁶, -C(O)NR²⁵R²⁶, -NR²⁵R²⁶, hydroxy, C₁₋₆alkyl and C₁₋₆alkoxy;
R¹⁷ is hydrogen, C₁₋₆alkyl, C₃₋₇cycloalkyl, C₃₋₆alkenyl or a 5 or 6 membered heterocyclic group, wherein the alkyl, cycloalkyl, alkenyl and heterocyclic groups are optionally substituted by up to three substituents independently selected from optionally substituted 5 or 6 membered heterocyclic group, optionally substituted 5 or 6 membered heteroaryl, -OR²⁷, -S(O)ₙR²⁷, -NR²⁷R²⁸, -CONR²⁷R²⁸, halogen and cyano;
R¹⁸ is hydrogen, -C(O)OR²⁹, -C(O)NHR²⁹, -C(O)CH₂NO₂ or -C(O)CH₂SO₂R⁷;
R¹⁹ is hydrogen, C₁₋₄alkyl optionally substituted by hydroxy or C₁₋₄alkoxy, C₃₋₇cycloalkyl, or optionally substituted phenyl or benzyl;
R²⁰ is halogen, C₁₋₄alkyl, C₁₋₄thioalkyl, C₁₋₄alkoxy, -NH₂, -NH(C₁₋₄alkyl) or -N(C₁₋₄alkyl)₂;
R²¹ is hydrogen, C₁₋₁₀alkyl, -(CH₂)paryl or -(CH₂)ₚheteroaryl;
R²² and R²³ are each independently hydrogen, -OR¹⁴, C₁₋₆alkyl, -(CH₂)_{q}aryl or-(CH₂)_{q}heterocyclyl;
R²⁴ is hydrogen, C₁₋₁₀alkyl, -(CH₂)ᵣaryl or -(CH₂)ᵣheteroaryl;
R²⁵ and R²⁶ are each independently hydrogen, -OR¹⁴, C₁₋₆alkyl, -(CH₂)ₛaryl or-(CH₂)ₛheterocyclyl;
R²⁷ and R²⁸ are each independently hydrogen, C₁₋₄alkyl or C₁₋₄alkoxyC₁₋₄alkyl;
R²⁹ is hydrogen,
C₁₋₆alkyl optionally substituted by up to three groups independently selected from halogen, cyano, C₁₋₄alkoxy optionally substituted by phenyl or C₁₋₄alkoxy,-C(O)C₁₋₆alkyl, -C(O)OC₁₋₆alkyl, -OC(O)C₁₋₆alkyl, -OC(O)OC₁₋₆alkyl,-C(O)NR³²R³³, -NR³²R³³ and phenyl optionally substituted by nitro or -C(O)OC₁₋₆alkyl,
-(CH₂)_{w}C₃₋₇cycloalkyl,
-(CH₂)_{w}heterocyclyl,
-(CH₂)_{w}heteroaryl,
-(CH₂)_{w}aryl,
C₃₋₆alkenyl, or
C₃₋₆alkynyl;
R³⁰ is hydrogen, C₁₋₄alkyl, C₃₋₇cycloalkyl, optionally substituted phenyl or benzyl, acetyl or benzoyl;
R³¹ is hydrogen or R²⁰, or R³¹ and R¹⁹ are linked to form the bivalent radical -O(CH₂)₂- or -(CH2)t-;
R³² and R³³ are each independently hydrogen or C₁₋₆alkyl optionally substituted by phenyl or -C(O)OC₁₋₆alkyl, or
R³² and R³³, together with the nitrogen atom to which they are bound, form a 5 or 6 membered heterocyclic group optionally containing one additional heteroatom selected from oxygen, nitrogen and sulfur;
X is -U(CH₂)ᵥ-;
U is a divalent radical selected from -N(R³⁰)-, -O-, -S(O)_{z}-, -N(R³⁰)C(O)-, -C(O)N(R³⁰)- and -N[C(O)R³⁰]-;
W is -C(R³¹)- or a nitrogen atom;
d is an integer from 1 to 5;
e is an integer from 2 to 4;
f, g, h, m, p, q, r, s and w are each independently integers from 0 to 4;
i is an integer from 1 to 6;
j, k, n and z are each independently integers from 0 to 2;
t is 2 or 3;
v is an integer from 1 to 8;
and pharmaceutically acceptable derivatives thereof.

According to another embodiment the present invention provides compounds of general formula (IA): 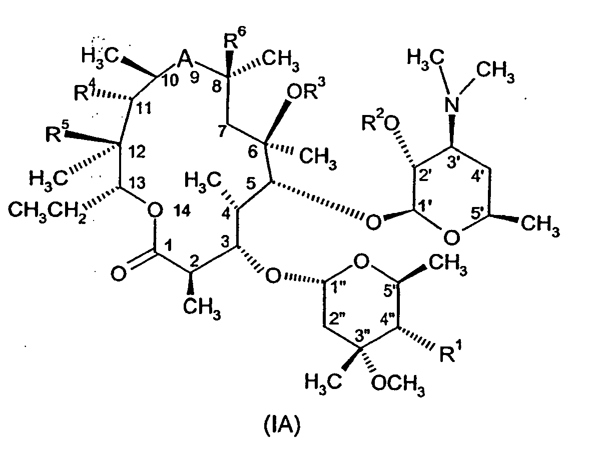 wherein
A is a bivalent radical selected from -C(O)-, -C(O)NH-, -NHC(O)-, -N(R⁷)-CH₂-, -CH₂-N(R⁷)-, -CH(NR⁸R⁹)- and -C(=NR¹⁰)-;
R¹ is -OC(O)(CH₂)_{d}XR¹¹;
R² is hydrogen or a hydroxyl protecting group;
R³ is hydrogen, C₁₋₄alkyl, or C₃₋₆alkenyl optionally substituted by 9 to 10 membered fused bicyclic heteroaryl;
R⁴ is hydroxy, C₃₋₆alkenyloxy optionally substituted by 9 to 10 membered fused bicyclic heteroaryl, or C₁₋₆alkoxy optionally substituted by C₁₋₆alkoxy or -O(CH₂)ₑNR⁷R¹²,
R⁵ is hydroxy, or
R⁴ and R⁵ taken together with the intervening atoms form a cyclic group having the following structure: 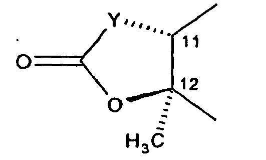 wherein Y is a bivalent radical selected from -CH₂-, -CH(CN)-, -O-, -N(R¹³)- and-CH(SR¹³)-;
R⁶ is hydrogen or fluorine;
R⁷ is hydrogen or C₁₋₆alkyl;
R⁸ and R⁹ are each independently hydrogen, C₁₋₆alkyl, -C(=NR¹⁰)NR¹⁴R¹⁵ or-C(O)R¹⁴, or
R⁸ and R⁹ together form =CH(CR¹⁴R¹⁵)_{f}aryl, =CH(CR¹⁴R¹⁵)_{f}heterocyclyl, =CR¹⁴R¹⁵ or =C(R¹⁴)C(O)OR¹⁴, wherein the alkyl, aryl and heterocyclyl groups are optionally substituted by up to three groups independently selected from R¹⁶;
R¹⁰ is -OR¹⁷, C₁₋₆alkyl, -(CH₂)garyl, -(CH₂)gheterocyclyl or -(CH₂)ₕO(CH₂)ᵢOR⁷, wherein each R¹⁰ group is optionally substituted by up to three groups independently selected from R¹⁶;
R¹¹ is a heterocyclic group having the following structure: 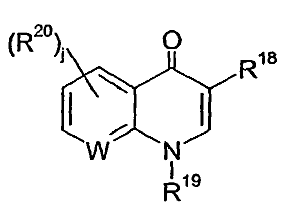 or 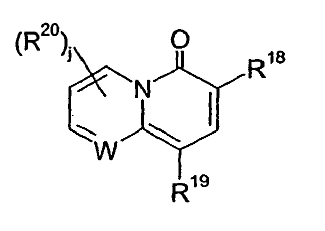 R¹² is hydrogen or C₁₋₆alkyl;
R¹³ is hydrogen or C₁₋₄alkyl optionally substituted by a group selected from optionally substituted phenyl, optionally substituted 5 or 6 membered heteroaryl and optionally substituted 9 to 10 membered fused bicyclic heteroaryl;
R¹⁴ and R¹⁵ are each independently hydrogen or C₁₋₆alkyl;
R¹⁶ is halogen, cyano, nitro, trifluoromethyl, azido, -C(O)R²¹, -C(O)OR²¹, -OC(O)R²¹,-OC(O)OR²¹, -NR²²C(O)R²³, -C(O)NR²²R²³, -NR²²R²³, hydroxy, C₁₋₆alkyl, -S(O)ₖC₁₋₆alkyl, C₁₋₆alkoxy, -(CH₂)ₘaryl or -(CH₂)ₘheteroaryl, wherein the alkoxy group is optionally substituted by up to three groups independently selected from -NR¹⁴R¹⁵, halogen and -OR¹⁴, and the aryl and heteroaryl groups are optionally substituted by up to five groups independently selected from halogen, cyano, nitro, trifluoromethyl, azido,-C(O)R²⁴, -C(O)OR²⁴, -OC(O)OR²⁴, -NR²⁵C(O)R²⁶, -C(O)NR²⁵R²⁶, -NR²⁵R²⁶, hydroxy, C₁₋₆alkyl and C₁₋₆alkoxy;
R¹⁷ is hydrogen, C₁₋₆alkyl, C₃₋₇cycloalkyl, C₃₋₆alkenyl or a 5 or 6 membered heterocyclic group, wherein the alkyl, cycloalkyl, alkenyl and heterocyclic groups are optionally substituted by up to three substituents independently selected from optionally substituted 5 or 6 membered heterocyclic group, optionally substituted 5 or 6 membered heteroaryl, -OR²⁷, -S(O)ₙR²⁷, -NR²⁷R²⁸, -CONR²⁷R²⁸, halogen and cyano;
R¹⁸ is hydrogen, -C(O)OR²⁹, -C(O)NHR²⁹ or -C(O)CH₂NO₂;
R¹⁹ is hydrogen, C₁₋₄alkyl optionally substituted by hydroxy or C₁₋₄alkoxy, C₃₋₇cycloalkyl, or optionally substituted phenyl or benzyl;
R²⁰ is halogen, C₁₋₄alkyl, C₁₋₄thioalkyl, C₁₋₄alkoxy, -NH₂, -NH(C₁₋₄alkyl) or -N(C₁₋₄alkyl)₂;
R²¹ is hydrogen, C₁₋₁₀alkyl, -(CH₂)paryl or -(CH₂)pheteroaryl;
R²² and R²³ are each independently hydrogen, -OR¹⁴, C₁₋₆alkyl, -(CH₂)qaryl or - (CH₂)_{q}heterocyclyl;
R²⁴ is hydrogen, C₁₋₁₀alkyl, -(CH₂)ᵣaryt or -(CH₂)ᵣheteroaryl;
R²⁵ and R²⁶ are each independently hydrogen, -OR¹⁴, C₁₋₆alkyl, -(CH₂)ₛaryl or-(CH₂)ₛheterocyclyl;
R²⁷ and R²⁸ are each independently hydrogen, C₁₋₄alkyl or C₁₋₄alkoxyC₁₋₄alkyl;
R²⁹ is hydrogen, C₁₋₆alkyl optionally substituted by up to three groups independently selected from halogen, C₁₋₄alkoxy, -OC(O)C₁₋₆alkyl, -OC(O)OC₁₋₆alkyl, -C(O)NR³²R33 and -NR³²R³³, -(CH₂)_{w}C₃₋₇cycloalkyl, C₃₋₆alkenyl or C₃₋₆alkynyl;
R³⁰ is hydrogen, C₁₋₄alkyl, C₃₋₇cycloalkyl, optionally substituted phenyl or benzyl, acetyl or benzoyl;
R³¹ is hydrogen or R²⁰, or R³¹ and R¹⁹ are linked to form the bivalent radical -O(CH₂)₂- or -(CH₂)ₜ-;
R³² and R³³ are each independently hydrogen or C₁₋₆alkyl optionally substituted by-C(O)OC₁₋₆alkyl, or
R³² and R³³, together with the nitrogen atom to which they are bound, form a 5 or 6 membered heterocyclic group optionally containing one additional heteroatom selected from oxygen, nitrogen and sulfur;
X is -U(CH₂)ᵥ-;
U is a divalent radical selected from -N(R³⁰)-, -O-, -S(O)_{z}-, -N(R³⁰)C(O)-, -C(O)N(R³⁰)- and -N[C(O)R³⁰]-;
W is -C(R³¹)- or a nitrogen atom;
d is an integer from 1 to 5;
e is an integer from 2 to 4;
f, g, h, m, p, q, r, s and w are each independently integers from 0 to 4;
i is an integer from 1 to 6;
j, k, n and z are each independently integers from 0 to 2;
t is 2 or 3;
v is an integer from 2 to 8;
and pharmaceutically acceptable derivatives thereof.

According to a further embodiment the present invention provides compounds of general formula (IB): 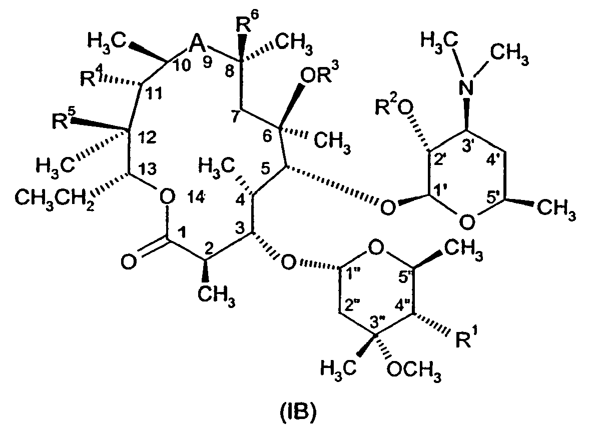 wherein
A is a bivalent radical selected from -C(O)-, -C(O)NH-, -NHC(O)-, -N(R⁷)-CH₂-, -CH₂-N(R⁷)-, -CH(NR⁸R⁹)- and -C(=NR¹⁰)-;
R¹ is -OC(O)(CH₂)_{d}XR¹¹;
R² is hydrogen or a hydroxyl protecting group;
R³ is hydrogen, C₁₋₄alkyl, or C₃₋₆alkenyl optionally substituted by 9 to 10 membered fused bicyclic heteroaryl;
R⁴ is hydroxy, C₃₋₆alkenyloxy optionally substituted by 9 to 10 membered fused bicyclic heteroaryl, or C₁₋₆alkoxy optionally substituted by C₁₋₆alkoxy or -O(CH₂)ₑNR⁷R¹², R⁵ is hydroxy, or
R⁴ and R⁵ taken together with the intervening atoms form a cyclic group having the following structure: 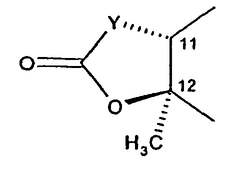 wherein Y is a bivalent radical selected from -CH₂-, -CH(CN)-. -O-, -N(R¹³)- and-CH(SR¹³)-;
R⁶ is hydrogen or fluorine;
R⁷ is hydrogen or C₁₋₆alkyl;
R⁸ and R⁹ are each independently hydrogen, C₁₋₆alkyl, -C(=NR¹⁰)NR¹⁴R¹⁵ or-C(O)R¹⁴, or
R⁸ and R⁹ together form =CH(CR¹⁴R¹⁵)_{f}aryl, =CH(CR¹⁴R¹⁵)_{f}heterocyclyl, =CR¹⁴R¹⁵ or =C(R¹⁴)C(O)OR¹⁴, wherein the alkyl, aryl and heterocyclyl groups are optionally substituted by up to three groups independently selected from R¹⁶;
R¹⁰ is -OR¹⁷, C₁₋₆alkyl, -(CH₂)_{g}aryl, -(CH₂)_{g}heterocyclyl or -(CH₂)ₕO(CH₂)ᵢOR⁷, wherein each R¹⁰ group is optionally substituted by up to three groups independently selected from R¹⁶;
R¹¹ is a heterocyclic group having the following structure: 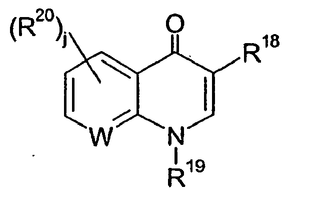 or 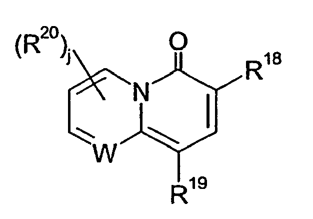 R¹² is hydrogen or C₁₋₆alkyl;
R¹³ is hydrogen or C₁₋₄alkyl substituted by a group selected from optionally substituted phenyl, optionally substituted 5 or 6 membered heteroaryl and optionally substituted 9 to 10 membered fused bicyclic heteroaryl;
R¹⁴ and R¹⁵ are each independently hydrogen or C₁₋₆alkyl;
R¹⁶ is halogen, cyano, nitro, trifluoromethyl, azido, -C(O)R²¹, -C(O)OR²¹, -OC(O)R²¹,-OC(O)OR²¹, -NR²²C(O)R²³, -C(O)NR²²R²³, -NR²²R²³, hydroxy, C₁₋₆alkyl, -S(O)ₖC₁₋₆alkyl, C₁₋₆alkoxy, -(CH₂)ₘaryl or -(CH₂)ₘheteroaryl, wherein the alkoxy group is optionally substituted by up to three groups independently selected from -NR¹⁴R¹⁵, halogen and -OR¹⁴, and the aryl and heteroaryl groups are optionally substituted by up to five groups independently selected from halogen, cyano, nitro, trifluoromethyl, azido,-C(O)R²⁴, -C(O)OR²⁴, -OC(O)OR²⁴, -NR²⁵C(O)R²⁶, -C(O)NR²⁵R²⁶, -NR²⁵R²⁶, hydroxy, C₁₋₆alkyl and C₁₋₆alkoxy;
R¹⁷ is hydrogen, C₁₋₆alkyl, C₃₋₇cycloalkyl, C₃₋₆alkenyl or a 5 or 6 membered heterocyclic group, wherein the alkyl, cycloalkyl, alkenyl and heterocyclic groups are optionally substituted by up to three substituents independently selected from optionally substituted 5 or 6 membered heterocyclic group, optionally substituted 5 or 6 membered heteroaryl, -OR²⁷, -S(O)ₙR²⁷, -NR²⁷R²⁸, -CONR²⁷R²⁸, halogen and cyano;
R¹⁸ is hydrogen, -C(O)OR²⁹, -C(O)NHR²⁹ or -C(O)CH₂NO₂;
R¹⁹ is hydrogen, C₁₋₄alkyl optionally substituted by hydroxy or C₁₋₄alkoxy, C₃₋₇cycloalkyl, or optionally substituted phenyl or benzyl;
R²⁰ is halogen, C₁₋₄alkyl, C₁₋₄thioalkyl, C₁₋₄alkoxy, -NH₂, -NH(C₁₋₄alkyl) or -N(C₁₋₄alkyl)₂;
R²¹ is hydrogen, C₁₋₁₀alkyl, -(CH₂)paryl or -(CH₂)pheteroaryl;
R²² and R²³ are each independently hydrogen, -OR¹⁴, C₁₋₆alkyl, -(CH₂)_{q}aryl or-(CH₂)_{q}heterocyclyl;
R²⁴ is hydrogen, C₁₋₁₀alkyl, -(CH₂)ᵣaryl or -(CH₂)ᵣheteroaryl;
R²⁵ and R²⁶ are each independently hydrogen, -OR¹⁴, C₁₋₆alkyl, -(CH₂)ₛaryl or-(CH₂)ₛheterocyclyl;
R²⁷ and R²⁸ are each independently hydrogen, C₁₋₄alkyl or C₁₋₄alkoxyC₁₋₄alkyl;
R²⁹ is hydrogen or C₁₋₆alkyl optionally substituted by up to three groups independently selected from halogen, C₁₋₄alkoxy, -OC(O)C₁₋₆alkyl and -OC(O)OC₁₋₆alkyl;
R³⁰ is hydrogen, C₁₋₄alkyl, C₃₋₇cycloalkyl, optionally substituted phenyl or benzyl, acetyl or benzoyl;
R³¹ is hydrogen or R²⁰, or R³¹ and R¹⁹ are linked to form the bivalent radical -O(CH₂)₂- or -(CH₂)ₜ-;
X is -U(CH₂)ᵥ-;
U is a divalent radical selected from -N(R³⁰)-, -O-, -S(O)_{z}-, -N(R³⁰)C(O)-, -C(O)N(R³⁰)- and -N[C(O)R³⁰]-;
W is -C(R³¹)- or a nitrogen atom;
d is an integer from 1 to 5;
e is an integer from 2 to 4;
f, g, h, m, p, q, r and s are each independently integers from 0 to 4;
i is an integer from 1 to 6;
j, k, n and z are each independently integers from 0 to 2;
t is 2 or 3;
v is an integer from 2 to 8;
and pharmaceutically acceptable derivatives thereof.

The term "pharmaceutically acceptable" as used herein means a compound which is suitable for pharmaceutical use. Salts and solvates of compounds of the invention which are suitable for use in medicine are those wherein the counterion or associated solvent is pharmaceutically acceptable. However, salts and solvates having non-pharmaceutically acceptable counterions or associated solvents are within the scope of the present invention, for example, for use as intermediates in the preparation of other compounds of the invention and their pharmaceutically acceptable salts and solvates.

The term "pharmaceutically acceptable derivative" as used herein means any pharmaceutically acceptable salt, solvate or prodrug, e.g. ester, of a compound of the invention, which upon administration to the recipient is capable of providing (directly or indirectly) a compound of the invention, or an active metabolite or residue thereof. Such derivatives are recognizable to those skilled in the art, without undue experimentation. Nevertheless, reference is made to the teaching of Burger's Medicinal Chemistry and Drug Discovery, 5^{th} Edition, Vol 1: Principles and Practice, which is incorporated herein by reference to the extent of teaching such derivatives. Preferred pharmaceutically acceptable derivatives are salts, solvates, esters, carbamates and phosphate esters. Particularly preferred pharmaceutically acceptable derivatives are salts, solvates and esters. Most preferred pharmaceutically acceptable derivatives are salts and esters, in particular salts.

The compounds of the present invention may be in the form of and/or may be administered as a pharmaceutically acceptable salt. For a review on suitable salts see Berge *et al.,* J. Pharm. Sci., 1977, 66, 1-19.

Typically, a pharmaceutically acceptable salt may be readily prepared by using a desired acid or base as appropriate. The salt may precipitate from solution and be collected by filtration or may be recovered by evaporation of the solvent. For example, an aqueous solution of an acid such as hydrochloric acid may be added to an aqueous suspension of a compound of formula (I) and the resulting mixture evaporated to dryness (lyophilised) to obtain the acid addition salt as a solid. Alternatively, a compound of formula (I) may be dissolved in a suitable solvent, for example an alcohol such as isopropanol, and the acid may be added in the same solvent or another suitable solvent. The resulting acid addition salt may then be precipitated directly, or by addition of a less polar solvent such as diisopropyl ether or hexane, and isolated by filtration.

Suitable addition salts are formed from inorganic or organic acids which form non-toxic salts and examples are hydrochloride, hydrobromide, hydroiodide, sulphate, bisulphate, nitrate, phosphate, hydrogen phosphate, acetate, trifluoroacetate, maleate, malate, fumarate, lactate, tartrate, citrate, formate, gluconate, succinate, pyruvate, oxalate, oxaloacetate, trifluoroacetate, saccharate, benzoate, alkyl or aryl sulphonates (eg methanesulphonate, ethanesulphonate, benzenesulphonate or p-toluenesulphonate) and isethionate. Representative examples include trifluoroacetate and formate salts, for example the bis or tris trifluoroacetate salts and the mono or diformate salts, in particular the tris trifluoroacetate salt and the diformate salt. A further representative example of a formate salt is the tris formate salt.

Pharmaceutically acceptable base salts include ammonium salts, alkali metal salts such as those of sodium and potassium, alkaline earth metal salts such as those of calcium and magnesium and salts with organic bases, including salts of primary, secondary and tertiary amines, such as isopropylamine, diethylamine, ethanolamine, trimethylamine, dicyclohexyl amine and N-methyl-D-glucamine.

Compounds of the invention may have both a basic and an acidic centre may therefore be in the form of zwitterions.

Those skilled in the art of organic chemistry will appreciate that many organic compounds can form complexes with solvents in which they are reacted or from which they are precipitated or crystallized. These complexes are known as "solvates". For example, a complex with water is known as a "hydrate". Solvates of the compound of the invention are within the scope of the invention. The salts of the compound of formula (I) may form solvates (e.g. hydrates) and the invention also includes all such solvates.

The term "prodrug" as used herein means a compound which is converted within the body, e.g. by hydrolysis in the blood, into its active form that has medical effects. Pharmaceutically acceptable prodrugs are described in T. Higuchi and V. Stella, "Prodrugs as Novel Delivery Systems", Vol. 14 of the A.C.S. Symposium Series, Edward B. Roche, ed., "Bioreversible Carriers in Drug Design", American Pharmaceutical Association and Pergamon Press, 1987, and in D. Fleisher, S. Ramon and H. Barbra "Improved oral drug delivery: solubility limitations overcome by the use of prodrugs", Advanced Drug Delivery Reviews (1996) 19(2) 115-130, each of which are incorporated herein by reference.

Prodrugs are any covalently bonded carriers that release a compound of structure (I) *in vivo* when such prodrug is administered to a patient. Prodrugs are generally prepared by modifying functional groups in a way such that the modification is cleaved, either by routine manipulation or *in vivo,* yielding the parent compound. Prodrugs include, for example, compounds of this invention wherein hydroxy, amine or sulfhydryl groups are bonded to any group that, when administered to a patient, cleaves to form the hydroxy, amine or sulfhydryl groups. Thus, representative examples of prodrugs include (but are not limited to) acetate, formate and benzoate derivatives of alcohol, sulfhydryl and amine functional groups of the compounds of structure (I). Further, in the case of a carboxylic acid (-COOH), esters may be employed, such as methyl esters, ethyl esters, and the like. Esters may be active in their own right and/or be hydrolysable under *in vivo* conditions in the human body. Suitable pharmaceutically acceptable *in vivo* hydrolysable ester groups include those which break down readily in the human body to leave the parent acid or its salt.

References hereinafter to a compound according to the invention include both compounds of formula (I) and their pharmaceutically acceptable derivatives.

With regard to stereoisomers, the compounds of structure (I) have more than one asymmetric carbon atom. In the general formula (I) as drawn, the solid wedge shaped bond indicates that the bond is above the plane of the paper. The broken bond indicates that the bond is below the plane of the paper.

It will be appreciated that the substituents on the macrolide may also have one or more asymmetric carbon atoms. Thus, the compounds of structure (I) may occur as individual enantiomers or diastereomers. All such isomeric forms are included within the present invention, including mixtures thereof.

Where a compound of the invention contains an alkenyl group, cis (Z) and trans (E) isomerism may also occur. The present invention includes the individual stereoisomers of the compound of the invention and, where appropriate, the individual tautomeric forms thereof, together with mixtures thereof.

Separation of diastereoisomers or cis and trans isomers may be achieved by conventional techniques, e.g. by fractional crystallisation, chromatography or H.P.L.C. A stereoisomeric mixture of the agent may also be prepared from a corresponding optically pure intermediate or by resolution, such as H.P.L.C., of the corresponding mixture using a suitable chiral support or by fractional crystallisation of the diastereoisomeric salts formed by reaction of the corresponding mixture with a suitable optically active acid or base, as appropriate.

The compounds of structure (I) may be in crystalline or amorphous form. Furthermore, some of the crystalline forms of the compounds of structure (I) may exist as polymorphs, which are included in the present invention.

Compounds wherein R² represents a hydroxyl protecting group are in general intermediates for the preparation of other compounds of formula (I).

When the group OR² is a protected hydroxyl group this is conveniently an ether or an acyloxy group. Examples of particularly suitable ether groups include those in which R² is a trialkylsilyl (i.e. trimethylsilyl). When the group OR² represents an acyloxy group, then examples of suitable groups R² include acetyl or benzoyl.

R⁶ is hydrogen or fluorine. However, it will be appreciated that when A is -C(O)NH- or-CH₂-N(R⁷)-, R⁶ is hydrogen.

When R¹¹ is a heterocyclic group having the following structure: 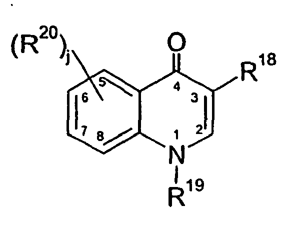 said heterocyclic is linked in the 5, 6, 7 or 8 position to the X group as above defined. In one embodiment, the heterocyclic is linked in the 6 or 7 position. In another embodiment, the heterocyclic is linked in the 5 or 8 position. When present, the R²⁰ group or groups may be attached at any position on the ring. In one embodiment, an R²⁰ group is attached at the 6 position.

When R¹¹ is a heterocyclic group having the following structure: 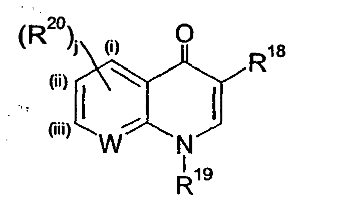 wherein W is -C(R³¹)- where R³¹ is R²⁰ or R³¹ and R¹⁹ are linked to form the bivalent radical -O(CH₂)₂- or -(CH₂)ₜ-, said heterocyclic is linked in the (i), (ii) or (iii) position to the X group as above defined. In one embodiment, the heterocyclic is linked in the (i) position. In another embodiment, the heterocyclic is linked in the (ii) or (iii) position.

When R¹¹ is a heterocyclic group having the following structure: 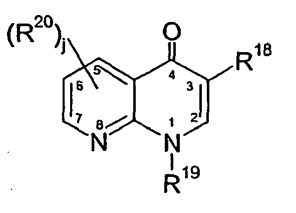 said heterocyclic is linked in the 5, 6 or 7 position to the X group as defined above. In one embodiment, the heterocyclic is linked in the 6 or 7 position. In another embodiment, the heterocyclic is linked in the 5 position.

When R¹¹ is a heterocyclic group having the following structure: 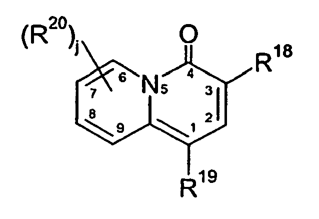 said heterocyclic is linked in the 6, 7, 8 or 9 position to the X group as above defined. In one embodiment, the heterocyclic is linked in the 7 or 8 position. In another embodiment, the heterocyclic is linked in the 6 or 9 position.

When R¹¹ is a heterocyclic group having the following structure: 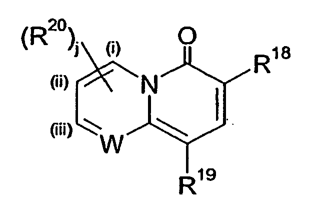 wherein W is -C(R³¹)- where R³¹ is R²⁰ or R³¹ and R¹⁹ are linked to form the bivalent radical -O(CH₂)₂- or -(CH₂)ₜ-, said heterocyclic is linked in the (i), (ii) or (iii) position to the X group as above defined. In one embodiment, the heterocyclic is linked in the (i) position. In another embodiment, the heterocyclic is linked in the (ii) or (iii) position.

When R¹¹ is a heterocyclic group having the following structure: 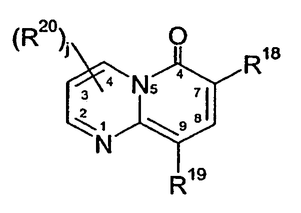 said heterocyclic is linked in the 2, 3 or 4 position to the X group as above defined. In one embodiment, the heterocyclic is linked in the 2 or 3 position. In another embodiment, the heterocyclic is linked in the 4 position.

The term "alkyl" as used herein as a group or a part of a group refers to a straight or branched hydrocarbon chain containing the specified number of carbon atoms. For example, C₁₋₁₀alkyl means a straight or branched alkyl containing at least 1, and at most 10, carbon atoms. Examples of "alkyl" as used herein include, but are not limited to, methyl, ethyl, n-propyl, n-butyl, n-pentyl, isobutyl, isopropyl, t-butyl, hexyl, heptyl, octyl, nonyl and decyl. A C₁₋₄alkyl group is preferred, for example methyl, ethyl, n-propyl, isopropyl, n-butyl, isobutyl or t-butyl.

The term "C₃₋₇cycloalkyl" group as used herein refers to a non-aromatic monocyclic hydrocarbon ring of 3 to 7 carbon atoms such as, for example, cyclopropyl, cyclobutyl, cyclopentyl, cyclohexyl or cycloheptyl.

The term "alkoxy" as used herein refers to a straight or branched chain alkoxy group containing the specified number of carbon atoms. For example, C₁₋₆alkoxy means a straight or branched alkoxy containing at least 1, and at most 6, carbon atoms. Examples of "alkoxy" as used herein include, but are not limited to, methoxy, ethoxy, propoxy, prop-2-oxy, butoxy, but-2-oxy, 2-methylprop-l-oxy, 2-methylprop-2-oxy, pentoxy and hexyloxy. A C₁₋₄alkoxy group is preferred, for example methoxy, ethoxy, propoxy, prop-2-oxy, butoxy, but-2-oxy or 2-methylprop-2-oxy.

The term "alkenyl" as used herein as a group or a part of a group refers to a straight or branched hydrocarbon chain containing the specified number of carbon atoms and containing at least one double bond. For example, the term "C₂₋₆alkenyl" means a straight or branched alkenyl containing at least 2, and at most 6, carbon atoms and containing at least one double bond. Similarly, the term "C₃₋₆alkenyl" means a straight or branched alkenyl containing at least 3, and at most 6, carbon atoms and containing at least one double bond. Examples of "alkenyl" as used herein include, but are not limited to, ethenyl, 2-propenyl, 3-butenyl, 2-butenyl, 2-pentenyl, 3-pentenyl, 3-methyl-2-butenyl, 3-methylbut-2-enyl, 3-hexenyl and 1,1-dimethylbut-2-enyl. It will be appreciated that in groups of the form -O-C₂₋₆alkenyl, the double bond is preferably not adjacent to the oxygen.

The term "alkynyl" as used herein as a group or a part of a group refers to a straight or branched hydrocarbon chain containing the specified number of carbon atoms and containing at least one triple bond. For example, the term "C₃₋₆alkenyl" means a straight or branched alkynyl containing at least 3, and at most 6, carbon atoms containing at least one triple bond. Examples of "alkynyl" as used herein include, but are not limited to, propynyl, 1-butynyl, 2-butynyl, 1-pentynyl and 3-methyl-1-butynyl.

The term "aryl" as used herein refers to an aromatic carbocyclic moiety such as phenyl, biphenyl or naphthyl.

The term "heteroaryl" as used herein, unless otherwise defined, refers to an aromatic heterocycle of 5 to 10 members, having at least one heteroatom selected from nitrogen, oxygen and sulfur, and containing at least 1 carbon atom, including both mono and bicyclic ring systems. Examples of heteroaryl rings include, but are not limited to, furanyl, thiophenyl, pyrrolyl, pyrazolyl, imidazolyl, oxazolyl, isoxazolyl, thiazolyl, isothiazolyl, triazolyl, oxadiazolyl, tetrazolyl, thiadiazolyl, pyridyl, pyridazinyl, pyrazinyl, pyrimidinyl, triazinyl, quinolinyl, isoquinolinyl, 1,2,3,4-tetrahydroisoquinolinyl, benzofuranyl, benzimidazolyl, benzothienyl, benzoxazolyl, 1,3-benzodioxazolyl, indolyl, benzothiazolyl, furylpyridine, oxazolopyridyl and benzothiophenyl.

The term "5 or 6 membered heteroaryl" as used herein as a group or a part of a group refers to a monocyclic 5 or 6 membered aromatic heterocycle containing at least one heteroatom independently selected from oxygen, nitrogen and sulfur. Examples include, but are not limited to, furanyl, thiophenyl, pyrrolyl, pyrazolyl, imidazolyl, oxazolyl, isoxazolyl, thiazolyl, isothiazolyl, triazolyl, oxadiazolyl, tetrazolyl, pyridyl, pyridazinyl, pyrazinyl, pyrimidinyl and triazinyl.

The term "9 to 10 membered fused bicyclic heteroaryl" as used herein as a group or a part of a group refers to quinolinyl, isoquinolinyl, 1,2,3,4-tetrahydroisoquinolinyl, benzofuranyl, benzimidazolyl, benzothienyl, benzoxazolyl, 1,3-benzodioxazolyl, indolyl, benzothiazolyl, furylpyridine, oxazolopyridyl or benzothiophenyl.

The term "heterocyclyl" as used herein, unless otherwise defined, refers to a monocyclic or bicyclic three- to ten-membered saturated or non-aromatic, unsaturated hydrocarbon ring containing at least one heteroatom selected from oxygen, nitrogen and sulfur. Preferably, the heterocyclyl ring has five or six ring atoms. Examples of heterocyclyl groups include, but are not limited to, pyrrolidinyl, tetrahydrofuranyl, tetrahydrothiophenyl, imidazolidinyl, pyrazolidinyl, piperidyl, piperazinyl, morpholino, tetrahydropyranyl and thiomorpholino.

The term "5 or 6 membered heterocyclic group" as used herein as a group or part of a group refers to a monocyclic 5 or 6 membered saturated hydrocarbon ring containing at least one heteroatom independently selected from oxygen, nitrogen and sulfur. Examples of such heterocyclyl groups include, but are not limited to, pyrrolidinyl, tetrahydrofuranyl, tetrahydrothiophenyl, imidazolidinyl, pyrazolidinyl, piperidyl, piperazinyl, morpholino, tetrahydropyranyl and thiomorpholino.

The term "halogen" refers to a fluorine, chlorine, bromine or iodine atom.

The terms "optionally substituted phenyl", "optionally substituted phenyl or benzyl", "optionally substituted 5 or 6 membered heteroaryl", "optionally substituted 9 to 10 membered fused bicyclic heteroaryl" or "optionally substituted 5 or 6 membered heterocyclic group" as used herein refer to a group which is substituted by 1 to 3 groups selected from halogen, C₁₋₄alkyl, C₁₋₄alkoxy, hydroxy, nitro, cyano, amino, C₁₋₄alkylamino or diC₁₋₄alkylamino, phenyl and 5 or 6 membered heteroaryl.

In one embodiment, A is -C(O)-, -C(O)NH-, -NHC(O)-, -N(R⁷)-CH₂-, -CH₂-N(R⁷)- or-CH(NR⁸R⁹)-. In another embodiment, A is -C(O)-, -C(O)NH-, -NHC(O)-, -CH₂-N(R⁷)-,-CH(NR⁸R⁹)- or -C(=NR¹⁰)-. In another embodiment, A is -C(O)-, -NHC(O)-, -N(R⁷)-CH₂-, -CH₂-N(R⁷)-, -CH(NR⁸R⁹)- or -C(=NR¹⁰)-. In another embodiment, A is -C(O)-,-C(O)NH-, -NHC(O)-, -CH₂-N(R⁷)- or -CH(NR⁸R⁹)-. In a further embodiment, A is -C(O)-, -N(R⁷)-CH₂- or -C(=NR¹⁰)-. Representative examples of A include -C(O)- and -N(R⁷)-CH₂-. A further representative example of A is -C(=NR¹⁰)-. In particular, A is -C(O)-.

A representative example of R² is hydrogen.

Representative examples of R³ include hydrogen and C₁₋₄alkyl, for example hydrogen and methyl. In particular, R³ is methyl.

In one embodiment, R⁴ and R⁵ are hydroxy, R⁴ is C₁₋₄alkoxy such as methoxy and R⁵ is hydroxy, or R⁴ and R⁵ taken together with the intervening atoms form a cyclic group having the following structure: 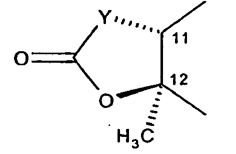 wherein Y is a bivalent radical selected from -CH₂-, -CH(CN)-, -O-, -N(R¹³)- and-CH(SR¹³)-. In another embodiment, R⁴ and R⁵ are hydroxy. In a further embodiment, R⁴ is C₁₋₄alkoxy such as methoxy and R⁵ is hydroxy. Alternatively, R⁴ and R⁵ taken together with the intervening atoms form a cyclic group having the following structure: 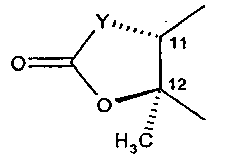 wherein Y is a bivalent radical selected from -O- and -N(R¹³)-.

A representative example of R⁶ is hydrogen.

A representative example of R⁷ is C₁₋₆alkyl, for example C₁₋₄alkyl, in particular methyl.

A representative example of R¹⁰ is -OR¹⁷.

In one embodiment, R¹¹ includes heterocyclic groups having the following structure: 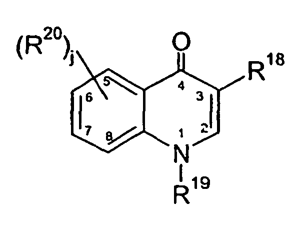 wherein the heterocyclic is linked in the 6 or 7 position to the X group as above defined, and heterocyclic groups having the following structure: 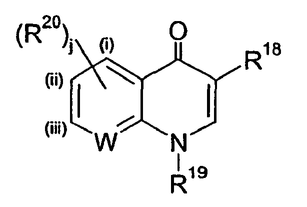 wherein W is -C(R³¹)- where R³¹ and R¹⁹ are linked to form the bivalent radical-O(CH₂)₂- or -(CH₂)ₜ-, in particular -(CH₂)ₜ-, and said heterocyclic is linked in the (i), (ii) or (iii) position, in particular the (ii) position, to the X group as above defined.

Representative examples of R¹¹ include heterocyclic groups having the following structure: 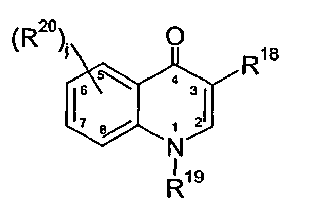 wherein the heterocyclic is linked in the 6 or 7 position to the X group as above defined.

Further representative examples of R¹¹ include heterocyclic groups having the following structure: 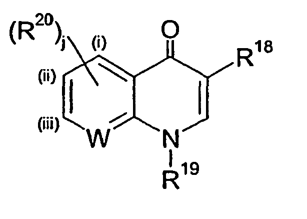 wherein W is -C(R³¹)- where R³¹ and R¹⁹ are linked to form the bivalent radical-O(CH₂)₂- or -(CH₂)ₜ-, in particular -(CH₂)ₜ-, and said heterocyclic is linked in the (i), (ii) or (iii) position, in particular the (ii) position, to the X group as above defined.

in one embodiment, R¹³ is hydrogen or C₁₋₄alkyl substituted by a group selected from optionally substituted phenyl, optionally substituted 5 or 6 membered heteroaryl and optionally substituted 9 to 10 membered fused bicyclic heteroaryl. In another embodiment, R¹³ is hydrogen or C₁₋₄alkyl. A representative example of R¹³ is hydrogen. A further representative example of R¹³ is methyl.

Representative examples of R¹⁷ include hydrogen and C₁₋₆alkyl, for example C₁₋₄alkyl, in particular methyl, optionally substituted by -OR²⁷.

In one embodiment, R¹⁸ is hydrogen, -C(O)OR²⁹, -C(O)NHR²⁹ or -C(O)CH₂NO₂. In another embodiment, R¹⁸ is -C(O)OR²⁹, -C(O)NHR²⁹ or -C(O)CH₂NO₂. In another embodiment, R¹⁸ is -C(O)OR²⁹. In a further embodiment, R¹⁸ is -C(O)OR²⁹, wherein R²⁹ is hydrogen, C₁₋₆alkyl optionally substituted by up to three groups independently selected from C₁₋₄alkoxy, -OC(O)C₁₋₆alkyl, -C(O)NR³²R³³ and -NR³²R³³, -(CH₂)_{w}C₃₋₇cycloalkyl, C₃₋₆alkenyl or C₃₋₆alkynyl. A representative example of R¹⁸ is -C(O)OR²⁹, wherein R²⁹ is hydrogen or C₁₋₄alkyl, for example hydrogen or methyl. Further representative examples of R¹⁸ include -C(O)OR²⁹, wherein R²⁹ is hydrogen; C₁₋₆alkyl, for example C₁₋₄alkyl such as methyl, ethyl, isopropyl, isobutyl or n-butyl, optionally substituted by up to three groups independently selected from C₁₋₄alkoxy such as methoxy, -OC(O)C₁₋₆alkyl such as -OC(O)t-butyl, -C(O)NR³²R³³ and -NR³²R³³;-(CH₂)_{W}C₃₋₇cycloalkyl, for example -(CH₂)_{w}C₃₋₆cycloalkyl such as -(CH₂)_{w}cyclopropyl; C₃₋₆alkenyl, for example C₃₋₄alkenyl such as 2-propenyl or 3-butenyl; or C₃₋₆alkynyl, for example C₃₋₄alkynyl such as 2-butynyl. In particular, R²⁹ is hydrogen.

In one embodiment, R¹⁹ is C₁₋₄alkyl, for example methyl or ethyl, optionally substituted by C₁₋₄alkoxy, for example methoxy, or R¹⁹ is C₃₋₇cycloalkyl, for example C₃₋₆cycloalkyl such as cyclopropyl. A representative example of R¹⁹ is C₁₋₄alkyl, in particular ethyl.

A representative example of R²⁰ is halogen, in particular fluorine.

A representative example of R²⁷ is C₁₋₄alkoxyC₁₋₄alkyl.

In one embodiment, R²⁹ is hydrogen or C₁₋₆alkyl optionally substituted by up to three groups independently selected from halogen, C₁₋₄alkoxy, -OC(O)C₁₋₆alkyl and-OC(O)OC₁₋₆alkyl. In another embodiment, R²⁹ is hydrogen, C₁₋₆alkyl optionally substituted by up to three groups independently selected from halogen, C₁₋₄alkoxy,-OC(O)C₁₋₆alkyl, -OC(O)OC₁₋₆alkyl, -C(O)NR³²R³³ and -NR³²R³³, -(CH₂)_{w}C_{3- 7}cycloalkyl, C₃₋₆alkenyl or C₃₋₆alkynyl. In a further embodiment, R²⁹ is hydrogen, C₁₋₆alkyl optionally substituted by up to three groups independently selected from C₁₋₄alkoxy, -OC(O)C₁₋₆alkyl, -C(O)NR³²R³³ and -NR³²R³³; -(CH₂)_{W}C₃₋₇cycloalkyl; C₃₋₆alkenyl; or C₃₋₆alkynyl. Representative examples of R²⁹ include hydrogen; C₁₋₆alkyl, for example C₁₋₄alkyl such as methyl, ethyl, isopropyl, 2-methylpropyl or n-butyl, optionally substituted by up to three groups independently selected from C₁₋₄alkoxy such as methoxy, -OC(O)C₁₋₆alkyl such as -OC(O)t-butyl, -C(O)NR³²R³³ and -NR³²R³³;-(CH₂)_{W}C₃₋₇cycloalkyl, for example -(CH₂)_{w}C₃₋₆cycloalkyl such as -(CH₂)_{w}cyclopropyl; C₃₋₆alkenyl, for example C₃₋₄alkenyl such as 2-propenyl or 3-butenyl; and C₃₋₆alkynyl, for example C₃₋₄alkynyl such as 2-butynyl. In particular, R²⁹ is hydrogen.

In one embodiment, R³⁰ is hydrogen or C₁₋₄alkyl. A representative example of R³⁰ is hydrogen.

In one embodiment, R³¹ is hydrogen or R³¹ and R¹⁹ are linked to form the bivalent radical -(CH₂)ₜ-. A representative example of R³¹ is hydrogen.

In one embodiment, R³² and R³³ are each independently hydrogen or C₁₋₆alkyl optionally substituted by -C(O)OC₁₋₆alkyl, or
R³² and R³³, together with the nitrogen atom to which they are bound, form a 5 or 6 membered heterocyclic group optionally containing one additional heteroatom selected from oxygen, nitrogen and sulfur.

In another embodiment, R³² and R³³ are each independently hydrogen or C₁₋₆alkyl, for example C₁₋₄alkyl such as methyl, optionally substituted by -C(O)OC₁₋₆alkyl, for example -C(O)OC₁₋₄alkyl such as -C(O)Oethyl.

In a further embodiment, R³² and R³³, together with the nitrogen atom to which they are bound, form a 6 membered heterocyclic group optionally containing one additional oxygen atom.

In one embodiment, X is -U(CH₂)ᵥ- wherein U is a divalent radical selected from -N(R³⁰)-, -O- and -S(O)_{z}-. In a further embodiment, X is -U(CH₂)ᵥ- wherein U is a divalent radical selected from -N(R³⁰)- and -O-. A representative example of X is -U(CH₂)ᵥ- wherein U is the divalent radical -N(R³⁰)-. A further representative example of X is -U(CH₂)ᵥ- wherein U is the divalent radical -O-.

A representative example of W is -C(R³¹)-.

Representative examples of Y include -O- and -N(R¹³)-.

In one embodiment, d is an integer from 2 to 5. A representative example of d is 1 to 3, for example 2. A further representative example of d is 5.

A representative example of w is 1.

Representative examples of t are 2 and 3. In particular, t is 3.

In one embodiment, v is an integer of from 2 to 8. A representative example of v is 2 to 4, for example 3.

Representative examples of j include 0 and 1. In particular, j is 0.

It is to be understood that the present invention covers all combinations of particular and preferred groups described hereinabove. It is also to be understood that the present invention encompasses compounds of formula (I) in which a particular group or parameter, for example R⁷, R¹⁴, R15, R¹⁶, R²⁰, R²¹, R²², R²³, R²⁴, R²⁵, R²⁶, R²⁷, R²⁸, R³², R³³, k, m, n, p, q, r and s may occur more than once. In such compounds it will be appreciated that each group or parameter is independently selected from the values listed.

In one embodiment, when A is -C(O)-, d is 2, X is -NH(CH₂)₃- and R¹¹ is a heterocyclic group of the following formula: 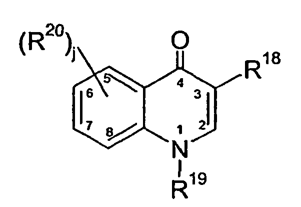 wherein the heterocyclic is linked in the 6 or 7 position to the X group, j is 0, R¹⁸ is carboxy and R¹⁹ is ethyl.

In a further embodiment, when A is -C(O)-, d is 2, X is -NH(CH₂)₃- and R¹¹ is a heterocyclic group of the following formula: 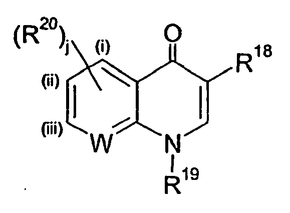 wherein W is -C(R³¹)- where R³¹ and R¹⁹ are linked to form the bivalent radical -(CH₂)ₜ-, said heterocyclic is linked in the (ii) or (iii) position to the X group, j is 0 and R¹⁸ is carboxy.

Particularly preferred compounds of the invention are:
4"-O-{3-[3-(3-carboxy-1,4-dihydro-1-ethyl-4-oxo-6-quinolinyl)propylamino]propionyl}-6-O-methylerythromycin A;
4"-*O*-{3-[3-(3-carboxy-1,4-dihydro-1-ethyl-4-oxo-6-quinolinyl)
propylamino]propionyl}-azithromycin-11,12-carbonate;
4"-*O*-{3-[3-(3-carboxy-1,4-dihydro-1-ethyl-4-oxo-6-quinolinyl} propylamino]propionyl}-6-O-methyl-11-desoxy-11-(R)-amino-erythromycin A 11,12-carbamate;
and pharmaceutically acceptable derivatives thereof.

Further particularly preferred compounds of the invention are:
4"-O-[3-[4-(2-carboxy-6,7-dihydro-1*H*,5*H*-pyrido[3,2,1-ij]-1-oxo-9-quinolinyl) propylamino]propionyl]-6-O-methyl erythromycin A;
4"-*O*-[3-[4-(3-carboxy-1-ethyl-1,4-dihydro-4-oxo-6-quinolinyl)propylamino]propionyl]-(9E)-*O*-({[2-(methyloxy)ethyl]oxy}methanoximino erythromycin A;
4"-*O*-[3-[4-(3-carboxy-1-ethyl-1,4-dihydro-4-oxo-6-quinolinyl)propylamino]propionyl]-(9E)-*O*-hydroximino erythromycin A;
4"-O-[3-[4-(2-carboxy-6,7-dihydro-1*H*,5*H*-pyrido[3,2,1-ij]-1-oxo-9-quinolinyl)propylamino]propionyl]-(9E)-*O*-hydroximino erythromycin A;
and pharmaceutically acceptable derivatives thereof.

Additional particularly preferred compounds of the invention are:
4"-*O*-{6-[3-(3-carboxy-1-ethyl-4-oxo-1,4-dihydro-quinolin-6-yl)-propoxy]-hexanoyl}-azithromycin;
4"-*O*-{6-[3-(3-carboxy-1-ethyl-4-oxo-1,4-dihydro-quinolin-6-yl)-propoxy]-hexanoyl}-clarithromycin;
and pharmaceutically acceptable derivatives thereof.

Compounds according to the invention also exhibit a broad spectrum of antimicrobial activity, in particular antibacterial activity, against a wide range of clinical pathogenic microorganisms. Using a standard microtiter broth serial dilution test, compounds of the invention have been found to exhibit useful levels of activity against a wide range of pathogenic microorganisims. In particular, the compounds of the invention may be active against strains of Staphylococcus aureus, Streptopococcus pneumoniae, Moraxella catarrhalis, Streptococcus pyogenes, Haemophilus influenzae, Enterococcus faecalis, Chlamydia pneumoniae, Mycoplasma pneumoniae and Legionella pneumophila. The compounds of the invention may also be active against resistant strains, for example erythromycin resistant strains. In particular, the compounds of the invention may be active against erythromycin resistant strains of Streptococcus pneumoniae, Streptococcus pyogenes and Staphylococcus aureus.

The compounds of the invention may therefore be used for treating a variety of diseases caused by pathogenic microorganisms, in particular bacteria, in human beings and animals. It will be appreciated that reference to treatment includes acute treatment or prophylaxis as well as the alleviation of established symptoms.

Thus, according to another aspect of the present invention we provide a compound of formula (I) or a pharmaceutically acceptable derivative thereof for use in therapy.

According to a further aspect of the invention we provide a compound of formula (I) or a pharmaceutically acceptable derivative thereof for use in the therapy or prophylaxis of systemic or topical microbial infections in a human or animal subject.

According to a further aspect of the invention we provide the use of a compound of formula (I) or a pharmaceutically acceptable derivative thereof in the manufacture of a medicament for use in the treatment or prophylaxis of systemic or topical microbial infections in a human or animal body.

According to a yet further aspect of the invention we provide a method of treatment of the human or non-human animal body to combat microbial infections comprising administration to a body in need of such treatment of an effective amount of a compound of formula (I) or a pharmaceutically acceptable derivative thereof.

While it is possible that, for use in therapy, a compound of the invention may be administered as the raw chemical it is preferable to present the active ingredient as a pharmaceutical formulation eg when the agent is in admixture with a suitable pharmaceutical excipient, diluent or carrier selected with regard to the intended route of administration and standard pharmaceutical practice.

Accordingly, in one aspect, the present invention provides a pharmaceutical composition or formulation comprising at least one compound of the invention or a pharmaceutically acceptable derivative thereof in association with a pharmaceutically acceptable excipient, diluent and/or carrier. The excipient, diluent and/or carrier must be "acceptable" in the sense of being compatible with the other ingredients of the formulation and not deleterious to the recipient thereof.

In another aspect, the invention provides a pharmaceutical composition comprising, as active ingredient, at least one compound of the invention or a pharmaceutically acceptable derivative thereof in association with a pharmaceutically acceptable excipient, diluent and/or carrier for use in therapy, and in particular, in the treatment of human or animal subjects suffering from a condition susceptible to amelioration by an antimicrobial compound.

In another aspect, the invention provides a pharmaceutical composition comprising a therapeutically effective amount of the compounds of the present invention and a pharmaceutically acceptable excipient, diluent and/or carrier (including combinations thereof).

There is further provided by the present invention a process of preparing a pharmaceutical composition, which process comprises mixing at least one compound of the invention or a pharmaceutically acceptable derivative thereof, together with a pharmaceutically acceptable excipient, diluent and/or carrier.

The compounds of the invention may be formulated for administration in any convenient way for use in human or veterinary medicine and the invention therefore includes within its scope pharmaceutical compositions comprising a compound of the invention adapted for use in human or veterinary medicine. Such compositions may be presented for use in a conventional manner with the aid of one or more suitable excipients, diluents and/or carriers. Acceptable excipients, diluents and carriers for therapetic use are well known in the pharmaceutical art, and are described, for example, in Remington's Pharmaceutical Sciences, Mack Publishing Co. (A. R. Gennaro edit. 1985). The choice of pharmaceutical excipient, diluent and/or carrier can be selected with regard to the intended route of administration and standard pharmaceutical practice. The pharmaceutical compositions may comprise as - or in addition to - the excipient, diluent and/or carrier any suitable binder(s), lubricant(s), suspending agent(s), coating agent(s), solubilising agent(s).

Preservatives, stabilisers, dyes and even flavouring agents may be provided in the pharmaceutical composition. Examples of preservatives include sodium benzoate, sorbic acid and esters of p-hydroxybenzoic acid. Antioxidants and suspending agents may be also used.

For some embodiments, the agents of the present invention may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e. g. as a carrier, diluent or solubiliser. Alpha-, beta- and gamma-cyclodextrins are most commonly used and suitable examples are described in WO 91/11172, WO 94/02518 and WO 98/55148.

The compounds of the invention may be milled using known milling procedures such as wet milling to obtain a particle size appropriate for tablet formation and for other formulation types. Finely divided (nanoparticulate) preparations of the compounds of the invention may be prepared by processes known in the art, for example see International Patent Application No. WO 02/00196 (SmithKline Beecham).

The routes for administration (delivery) include, but are not limited to, one or more of: oral (e. g. as a tablet, capsule, or as an ingestable solution), topical, mucosal (e. g. as a nasal spray or aerosol for inhalation), nasal, parenteral (e. g. by an injectable form), gastrointestinal, intraspinal, intraperitoneal, intramuscular, intravenous, intrauterine, intraocular, intradermal, intracranial, intratracheal, intravaginal, intracerebroventricular, intracerebral, subcutaneous, ophthalmic (including intravitreal or intracameral), transdermal, rectal, buccal, epidural and sublingual.

There may be different composition/formulation requirements depending on the different delivery systems. By way of example, the pharmaceutical composition of the present invention may be formulated to be delivered using a mini-pump or by a mucosal route, for example, as a nasal spray or aerosol for inhalation or ingestable solution, or parenterally in which the composition is formulated by an injectable form, for delivery, by, for example, an intravenous, intramuscular or subcutaneous route. Alternatively, the formulation may be designed to be delivered by both routes.

Where the agent is to be delivered mucosally through the gastrointestinal mucosa, it should be able to remain stable during transit though the gastrointestinal tract; for example, it should be resistant to proteolytic degradation, stable at acid pH and resistant to the detergent effects of bile.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

It is to be understood that not all of the compounds need be administered by the same route. Likewise, if the composition comprises more than one active component, then those components may be administered by different routes.

The compositions of the invention include those in a form especially formulated for parenteral, oral, buccal, rectal, topical, implant, ophthalmic, nasal or genito-urinary use. For some applications, the agents of the present invention are delivered systemically (such as orally, buccally, sublingually), more preferably orally. Hence, preferably the agent is in a form that is suitable for oral delivery.

If the compound of the present invention is administered parenterally, then examples of such administration include one or more of: intravenously, intraarterially, intraperitoneally, intrathecally, intraventricularly, intraurethrally, intrasternally, intracranially, intramuscularly or subcutaneously administering the agent; and/or by using infusion techniques.

For parenteral administration, the compound is best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

The compounds according to the invention may be formulated for use in human or veterinary medicine by injection (e.g. by intravenous bolus injection or infusion or via intramuscular, subcutaneous or intrathecal routes) and may be presented in unit dose form, in ampoules, or other unit-dose containers, or in multi-dose containers, if necessary with an added preservative. The compositions for injection may be in the form of suspensions, solutions, or emulsions, in oily or aqueous vehicles, and may contain formulatory agents such as suspending, stabilising, solubilising and/or dispersing agents. Alternatively the active ingredient may be in sterile powder form for reconstitution with a suitable vehicle, e.g. sterile, pyrogen-free water, before use.

The compounds of the invention can be administered (e. g. orally or topically) in the form of tablets, capsules, ovules, elixirs, solutions or suspensions, which may contain flavouring or colouring agents, for immediate-, delayed-, modified-, sustained-, pulsed- or controlled-release applications.

The compounds of the invention may also be presented for human or veterinary use in a form suitable for oral or buccal administration, for example in the form of solutions, gels, syrups, mouth washes or suspensions, or a dry powder for constitution with water or other suitable vehicle before use, optionally with flavouring and colouring agents. Solid compositions such as tablets, capsules, lozenges, pastilles, pills, boluses, powder, pastes, granules, bullets or premix preparations may also be used. Solid and liquid compositions for oral use may be prepared according to methods well known in the art. Such compositions may also contain one or more pharmaceutically acceptable carriers and excipients which may be in solid or liquid form.

The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia.

Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the agent may be combined with various sweetening or flavouring agents, colouring matter or dyes, with emulsifying and/or suspending agents and with diluents such as water, ethanol, propylene glycol and glycerin, and combinations thereof.

The compounds of the invention may also be administered orally in veterinary medicine in the form of a liquid drench such as a solution, suspension or dispersion of the active ingredient together with a pharmaceutically acceptable carrier or excipient.

The compounds of the invention may also, for example, be formulated as suppositories e.g. containing conventional suppository bases for use in human or veterinary medicine or as pessaries e.g. containing conventional pessary bases.

The compounds according to the invention may be formulated for topical administration, for use in human and veterinary medicine, in the form of ointments, creams, gels, hydrogels, lotions, solutions, shampoos, powders (including spray or dusting powders), pessaries, tampons, sprays, dips, aerosols, drops (e.g. eye ear or nose drops) or pourons.

For application topically to the skin, the agent of the present invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water.

Alternatively, it can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

The compounds may also be dermally or transdermally administered, for example, by use of a skin patch.

For ophthalmic use, the compounds can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

As indicated, the compound of the present invention can be administered intranasally or by inhalation and is conveniently delivered in the form of a dry powder inhaler or an aerosol spray presentation from a pressurised container, pump, spray or nebuliser with the use of a suitable propellant, e. g. dichlorodifluoromethane, trichlorofluoromethane, dichlorotetrafluoroethane, a hydrofluoroalkane such as 1,1,1,2-tetrafluoroethane (HFA 134AT"") or 1,1,1,2,3,3,3-heptafluoropropane (HFA 227EA), carbon dioxide or other suitable gas. In the case of a pressurised aerosol, the dosage unit may be determined by providing a valve to deliver a metered amount. The pressurised container, pump, spray or nebuliser may contain a solution or suspension of the active compound, e. g. using a mixture of ethanol and the propellant as the solvent, which may additionally contain a lubricant, e. g. sorbitan trioleate.

Capsules and cartridges (made, for example, from gelatin) for use in an inhaler or insufflator may be formulated to contain a powder mix of the compound and a suitable powder base such as lactose or starch.

For topical administration by inhalation the compounds according to the invention may be delivered for use in human or veterinary medicine via a nebuliser.

The compounds of the invention may also be used in combination with other therapeutic agents. The invention thus provides, in a further aspect, a combination comprising a compound of the invention or a pharmaceutically acceptable derivative thereof together with a further therapeutic agent.

When a compound of the invention or a pharmaceutically acceptable derivative thereof is used in combination with a second therapeutic agent active against the same disease state the dose of each compound may differ from that when the compound is used alone. Appropriate doses will be readily appreciated by those skilled in the art. It will be appreciated that the amount of a compound of the invention required for use in treatment will vary with the nature of the condition being treated and the age and the condition of the patient and will be ultimately at the discretion of the attendant physician or veterinarian. The compounds of the present invention may for example be used for topical administration with other active ingredients such as corticosteroids or antifungals as appropriate.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations by any convenient route.

When administration is sequential, either the compound of the invention or the second therapeutic agent may be administered first. When administration is simultaneous, the combination may be administered either in the same or different pharmaceutical composition.

When combined in the same formulation it will be appreciated that the two compounds must be stable and compatible with each other and the other components of the formulation. When formulated separately they may be provided in any convenient formulation, conveniently in such manner as are known for such compounds in the art.

The compositions may contain from 0.01-99% of the active material. For topical administration, for example, the composition will generally contain from 0.01-10%, more preferably 0.01-1% of the active material.

Typically, a physician will determine the actual dosage which will be most suitable for an individual subject. The specific dose level and frequency of dosage for any particular individual may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy.

For oral and parenteral administration to humans, the daily dosage level of the agent may be in single or divided doses.

For systemic administration the daily dose as employed for adult human treatment will range from 2-100mg/kg body weight, preferably 5-60mg/kg body weight, which may be administered in 1 to 4 daily doses, for example, depending on the route of administration and the condition of the patient. When the composition comprises dosage units, each unit will preferably contain 200mg to 1g of active ingredient. The duration of treatment will be dictated by the rate of response rather than by arbitrary numbers of days.

Compounds of general formula (I) and salts thereof may be prepared by the general methods outlined hereinafter, said methods constituting a further aspect of the invention. In the following description, the groups R¹ to R³³, A, X, Y, U, W, d, e, f, g, h, i, j, k, m, n, p, q, r, s, t, v, w and z have the meaning defined for the compounds of formula (I) unless otherwise stated.

The group X^{a}R^{11a} is XR¹¹ as defined for formula (I) or a group convertible to XR¹¹. Conversion of a group X^{a}R^{11a} to a XR¹¹ group typically arises if a protecting group is needed during the reactions described below. A comprehensive discussion of the ways in which such groups may be protected and methods for cleaving the resulting protected derivatives is given by for example T.W. Greene and P.G.M Wuts in Protective Groups in Organic Synthesis 2^{nd} ed., John Wiley & Son, Inc 1991 and by P.J. Kocienski in Protecting. Groups, Georg Thieme Verlag 1994 which are incorporated herein by reference. Examples of suitable amino protecting groups include acyl type protecting groups (e.g. formyl, trifluoroacetyl and acetyl), aromatic urethane type protecting groups (e.g. benzyloxycarbonyl (Cbz) and substituted Cbz, and 9-fluorenylmethoxycarbonyl (Fmoc)), aliphatic urethane protecting groups (e.g. t-butyloxycarbonyl (Boc), isopropyloxycarbonyl and cyclohexyloxycarbonyl) and alkyl type protecting groups (e.g. benzyl, trityl and chlorotrityl). Examples of suitable oxygen protecting groups may include for example alkyl silyl groups, such as trimethylsilyl or tert-butyldimethylsilyl; alkyl ethers such as tetrahydropyranyl or tert-butyl; or esters such as acetate. Hydroxy groups may be protected by reaction of for example acetic anhydride, benzoic anhydride or a trialkylsilyl chloride in an aprotic solvent. Examples of aprotic solvents are dichloromethane, N,N-dimethylformamide, dimethylsulfoxide, tetrahydrofuran and the like.

Compounds of formula (I) may be prepared by reaction of a 4" hydroxy compound of formula (II) wherein R² is a hydroxy protecting group with a suitable activated and protected derivative of the carboxylic acid (III), followed where necessary by subsequent removal of the hydroxyl protecting group R² and conversion of the X^{a}R^{11a} group to XR¹¹.

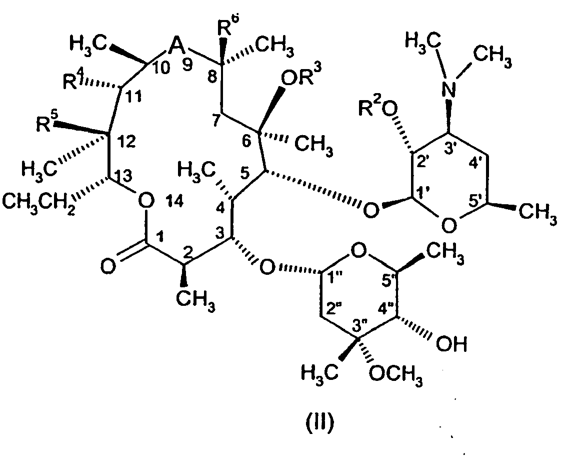

HOC(O)(CH₂)_{d}X^{a}R^{11a} (III)

Suitable activated derivatives of the carboxyl group include the corresponding acyl halide, mixed anhydride or activated ester such as a thioester. The reaction is preferably carried out in a suitable aprotic solvent such as a halohydrocarbon (e.g. dichloromethane) or N,N-dimethylformamide optionally in the presence of a tertiary organic base such as dimethylaminopyridine or triethylamine or in the presence of inorganic base (eg sodium hydroxide) and at a temperature within the range of 0° to 120°C. The compounds of formula (II) and (III) may also be reacted in the presence of a carbodiimide such as dicyclohexylcarbodiimide (DCC).

In a further embodiment of the invention, compounds of formula (I) wherein U is a group selected from -N(R³⁰)- and -S-, may be prepared by reaction of compounds of formula (IV), 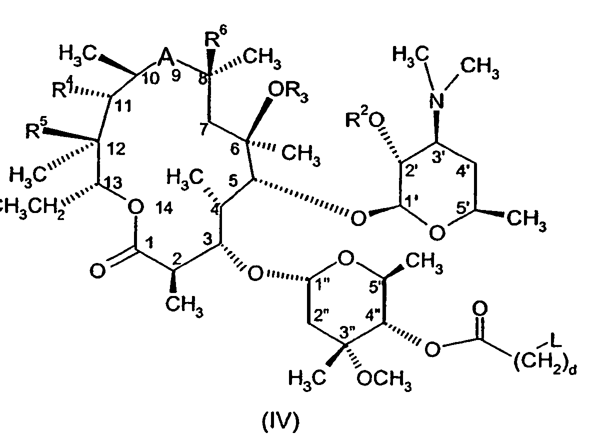 wherein d is an integer from 1 to 5 and L is a suitable leaving group, with X^{a}R^{11a} (V) in which U is a group selected from -N(R³⁰)- and -S-. The reaction is preferably carried out in a solvent such as a halohydrocarbon (e.g. dichloromethane), an ether (e.g. tetrahydrofuran or dimethoxyethane), acetonitrile or ethyl acetate and the like, dimethylsulfoxide, N,N-dimethylformamide or 1-methyl-pyrrolidone and in the presence of a base, followed, if desired, by removal of the hydroxyl protecting group R² and conversion of the X^{a}R^{11a} group to XR¹¹. Examples of the bases which may be used include organic bases such as diisopropylethylamine, triethylamine and 1,8-diazabicyclo[5.4.0]undec-7-ene, and inorganic bases such as potassium hydroxide, cesium hydroxide, tetraalkylammonium hydroxide, sodium hydride, potassium hydride and the like. Suitable leaving groups for this reaction include halide (e.g. chloride, bromide or iodide) or a sulfonyloxy group (e.g. tosyloxy or methanesulfonyloxy).

Compounds of formula (IV) may be prepared by reaction of a compound of formula (II), wherein R² is a hydroxyl protecting group, with a suitable activated derivative of the carboxylic acid HOC(O)(CH₂)_{d}L (VI), wherein L is a suitable leaving group as above defined. Suitable activated derivatives of the carboxyl group are those defined above for carboxylic acid (III). The reaction is carried out using the conditions described above for the reaction of a compound of formula (II) with carboxylic acid (III).

In a preferred embodiment of the invention, compounds of formula (I) wherein d is 2 and U is a group selected from -N(R³⁰)- and -S-, may be prepared by Michael reaction of a compound of formula (VII), wherein R² is optionally a hydroxyl protecting group 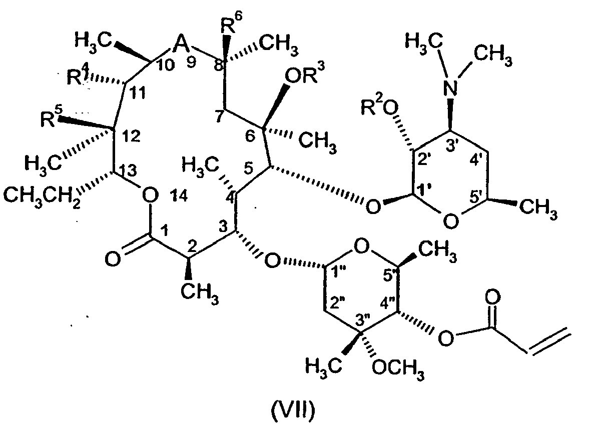 with a compound of formula X^{a}R^{11a} (V). The reaction is suitably carried out in a solvent such as dimethylsulfoxide, N,N-dimethylformamide, 1-methyl-pyrrolidone, a halohydrocarbon (e.g. dichloromethane), an ether (e.g. tetrahydrofuran or dimethoxyethane), acetonitrile or alcohol (e.g methanol or isopropanol) and the like, and in the presence of a base, followed, if desired, by removal of hydroxyl protecting group R² and conversion of the X^{a}R^{11a} group to XR¹¹.

Compounds of formula (I) may be converted into other compounds of formula (I). Thus compounds of formula (I) wherein U is -S(O)_{z}- and z is 1 or 2 may be prepared by oxidation of the corresponding compound of formula (I) wherein z is 0. The oxidation is preferably carried out using a peracid, e.g. peroxybenzoic acid, followed by treatment with a phosphine, such as triphenylphosphine. The reaction is suitably carried out in an organic solvent such as methylene chloride. Compounds of formula (I) wherein U is -N(R³⁰)- and R³⁰ is C₁₋₄alkyl can be prepared from compounds wherein R³⁰ is hydrogen by reductive alkylation.

Compounds of formula (II) wherein A is -C(O)NH- or -NHC(O)-, R⁴ or R⁵ are hydroxy, R³ is hydrogen and R⁶ is hydrogen are known compounds or they may be prepared by analogous methods to those known in the art. Thus they can be prepared according to the procedures described in EP 507595 and EP 503932.

Compounds of formula (II), wherein A is -C(O)NH- or -NHC(O)-, R⁴ or R⁵ are hydroxy and R³ is C₁₋₄alkyl or C₃₋₆alkenyl optionally substituted by 9 to 10 membered fused bicyclic heteroaryl and R⁶ is hydrogen are known compounds or they may be prepared by analogous methods to those known in the art. Thus they can be prepared according to the procedures described in WO 9951616 and WO 0063223.

Compounds of formula (II), wherein A is -C(O)NH-, R⁴ and R⁵ taken together with the intervening atoms form a cyclic group having the following structure: 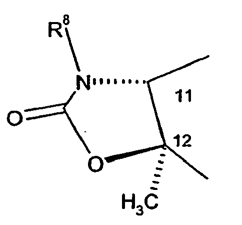 R³ is C₁₋₄alkyl, or C₃₋₆alkenyl optionally substituted by 9 to 10 membered fused bicyclic heteroaryl and R⁶ is hydrogen are known compounds or they may be prepared by analogous methods to those known in the art. Thus they can be prepared according to the procedures described in US 6262030.

Compounds of formula (II), wherein A is -C(O)-, -C(O)NH-, -NHC(O)-, -N(R⁷)-CH₂-, -CH₂-N(R⁷)- or -CH(NR⁸R⁹)-, R⁴ or R⁵ are hydroxy or R⁴ and R⁵ taken together with the intervening atoms form a cyclic group having the following structure: 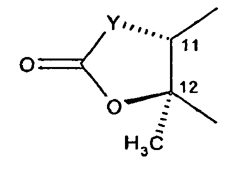 wherein Y is a bivalent radical selected from -O- and -N(R¹³)-, and R³ is C₁₋₄alkyl, or C₃₋₆alkenyl optionally substituted by 9 to 10 membered fused bicyclic heteroaryl are known compounds or they may be prepared by analogous methods to those known in the art. Thus they can be prepared according to the procedures described in EP 307177, EP 248279, WO 0078773, WO 9742204.

Compounds of formula (II), wherein A is -C(O)NH-, -NHC(O)-, -N(CH₃)-CH₂- or -CH₂-N(CH₃)-, R⁴ or R⁵ are hydroxy or R⁴ and R⁵ taken together with the intervening atoms form a cyclic group having the following structure: 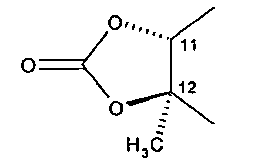 and R⁶ is hydrogen are known compounds or they may be prepared by analogous methods to those known in the art. Thus they can be prepared according to the procedures described in EP 508699 and J.Chem. Res.Synop (1988 pages 152-153), US 6262030.

Compounds of formula (II), wherein A is -C(=NR¹⁰)-, R⁴ or R⁵ are hydroxy or R⁴ and R⁵ taken together with the intervening atoms form a cyclic group having the following structure: 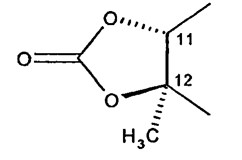 and R⁶ is hydrogen, are known compounds or they may be prepared by analogous methods to those known in the art. Thus they can be prepared according to the procedures described in EP 284203.

Compounds of formula (II), wherein A is -C(O)-, R⁴ and R⁵ taken together with the intervening atoms form a cyclic group having the following structure: 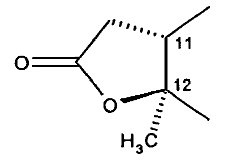 R⁶ is hydrogen and R³ is C₁₋₄ alkyl may be prepared by decarboxylation of a compound of formula (VIII), wherein R³⁴ is hydroxy protecting group followed, if required, by removal of the protecting group R² or R³⁴.

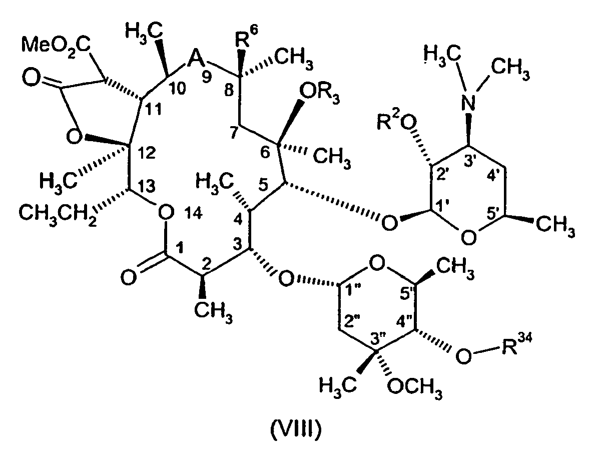

The decarboxylation may be carried out in the presence of a lithium salt such as lithium chloride, preferably in an organic solvent such as dimethylsulfoxide.

Compounds of formula (II), wherein A is -C(O)-, R⁴ and R⁵ taken together with the intervening atoms form a cyclic group having the following structure: 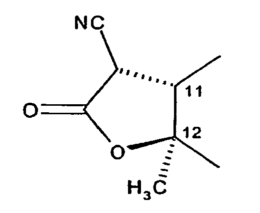 and R₃ is C₁₋₄ alkyl may be prepared according to the procedures described in WO 02/50091 and WO 02/50092.

Compounds of formula (III) wherein X is -U(CH₂)ᵥ-, in which U is -N(R³⁰)-, -O- or -S-, may be prepared by reaction of X^{a}R^{11a} (V), wherein X^{a} has the meaning defined above with R³⁵OC(O)(CH₂)_{d}L (IX) wherein R³⁵ is carboxyl protecting group and L is a suitable leaving group, followed by removal of R³⁵. Suitable R³⁵ carboxyl protecting group include t-butyl, allyl or benzyl.

In order that the invention may be more fully understood the following examples are given by way of illustration only.

The following abbreviations are used in the text: Ac for acetyl, BOC for t-butoxycarbonyl, DCM for dichloromethane, DMAP for 4-dimethylaminopyridine, DMF for N,N-dimethylformamide, DMSO for dimethyl sulfoxide, EtOAc for ethyl acetate, Me for methyl, MeOH for methanol, TEA for triethylamine and TFA for trifluoroacetic acid.

### Examples

2'-*O*-Acetyl-6-*O*-methyl-erythromycin A may be prepared by the procedure described by W. R. Baker *et al.* in *J. Org. Chem.* **1988,** 53, 2340, 2'-*O*-acetyl-azithromycin-11,12-carbonate may be prepared by the procedure described by S. Djokic *et al.* in *J. Chem. Res. (S)* **1988**, 152 and 11-*O*-(9E)-methoximino erythromycin A may be prepared according to the procedure described by E. Hunt *et al.* in *J. Chem. Soc.,* **1989,** 1726.

### Nomenclature

In the Examples, compounds of formula (I) in which R¹¹ is a tricyclic heterocyclic group are referred to using the numbering system below:

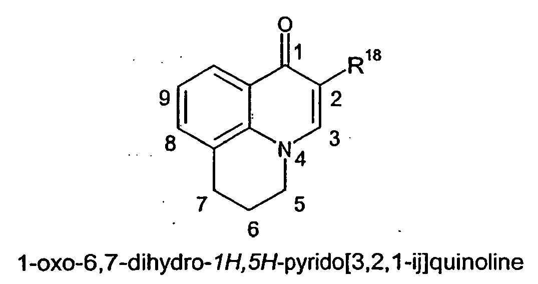

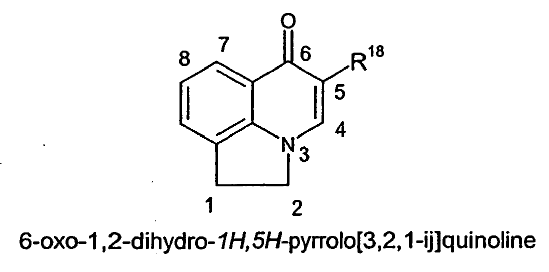

### Intermediate 1: 7-(3-Aminopropyl)-1,4-dihydro-1-ethyl-6-fluoro-4-oxo-quinoline-3-carboxylic acid sodium salt

### a) 7-(3-t-Butoxycarbonylamino-prop-1-ynyl)-1,4-dihydro-1-ethyt-6-fluoro-4-oxo-quinoline-3-carboxylic acid ethyl ester.

1,4-Dihydro-1-ethyl-6-fluoro-7-iodo-4-oxo-quinoline-3-carboxylic acid ethyl ester (0.495 g, 1.265 mmol), copper (I) iodide (26 mg, 013 mmol) and triethylamine (6.16 mL, 44 mmol) were suspended in dry acetonitrile (22 mL). The light green suspension was heated to 50°C whilst argon was bubbled through. After 20 min, dichlorobis(triphenylphosphine)palladium (II) (0.026g, 0.0379 mmol) and *N-t*-butoxycarbonylpropargylamine (0.341 g, 2.05 mmol) were added and the brown suspension was heated under reflux. After 2 h the reaction mixture was cooled, filtered and concentrated. The residue was taken up in dichloromethane and washed with water. The organic phase was dried and concentrated to provide a brown oil which was purified by chromatography on silica gel eluting with 0-2.5% (9:1 MeOH/20 M NH₃) in dichloromethane to yield the title compound as a beige solid; ESMS *m*/*z* 417 [M+H]⁺.

1,4-Dihydro-1-ethyl-6-fluoro-7-iodo-4-oxo-quinoline-3-carboxylic acid can, for example, be prepared by the following method: 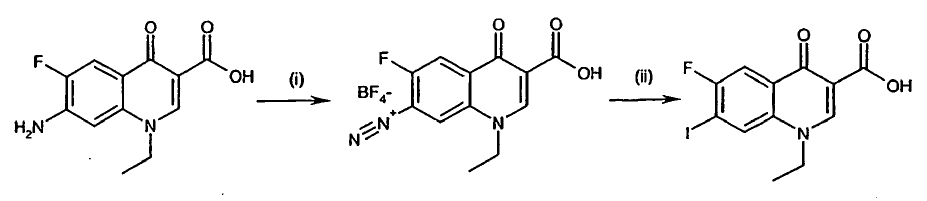 wherein step (i) is carried out according to the procedure described by C.B. Ziegler, W. V. Curran, N. A. Kuck, S. M. Harris and Y-I Lin in *J. Het. Chem.,* **1989,** *26*, 1141 and step (ii) is carried out using sodium iodide, for example by the method of *J. Med. Chem.,* **2002,** *67*, 843.

### b) 7-(3-t-Butoxycarbonylaminopropyl)-1,4-dihydro-1-ethyl-6-fluoro-4-oxo-quinoline-3-carboxylic acid ethyl ester.

A solution of **Intermediate 1a** (0.322 mg, 0.77 mmol) in dichloromethane (12 mL) was treated with 10% palladium on carbon (60 mg) and hydrogenated at room temperature and atmospheric pressure overnight. The reaction mixture was filtered and concentrated to yield the title compound as a yellow solid; ESMS *m*/*z* 421 [M+H]⁺.

### c) 7-(3-Aminopropyl)-1,4-dihydro-1-ethyl-6-fluoro-4-oxo-quinoline-3-carboxylic acid ethyl ester.

To a solution of **Intermediate 1b** (254 mg, 0.6 mmol) in dichloromethane (6 mL) was added trifluoroacetic acid (0.66 mL). After 0.75 h at room temperature the reaction mixture was concentrated and the residue was applied to a Varian Bond Elute SCX cartridge. Flushing with MeOH and subsequent elution with 0.04 M NH₃ in MeOH up to 2.0 M NH₃ in MeOH to provided the title compound as a yellow oil; ESMS *m*/*z* 321 [M+H]⁺.

### d) 7-(3-Ammopropyl)-1,4-dihydro-1-ethyl-6-fluoro-4-oxo-quinline-3-carboxylic acid sodium salt.

**Intermediate 1b** (188 mg, 0.59 mmol) was suspended in 1,4-dioxan (6 mL) and treated with 2N aqueous sodium hydroxide (0.28 mL). The suspension was sonicated for 2 h then treated with excess solid carbon dioxide. Evaporation of the dioxan and filtration of the resultant mixture gave the title compound as a yellow solid. ESMS *m*/*z* 293 [M+H]⁺.

### Intermediate 2: 6-(3-Aminopropyl)-1,4-dihydro-1-ethyl-4-oxo-quinoline-3-carboxylic acid trifluoroacetate salt

### a) 1,4-Dihydro-1-ethyl-6-iodo-4-oxo-quinoline-3-carboxylic acid ethyl ester.

A mixture of 1,4-dihydro-6-iodo-4-oxo-quinoline-3-carboxylic acid (J. Ellis, E. Gellert, J. Robson, *Aust. J. Chem.,* **1973,** *26*, 907) (3.15 g, 10 mmol), potassium carbonate (6.9 g, 50 mmol) and iodoethane (15.6 g, 100 mmol) in dry DMF was heated at 70°C with vigorous stirring. After 16 h the mixture was cooled and diluted with ethyl acetate. The resultant mixture was washed with water and the organic phase separated, dried and evaporated to yield the title compound as pale yellow solid, ¹H NMR δ (CDCl₃) 1.41 (3H, t, J = 7.1 Hz), 1.54 (3H, t, J = 7.3 Hz), 4.23 (2H, q, J = 7.2 Hz), 4.40 (2H, q, J = 7.1 Hz), 7.20 (1H, d, J = 8.9 Hz), 7.95 (1H, dd, J = 2.1 & 8.9 Hz), 8.48 (1H, s), 8.86 (1H, d, J = 2.1 Hz).

### b) 6-(3-t-Butoxycarbonylamino-prop-1-ynyl)-1,4-dihydro-1-ethyl-4-oxo-quinoline-3-carboxylic acid ethyl ester.

Using a similar procedure to that described in **Intermediate 1a,** a mixture of **Intermediate 2a** (0.371g, 1mmol) and *N-t*-butoxycarbonylpropargylamine (0.264 g, 1.7 mmol) gave the title compound as a yellow solid; ESMS *m*/*z* 399 [M+H]⁺.

### c) 6-(3-t-Butoxycarbonylaminopropyl)-1,4-dihydro-1-ethyl-4-oxo-quinoline-3-carboxylic acid ethyl ester.

Using a similar procedure to that described in **Intermediate 1b, Intermediate 2b** (0.366 mg, 0.77 mmol) gave the title compound as a yellow oil; ESMS *m*/*z* 403 [M+H]⁺.

### d) 6-(3-Aminopropyl)-1,4-dihydro-1-ethyl-4-oxo-quinoline-3-carboxylic acid ethyl ester.

Using a similar procedure to that described in **Intermediate 1c, Intermediate 2c** (355 mg, 0.88 mmol) gave the title compound as a yellow oil; ESMS *m*/*z* 303 [M+H]⁺.

### e) 6-(3-Aminopropyl)-1,4-dihydro-1-ethyl-4-oxo-quinoline-3-carboxylic acid sodium salt.

Using a similar procedure to that described in **Intermediate 1d, Intermediate 2d** (250 mg, 0.83 mmol) gave the title compound as a yellow solid; ESMS *m*/*z* 275 [M+H]⁺.

### f) 6-(3-Aminopropyl)-1,4-dihydro-1-ethyl-4-oxo-quinoline-3-carboxylic acid trifluoroacetate salt.

**Intermediate 2e** (0.06 g, 0.2 mmol) was subjected to reverse phase HPLC purification to give the title compound as white solid; ¹H NMR δ [(CD₃)₂SO] 1.54 (3H, t, J = 7.2 Hz), 2.0-2.1 (2H, m), 2.9-3.0 (4H, m), 4.58 (2H, q, J = 7.2 Hz), 7.85,(1H, dd, J = 2.2 & 8.8 Hz), 7.96 (1H, d, J = 8.8 Hz), 8.36 (1H, d, J = 1.8 Hz), 8.97 (1H, s).

### Intermediate 3: 2'-O-Acetyl-4"-O-Propenoyl-azithromycin-11,12-carbonate

A solution of 2'-O-acetyl-azithromycin-11,12-carbonate (10.9 g) in toluene (300 mL) was stirred at room temperature under argon atmosphere. To this solution TEA (12.66 mL) and 3-chloro-propionyl chloride (1.94 mL) were added in two portions over a period of 10 minutes. After 20 minutes the solution was diluted with a saturated aqueous solution of NaHCO₃ (300 mL) and extracted with toluene (4x80 mL). The collected organic phase was dried, filtered and concentrated under reduced pressure affording the title compound (11.0 g).
MS; m/z (ES): 872 [MH]⁺.

### Intermediate 4: 4"-O-Propenoyl-azithromycin-11,12-carbonate

A solution of **Intermediate 3** (11.0 g) in MeOH (200 mL) was stirred at room temperature for 48 h. The solvent was evaporated under reduced pressure affording the title compound (9.81 g).
MS; m/z (ES): 829.1 [MH]⁺.
¹H-NMR (500 MHz,) δ: 6.45 (d, 1H), 6.17 (dd, 1H), 5.87 (d, 1H), 5.11 (d, 1H), 4.88 (dd, 1H), 4.77 (d, 1H), 4.53 (d, 1H), 4.47-4.40 (m, 3H), 3.72 (m, 1H), 3.60 (d, 1H), 3.33 (s, 3H), 3.25 (dd, 1H), 2.87-2.85 (m, 2H), 2.58 (m, 1H), 2.44-2.38 (m, 2H), 2.32 (s, 6H), 2.21 (s, 3H), 2.06 (m, 1H), 2.00 (m, 1H), 1.92 (m, 1H), 1.84 (m, 1H), 170-1.56 (m, 4H), 1.45 (s, 3H), 1.40 (dd, 1H), 1.29 (s, 3H), 1.25 (m, 1H), 1.22 (d, 3H), 1.18 (d, 6H), 1.12 (s, 3H), 108-1.06 (2d, 6H), 0.93 (m, 6H).

### Intermediate 5: 4"-O-Propenoyl-azithromycin

To a solution of **Intermediate 4** (1.3 g) in acetonitrile (50 mL), a saturated aqueous solution of potassium carbonate (30 mL) was added at room temperature. The resulting mixture was heated to 70°C for 8 h. The mixture was then diluted with water (100 mL), extracted with EtOAc (4x30 mL). The collected organic phase was dried, filtered and concentrated under reduced pressure. The crude product was purified by flash chromatography (eluent: DCM/MeOH/NH₃ 90/9/0.5) affording the title compound (530 mg).
MS; m/z (ES): 804 [MH]⁺.

### Intermediate 6: 2'-O-Acetyl-4"-O-propenoyl-6-O-methylerythromycin A

To a solution of 2'-*O*-acetyl-6-*O*-methyl-erythromycin A (1.1 g) in DCM (20 mL) pyridine (1.7 mL) and acryl chloride (1.1 mL) were added at 0°C. After 2 h a further addition of pyridine (1.7 mL) and of acryl chloride (1.1 mL) was performed. The reaction mixture was quenched with a saturated solution of NH₄Cl (10 mL) and extracted with DCM (3x20 mL). The organic phase was washed with a saturated solution of NaHCO₃ (10 mL), water (10 mL), dried over Na₂SO₄, filtered and evaporated under reduced pressure. The crude product was purified by flash-chromatography (DCM/MeOH/NH₃ 95/5/0.5) affording the title compound (470 mg); ESMS m/z 844 [M+H]⁺.

### Intermediate 7: 2'-O-Acetyl-6-O-methyl-11-desoxy-11-(R)-amino-erythromycin A 11,12-carbamate

To a solution of 6-O-methyl-11-desoxy-11-(R)-amino-erythromycin A 11,12-carbamate (Alihodzic *et al.,* WO 03/042228) in dichloromethane (50 mL) was added NaHCO₃ (478 mg) at room temperature. To this solution acetic anhydride (0.153 mL) was added and stirred overnight. To this mixture brine (50 mL) and water (20 mL) were added. The organic layer was separated, washed with brine (20 mL), dried, filtered and evaporated under reduced pressure, affording the title compound (1.2 g).
MS; m/z (ES): 816.2 [MH]⁺.

### Intermediate 8: 2'-O-Acetyl-4"-O-propenoyl-6-O-methyl-11-desoxy-11-(R)-amino-erythromycin A 11,12-carbamate

**Intermediate 7** was dissolved in toluene (50 mL) and the solvent was evaporated. This was performed 2 times. After that the residue was again dissolved in toluene (45 mL) and stirred under argon. To this solution TEA (1.8 mL) and 3-chloropropionylchloride (0.40 mL) (in 3 portions in a period of 20 minutes) were added. 20 min later a saturated aqueous solution of NaHCO₃ (50 mL) was added. The aqueous solution was extracted with toluene (3x50 mL), the combined organic solution dried over K₂CO₃ and the solvent removed under reduced pressure affording the title compound (1.04 g).
MS; m/z (ES): 870.1 [MH]⁺.

### Intermediate 9: 4"-O-Propenoyl-6-O-methylerythromycin A

**Intermediate 6** (1.82 g) was dissolved in MeOH (100 mL) and stirred at 60°C for 4 h, then at room temperature for 16 h. The solvent was evaporated under reduced pressure and the crude product was purified by flash chromatography (eluent: MeOH/DCM/NH₄OH 5/90/0) affording the title compound (1.4 g).
MS; m/z (ES): 802 [MH]⁺.
¹H-NMR (500 MHz) δ: 6.44 (d, 1H), 6.13 (dd, 1H), 5.89 (d, 1H), 5.07 (d, 1H), 5.00 (d, 1H), 4.75 (d, 1H), 4.60 (d, 1H), 4.38 (m, 1H), 3.97 (s, 1H), 3.80-3.73 (m, 2H), 3.66 (d, 1H), 3.46 (s, 1H), 3.32 (s, 3H), 3.21-3.18 (m, 2H), 3.04 (s, 3 H), 3.00 (m, 1H), 2.92 (m, 1H), 2.56 (m, 2H), 2.43 (d, 1H), 2.31 (s, 6H).
¹³C-NMR (75 MHz) δ: 221.0; 175.7; 165.8; 131.5; 128.0; 102.1; 96.0; 80.5, 78.8, 78.3; 78.0; 76.6; 74.3, 72.7; 71.1; 69.1; 67.8; 65.3; 63.2: 50.7; 49.5; 45.3; 44.9; 40.3; 39.2; 38.8; 37.2; 35.2; 28.9; 21.7, 21.1; 19.7, 18.3, 18.0, 15.9; 12.3; 10.6; 9.1.

### Intermediate 10: O-(9E)-Methoximino-4"-O-propenoyl erythromycin A

### a) 2'-O-Acetyl-O-(9E)-methoximino erythromycin A

A solution of 11-*O*-(9E)-methoximino erythromycin A (5.7 g, 7.4 mmol) in dichloromethane (70 mL) was treated with triethylamine (1.63 g, 16 mmol) followed by acetic anhydride (1.27 g, 12.5 mmol). After stirring overnight at room temperature the mixture was diluted with dichloromethane and washed with aqueous sodium bicarbonate. The organic layer was separated, dried and evaporated to yield the title product as a solid. ESMS *m*/*z* 806 [MH⁺].

### b) 2'-O-Acetyl-(9E)-methoximino-4"-O-propenoyl erythromycin A

Using a similar procedure to that described in **Intermediate 3, Intermediate 10a** (5.3 g, 6.6 mmol) gave the title compound as a white solid. ESMS *m*/*z* 860 [MH⁺].

### c) O-(9E)-Methoximino-4"-O-propenoyl erythromycin A

Using a similar procedure to that described in **Intermediate 4, Intermediate 10b** (4.17 g, 4.86 mmol) gave the title compound as a white solid. ESMS *m*/*z* 818 [MH⁺].

### Intermediate 11: 6-(3-Aminopropyl)-1,4-dihydro-1-ethyl-4-oxo-puinoline-3-carboxylic acid sodium salt

### a) 6-(3-t-Butoxycarbonylamino-prop-1-ynyl)-1,4-dihydro-1-ethyl-4-oxo-quinoline-3-carboxylic acid ethyl ester

1,4-Dihydro-l-ethyl-6-iodo-4-oxo-quinoline-3-carboxylic acid ethyl ester (0.469 g, 1.265 mmol), copper (I) iodide (26 mg, 0.13 mmol) and triethylamine (6.16 mL, 44 mmol) were suspended in dry acetonitrile (22 mL). The light green suspension was heated to 50°C whilst argon was bubbled through. After 20 min, dichlorobis(triphenylphosphine)palladium (II) (0.026g, 0.0379 mmol) and *t*-butoxycarbonylpropargylamine (0.341 g, 2.05 mmol) were added and the brown suspension was heated under reflux. After 2 h the reaction mixture was cooled, filtered and concentrated. The residue was taken up in dichloromethane and washed with water. The organic phase was dried and concentrated to provide a brown oil which was purified by chromatography on silica gel eluting with 0-2.5% (9:1 MeOH/20 M NH₃) in dichloromethane to yield the title compound as a beige solid. ESMS *m*/*z* 399 (MH⁺).

### b) 6-(3-t-Butoxycarbonylaminopropyl)-1,4-dihydro-1-ethyl-4-oxo-quinoline-3-carboxylic acid ethyl ester

6-(3-*t*-Butoxycarbonylaminoprop-1-ynyl)-1,4-dihydro-1-ethyl-4-oxo-quinoline-3-carboxylic acid ethyl ester (0.306 g, 0.77 mmol) was dissolved in dichloromethane (12 mL) treated with 10% palladium on carbon (0.06 g) and hydrogenated at room temperature and atmospheric pressure overnight. The reaction mixture was filtered and concentrated to yield the title compound as a yellow solid. ESMS *m*/*z* 403 (MH⁺).

### c) 6-(3-Aminopropyl)-1,4-dihydro-1-ethyl-4-oxo-quinoline-3-carboxylic acid ethyl ester

6-(3-*t*-Butoxycarbonylaminopropyl)-1,4-dihydro-1-ethyl-4-oxo-quinoline-3-carboxylic acid ethyl ester (0.242 g, 0.6 mmol) was dissolved in dichloromethane (6 mL) and trifluorocetic acid (0.66 mL) was added. After 0.75 h at room temperature the reaction mixture was concentrated and the residue was applied to a Varian Bond Elute SCX cartridge. Flushing with MeOH and subsequent elution with 0.04 M NH₃ in MeOH up to 2.0 M NH₃ in MeOH to provided the title compound as a yellow oil. ESMS *m*/*z* 303 (MH⁺).

### d) 6-(3-Aminopropyl)-1,4-dihydro-1-ethyl-4-oxo-quinoline-3-carboxylic acid sodium salt

6-(3-Aminopropyl)-1,4-dihydro-1-ethyl-4-oxo-quinoline-3-carboxylic acid ethyl ester (0.179 g, 0.59 mmol) was suspended in 1,4-dioxan (6 mL) and treated with 2M aqueous sodium hydroxide (0.28 mL). The suspension was sonicated for 2 h then treated with excess solid carbon dioxide. Evaporation of the dioxan and filtration of the resultant mixture gave the title compound as a yellow solid. ESMS *m*/*z* 275 (MH⁺).

### Intermediate 12: 4"-O-[3-[4-(3-Carboxy-1-ethyl-1,4-dihydro-4-oxo-6-guinolinyl) propylamino]propionyl]-2'-O-acetyl-(9E)-O-methoximino erythromycin A

Using a similar procedure to that described for the preparation of **Example 1a, Intermediate 10b** (0.36g, 0.41 mmol) and **Intermediate 2f** (0.165 g, 0.41 mmol) gave the title compound. ESMS *m*/*z* 1133[MH⁺].

### Intermediate 13: Diethyl 2-((3,4-dihydro-2H-quinolin-1-yl)methylene)malonate

A mixture of tetrahydroquinoline (13.32g, 100mmol) and diethyl ethoxymethylenemalonate (21.62g, 100mmol) was heated to 130°C using a Dean-Stark apparatus. After 1 hour the reaction mixture was concentrated to give the title compound as a brown oil. ESMS *m*/*z* 304 (MH⁺).

### Intermediate 14: Ethyl 1-oxo-6,7-dihydro-1H,5H-pyrido [3,2,1-ij] quinoline-2-carboxylate

**Intermediate 13** (2.5g, 8.24mmol) was dissolved in polyphosphoric acid and the viscous mixture stirred for 4 hours at 110°C. The reaction mixture was cooled down before adding ice. The resulting precipitate was filtered off, washed with water then dried in a dessicator in the presence of phosphorous pentoxide to give the title compound as a beige solid. ESMS *m*/*z* 258 (MH⁺). ¹H NMR (DMSO-*d₆*) δ 8.55(s, 1H), 8.05 (dd, 1H), 7.54 (dd, 1H), 7.36 (dd, 1H), 4.27 (q, 2H), 4.22 (q, 2H), 3.00 (t, 2H), 2.10 (tt, 2H), 1.28 (t, 3H).

### Intermediate 15: Ethyl 9-bromo-1-oxo-6,7-dihydro-1H,5H-pyrido [3,2,1-ij] quinoline-2-carboxylate

**Intermediate 14** (290mg, 1.13mmol) was dissolved in acetic acid (3mL) and bromine (197mg, 1.23mmol) was added dropwise. The reaction was followed by LC/MS, additional bromine (2 X 197mg) was added. After 24 hours water was added and the precipitate was filtered off, washed with diethyl ether then dried in a dessicator in the presence of phosphorous pentoxide to provide an orange solid which was purified by chromatography on silica gel eluting with 0-1.5% (9:1 MeOH/20 M NH₃) in dichloromethane to yield the title compound as a white solid. ESMS *m*/*z* 336/338 (MH⁺). ¹H NMR (CDCl₃) δ 8.34(d, 1H), 8.31 (s, 1H), 7.48 (d, 1H), 4.37 (q, 2H), 4.17 (t, 2H), 3.03 (t, 2H), 2.23 (tt, 2H), 1.40 (t, 3H).

### Intermediate 16: Ethyl 9-(3-tert-butoxycarbonylamino-prop-1-ynyl)-1-oxo-6,7-dihydro-1H,5H-pyrido [3,2,1-ij] quinoline-2-carboxylate

A yellow suspension of palladium acetate (73mg, 0.32 mmol) and triphenylphosphine (191mg, 0.72mmol) in dry tetrahydrofuran (6mL) under argon was cooled to 0°C. A solution of n-butyllithium (2.5M in hexanes, 284µL) was added dropwise and after 15 minutes the dark green suspension is warmed to room temperature for 15 minutes. This suspension is then cannulated under argon into a white suspension of **Intermediate 15** (337mg, 1mmol), copper iodide (84mg, 0.44mmol) and *t*-butoxycarbonylpropargylamine (198mg, 1.28 mmol) in diethylamine (6mL). The brown suspension is warmed to 45°C for 2 hours then filtered off and preabsorbed on silica gel. Chromatography on silica gel eluting with 0-5% (9:1 MeOH/20 M NH₃) in dichloromethane provided the title compound as a brown oil. ESMS *m*/*z* 411 (MH⁺). ¹H NMR (CDCl₃) δ 8.23(s, 1H), 8.12 (d, 1H), 7.29 (d, 1H), 5.1 (m, 1H), 4.35 (q, 2H), 4.15 (m, 2x2H), 2.97 (t, 2H), 2.19 (tt, 2H), 1.49 (s, 9H), 1.38 (t, 3H).

### Intermediate 17: Ethyl 9-(3-tert-butoxycarbonylamino-propyl)-1-oxo-6,7-dihydro-1H,5H-pyrido [3,2,1-ij] guinoline-2-carboxylate

**Intermediate 16** (318 mg, 0.77 mmol) was dissolved in dichloromethane (50 mL), treated with 10% palladium on carbon (200 mg) and hydrogenated at room temperature and atmospheric pressure overnight. The reaction mixture was filtered and concentrated to provide a brown oil which was purified by chromatography on silica gel eluting with 0-1% (9:1 MeOH/20 M NH₃) in dichloromethane to yield the title compound as a brown oil. ESMS *m*/*z* 415 (MH⁺). ¹H NMR (CDCl₃) δ 8.34(s, 1H), 8.11 (bs, 1H), 7.25 (bs, 1H), 4.60 (m, 1H), 4.37 (q, 2H), 4.17 (t, 2H), 3.13 (q, 2H), 3.02 (t, 2H), 2.71 (t,2H), 2.20 (tt, 2H), 1.85 (tt, 2H), 1.44 (s, 9H), 1.40 (t, 3H).

### Intermediate 18: 9-(3-tert-Butoxycarbonylamino-propyl)-1-oxo-6,7-dihydro-1H,5H-pyrido [3,2,1-ij] quinoline-2-carboxylic acid sodium salt

**Intermediate 17** (240 mg, 0.59 mmol) was dissolved in tetrahydrofuran (3 mL) and treated with 2N aqueous sodium hydroxide (0.32 mL). The solution was heated to 50°C overnight then treated with excess solid carbon dioxide. Evaporation of the solvent gave the title compound as a beige solid. ESMS *mlz* 387 (MH⁺). NMR (DMSO-*d₆*) δ 8.83 (s, 1H), 8.11 (bs, 1H), 7.99 (s, 1H), 7.57 (s, 1H), 6.89 (bt, 1H), 4.41 (bt, 2H), 3.04 (t, 2H), 2.94 (q, 2H), 2.71 (t,2H), 2.13 (m, 2H), 1.74 (m, 2H), 1.37 (s, 9H).

### Intermediate 19: 9-(3-Amino-propyl)-1-oxo-6,7-dihydro-1H,5H-pyrido [3,2,1-ij] quinoline-2-carboxylic acid trifluoroacetate salt

**Intermediate 18** (224 mg, 0.58 mmol) was dissolved in trifluoroacetic acid (3 mL). After 0.5 h at room temperature the reaction mixture was concentrated to provide the title compound as a beige solid. ESMS *m*/*z* 287 (MH⁺). NMR (MeOD-*d₄*) δ 8.83 (s, 1H), 8.15 (d, 1H), 7.62 (d, 1H), 4.43 (t, 2H), 3.14 (t, 2H), 2.98 (t, 2H), 2.89 (t,2H), 2.66 (tt, 2H), 2.05 (tt, 2H).

### Intermediate 20: Diethyl 2-((2,3-dihydro-indol-1-ylmethylene)malonate

Using a similar procedure to that described in **Intermediate 13** a mixture of indoline (11.9g, 100mmol) and diethyl ethoxymethylenemalonate (21.62g, 100mmol) at 110°C gave the title compound as a brown oil. ESMS m/z 290 (MH⁺).

### Intermediate 21: 6-Oxo-1,2-dihydro-6H-pyrrolo [3,2,1-ij] quinoline-5-carboxylic acid

Using a similar procedure to that described in **Intermediate 14** a mixture of **Intermediate 20** (28.9g, 100mmol) and polyphosphoric acid (85g) at 130°C gave the title compound as a brown oil. ¹H NMR (DMSO-*d₆*) δ 15.6 (s,1H), 9.11 (s, 1H), 7.97 (dd, 1H), 7.78 (dd, 1H), 7.57 (dd, 1H), 4.77 (t, 2H), 3.57 (t, 2H).

### Intermediate 22 : Ethyl 6-oxo-1,2-dihydro-6H-pyrrolo [3,2,1-ij] quinoline-5-carboxylate

**Intermediate 21** (900mg, 4.19mmol) was solubilised in warm dimethylformamide (50 mL) then potassium carbonate (2.89g, 20.95mmol) and iodoethane (3.35 mL, 41.9mmol) were added. The brown suspension was stirred at 70°C for 3 hours then the reaction mixture was concentrated. The residue was taken up in methanol, the solid filtered off and the filtrate preabsorbed on silica gel. Purification by chromatography on silica gel eluting with 0-5% (9:1 MeOH/20 M NH₃) in dichloromethane provided the title compound as a beige solid. ESMS *m*/*z* 266 (MNa⁺). ¹H NMR (CDCl₃) δ 8.57 (s, 1H), 8.08(dd, 1H), 7.46 (dd, 1H), 7.32 (dd, 1H), 4.55 (t, 2H), 4.38 (q, 2H), 3.57 (t, 2H), 1.41 (t, 3H).

### Intermediate 23: Ethyl 8-bromo-6-oxo-1,2-dihydro-6H-pyrrolo [3,2,1-ij] quinoline-5-carboxylate

Using a similar procedure to that described in **Intermediate 15** a mixture of **Intermediate 22** (320mg, 1.3mmol), acetic acid (3ml) and bromine (222µL, 4.34mmol) provided the title compound as a yellow solid. ESMS *mlz* 322/324 (MH⁺). ¹H NMR (CDCl₃) δ 8.49(s, 1H), 8.15 (bs, 1H), 7.53 (bs, 1H), 4.56 (t, 2H), 4.36 (q, 2H), 3.56 (t, 2H), 1.39 (t, 3H).

### Intermediate 24: Ethyl 8-(3-tert-butoxycarbonylamino-prop-1-ynyl)-6-oxo-1,2-dihydro-6H-pyrrolo [3,2,1-ij] quinoline-5-carboxylate

Using a similar procedure to that described in **Intermediate 16** a mixture of palladium acetate (158mg, 0.69 mmol), triphenylphosphine (415mg, 1.56mmol), tetrahydrofuran (13mL), n-butyllithium (1.5M in hexanes, 968µL), **Intermediate 23** (700mg, 2.17mmol), copper iodide (182mg, 0.96mmol), *t*-butoxycarbonylpropargylamine (430mg, 2.77 mmol) and diethylamine (13mL) provided the title compound as a brown solid. ESMS *m*/*z* 397 (MH⁺). ¹H NMR (CDCl₃) δ 8.43(s, 1H), 7.98 (bs, 1H), 7.36 (bs, 1H), 4.95 (m, 1H), 4.53 (t, 2H), 4.36 (q, 2H), 4.17 (m, 2H), 3.50 (t, 2H), 1.48 (s, 9H), 1.39 (t, 3H).

### Intermediate 25: Ethyl 8-(3-tert-butoxycarbonylamino-propyl)-6-oxo-1,2-dihydro-6H-pyrrolo [3,2,1-ij] quinoline-5-carboxylate

Using a similar procedure to that described in **Intermediate 17, Intermediate 24** (396 mg, 1 mmol), dichloromethane (80 mL) and 10% palladium on carbon (400 mg) provided the title compound as a yellow oil. ESMS *m*/*z* 401 (MH⁺). ¹H NMR (CDCl₃) δ 8.54(s, 1H), 7.88 (bs, 1H), 7.32 (bs, 1H), 4.57 (m, 1H), 4.54 (t, 2H), 4.37 (q, 2H), 3.54 (t, 2H), 3.14 (td, 2H), 2.75 (t, 2H), 1.83 (tt, 2H), 1.44 (s, 9H), 1.40 (t, 3H).

### Intermediate 26: 8-(3-tert-Butoxycarbonylamino-propyl)-6-oxo-1,2-dihydro-6H-pyrrolo [3,2,1-ij] quinoline-5-carboxylic acid sodium salt

Using a similar procedure to that described in **Intermediate 18, Intermediate 25** (290 mg, 0.72 mmol), tetrahydofuran (3 mL), dioxan (3 mL) and 2N aqueous sodium hydroxide (800 uL) heated at 60°C provided the title compound as a beige solid. ESMS *m*/*z* 373 (MH⁺). ¹H NMR (DMSO-*d₆*) δ 8.80 (bs, 1H), 7.68 (bs, 1H), 7.48 (bs, 1H), 6.85 (bt, 1H), 4.62 (bt, 2H), 3.51 (t, 2H), 2.95 (td, 2H), 2.73 (t,2H), 1.71 (tt, 2H), 1.34 (s, 9H).

### Intermediate 27: 8-(3-Amino-propyl)-6-oxo-1,2-dihydro-6H-pyrrolo [3,2,1-ij] quinoline-5-carboxylic acid 2,2,2-trifluoroacetate salt

Using a similar procedure to that described in **Intermediate 19, Intermediate 26** (285 mg, 0.72 mmol) and trifluoroacetic acid (3mL) provided after FLEX purification the title compound as a pink solid. ESMS *m*/*z* 273 (MH⁺). ¹H NMR (DMSO-*d₆*) δ 15.65 (bs, 1H), 9.07 (s, 1H), 7.85 (bs, 3H), 7.81 (bs, 1H), 7.66 (bs, 1H), 4.77 (t, 2H), 3.55 (hidden t, 2H), 2.83 (m, 4H), 1.91 (tt, 2H).

### Intermediate 28a: 2'-O-Acetyl-O-(9E)-1-(methyloxy)-2-{[(methyloxy)methyl]oxy}ethanoximino erythromycin A

Using a similar procedure to that described in **Intermediate 10a**, *O*-(9E)- 1-(methyloxy)-2-{[(methyloxy)methyl]oxy}ethanoximino erythromycin A (6.25 g, 7.47 mmol) gave the title compound as a white solid. ESMS *m*/*z* 880 [MH⁺].

### Intermediate 28b: 2'-O-Acetyl-(9E)-1-(methyloxy)-2-{[(methyloxy)methyl]oxy}ethano oximino-4"-O-propenoyl erythromycin A

Using a similar procedure to that described in **Intermediate 3, Intermediate 28a** (6.57 g, 7.47 mmol) gave the title compound as a white solid. ESMS *m*/*z* 934 [MH⁺].

### Intermediate 28c: O-(9E)-1-(Methyloxy)-2-{[(methyloxy)methyl]oxy}ethano oximino-4"-O-propenoyl erythromycin A

Using a similar procedure to that described in **Intermediate 4, Intermediate 28b** (5.45 g, 5.84 mmol) gave the title compound as a white solid. ESMS *m*/*z* 892 [MH⁺].

### Intermediate 29a: 2'-O-Acetyl-O-(9E)-acetylhydroximino erythromycin A

Using a similar procedure to that described in **Intermediate 10a,** *O*-(9E)-hydroximino erythromycin A (*Tetrahedron Lett.,* 1967:1645, **1967**) (1.63 g, 1.96 mmol) gave the title compound as a white solid. ESMS *m*/*z* 834 [MH⁺].

### Intermediate 29b: 2'-O-Acetyl-(9E)-acetylhydroximino-4"-O-propenoyl erythromycin A

Using a similar procedure to that described in **Intermediate 3, Intermediate 29a** (1.20 g, 1.35 mmol) gave the title compound as a white solid. ESMS *m*/*z* 888 [MH⁺].

### Intermediate 29c: O-(9E)-Oximino-4"-O-propenoyl erythromycin A

Using a similar procedure to that described in **Intermediate** 4, **Intermediate 29b** (1.00 g, 1.24 mmol) gave the title compound as a white solid. ESMS *m*/*z* 804 [MH⁺].

### General Procedure for the Preparation of Quinolone Esters 30(a-p)

A solution of 6-[3-({[(1,1-dimethylethyl)oxy]carbonyl}amino)propyl]-1-ethyl-4-oxo-1,4-dihydro-3-quinolinecarboxylic acid (0.85 g, 2.27 mmol) and potassium carbonate (0.63 g, 4.54 mmol) in DMF (15 mL) at 60°C was treated with the requisite alkylating agent (2 equivs). The reaction was assayed by LC/MS. Once complete, the mixture was cooled and the DMF evaporated and the residue partioned between water and dichloromethane. The organic phase was separated, dried and evaporated. Chromatography over silica gel eluting with dichloromethane containing an increasing concentration of methanol/ammonium hydroxide gave the *N*-Boc protected intermediate. After treatment with TFA (1 mL) and evaporation the amine trifluoroacetate salts **30(a-p**), described below were obtained.

### Intermediate 30a: 6-(3-Aminopropyl)-1-ethyl-4-oxo-1,4-dihydroquinoline-3-carboxylic acid ethyl ester

ESMS *m*/*z* 303 [MH⁺].

### Intermediate 30b: 6-(3-Aminopropyl)-1-ethyl-4-oxo-1,4-dihydroquinoline-3-carboxylic acid butyl ester

ESMS *m*/*z* 431 [MH⁺].

### Intermediate 30c: 6-(3-Aminopropyl)-1-ethyl-4-oxo-1,4-dihydroquinoline-3-carboxylic acid pivaloyloxymethyl ester

ESMS *m*/*z* 389 [MH⁺].

### Intermediate 30d: 6-(3-Aminopropyl)-1-ethyl-4-oxo-1,4-dihydroquinoline-3-carboxylic acid 2-(1-N-piperidinyl)ethyl ester

ESMS *m*/*z* 386 [MH⁺].

### Intermediate 30e: 6-(3-Aminopropyl)-1-ethyl-4-oxo-1,4-dihydroguinoline-3-carboxylic acid 2-methoxyethyl ester

ESMS *m*/*z* 333 [MH⁺].

### Intermediate 30f: 6-(3-Aminopropyl)-1-ethyl-4-oxo-1,4-dihydroquinoline-3-carboxylic acid 2-(N,N-dimethylaminocarbonyl)methyl ester

ESMS *m*/*z* 360 [MH⁺].

### Intermediate 30g: 6-(3-Aminopropyl)-1-ethyl-4-oxo-1,4-dihydroquinoline-3-carboxylic acid 2-(1-N-morphilino)ethyl ester

ESMS *m*/*z* 388 [MH⁺].

### Intermediate 30h: 6-(3-Aminopropyl)-1-ethyl-4-oxo-1,4-dihydroquinoline-3-carboxylic acid (ethoxycarbonylmethylcarbamoyl)methyl ester

ESMS *m*/*z* 418 [MH⁺].

### Intermediate 30i: 6-(3.Aminopropvl)-1-ethvl-4-oxo-1,4-dihvdroquinoline-3-carboxylic acid i-propyl ester

ESMS *mlz* 317 [MH⁺].

### Intermediate 30i: 6-(3-Aminopropyl)-1-ethyl-4-oxo-1,4-dihydroctuinoline-3-carboxylic acid i-butyl ester

ESMS *mlz* 331 [MH⁺].

### Intermediate 30k: 6-(3-Aminopropyl)-1-ethyl-4-oxo-1,4-dihydroquinoline-3-carboxylic acid allyl ester

ESMS *m*/*z* 315 [MH⁺].

### Intermediate 30m: 6-(3-Aminopropyl)-1-ethyl-4-oxo-1,4-dihydroquinoline-3-carboxylic acid cyclopropylmethyl ester

ESMS *mlz* 329 [MH⁺].

### Intermediate 30n: 6-(3-Aminopropyl)-1-ethyl-4-oxo-1,4-dihydroquinoline-3-carboxylic acid 3-butenyl ester

ESMS *m*/*z* 329 [MH⁺].

### Intermediate 30p: 6-(3-Aminopropyl)-1-ethyl-4-oxo-1,4-dihydroquinoline-3-carboxylic acid 2-butynyl ester

ESMS *m*/*z* 327 [MH⁺].

### Intermediate 31: 6-(3-Aminopropyl)-1,4-dihydro-1-methyl-4-oxo-quinoline-3-carboxylic acid trifluoroacetate salt

### a) 6-(3-t-Butoxycarbonylamino-prop-1-ynyl)-1,4-dihydro-4-oxo-quinoline-3-carboxylic acid ethyl ester.

Using a similar procedure to that described in **Intermediate 1a**, a mixture of 1,4-dihydro-6-iodo-4-oxo-quinoline-3-carboxylic acid ethyl ester (J. Tucker; V. Vaillancourt; J. Strohbach; K. Romines; M. Schnute; M. Cudahy; S. Thaisrivongs and S. Turner, WO 99/32450) (1.97 g, 5.73 mmol) and *N-t*-butoxycarbonylpropargylamine (1.34 g, 8.6 mmol) in *N,N*-dimethylformamide (50 mL) at 57°C gave the title compound as a cream solid; ESMS *m*/*z* 371 [M+H]⁺.

### b) 6-(3-t-Butoxycarbonylaminopropyl)-1,4-dihydro-4-oxo-quinoline-3-carboxylic acid ethyl ester.

Using a similar procedure to that described in **Intermediate 1b, Intermediate 31a** ( 1.00 g, 2.71 mmol) in dichloromethane:methanol 3:1 (100 mL) gave the title compound as a tan solid; ESMS *m*/*z* 375 [M+H]⁺.

### c) 6-(3-t-Butoxycarbonylaminopropyl)-1,4-dihydro-1-methyl-4-oxo-quinoline-3-carboxylic acid ethyl ester.

To a mixture of **Intermediate 31b** (0.496 g, 1.32 mmol), and potassium carbonate (0.274 g, 1.98 mmol) in *N,N*-dimethylformamide (5 mL) was added iodomethane (0.17 mL, 2.65 mmol). After 4.5 h the mixture was diluted with ethyl acetate, filtered, then concentrated *in vacuo.* The residue was taken up in water, extracted with ethyl acetate, then the organic layers combined, dried (MgSO₄), filtered, and concentrated *in vacuo* to give the title compound as a cream solid; ESMS *m*/*z* 389 [M+H]⁺.

### d) 6-(3-t-Butoxycarbonylaminopropyl)-1,4-dihydro-1-methyl-4-oxo-quinoline-3-carboxylic acid.

A solution of **Intermediate 31c** (0.494 g, 1.27 mmol) in tetrahydrofuran (8 mL) was treated with 0.2 N aqueous sodium hydroxide (7.6 mL). After 29 h the mixture was concentrated *in vacuo.* The resulting residue was taken up in water, treated with excess solid carbon dioxide, and filtered to give the title compound as a cream solid; ESMS *m*/*z* 361 [M+H]⁺.

### e) 6-(3-Aminopropyl)-1,4-dihydro-1-methyl-4-oxo-quinoline-3-carboxylic acid trifluoroacetate salt.

A solution of **Intermediate 31d** (0.388 g, 1.08 mmol) in dichloromethane (6 mL) was treated with trifluoroacetic acid (2 mL). After 35 min the solvent was removed *in vacuo,* the residue taken up in toluene (20 mL), the mixture concentrated *in vacuo*, then the residue taken up in dichloromethane (20 mL), and concentrated *in vacuo* to give the title compound as a cream solid; ESMS *m*/*z* 261 [M+H]⁺.

### Intermediate 32: 6-(3-Aminopropyl)-1,4-dihydro-1-(2-methoxyethyl)-4-oxo-auinoline-3-carboxylic acid trifluoroacetate salt

### a) 6-(3-t-Butoxycarbonylaminopropyl)-1,4-dihydro-1-(2-methoxyethyl)-4-oxo-quinoline-3-carboxylic acid ethyl ester.

To a mixture of **Intermediate 31b** (0.491 g, 1.31 mmol), sodium carbonate (0.209 g, 1.97 mmol), and sodium iodide (0.235 g, 1.57 mmol) in *N,N*-dimethylformamide (5 mL) was added 1-bromo-2-methoxyethane (0.15 mL, 1.57 mmol). After stirring at r.t. for 17.5 h the mixture was heated to 67°C for a further 31 h. Additional sodium carbonate (0.050 g, 0.47 mmol) and 1-bromo-2-methoxyethane (0.04 mL, 0.43 mmol) was then added and heating continued for a further 65 h. The mixture was then diluted with ethyl acetate, filtered, and concentrated *in vacuo.* This residue was taken up in water, extracted with ethyl acetate, then the organic layers combined, dried (MgSO₄), filtered, and concentrated *in vacuo* to give a residue which was purified by flash chromatography (silica gel, 0-50% ethyl acetate in dichloromethane) to give the title compound as a cream solid; ESMS *m*/*z* 433 [M+H]⁺.

### b) 6-(3-t-Butoxycarbonylaminopropyl)-1,4-dihydro-1-(2-methoxyethyl)-4-oxo-quinoline-3-carboxylic acid.

Using a similar procedure to that described in **Intermediate 31d, Intermediate 32a** (0.287 g, 0.66 mmol) gave the title compound as a cream solid; ESMS *m*/*z* 405 [M+H]⁺.

### c) 6-(3-Aminopropyl)-1,4-dihydro-1-(2-methoxyethyl)-4-oxo-quinoline-3-carboxylic acid trifluoroacetate salt.

Using a similar procedure to that described in **Intermediate 31e, Intermediate 32b** (0.178 g, 0.44 mmol) gave the title compound as a cream solid; ESMS *m*/*z* 305 [M+H]⁺.

### Intermediate 33: 6-(3-Aminopropyl)-1,4-dihydro-1-cyclopropyl-4-oxo-quinoline-3-carboxylic acid trifluoroacetate salt

### a) 6-(3-t-Butoxycarbonylamino-prop-1-ynyl)-1,4-dihydro-1-cyclopropyl-4-oxo-quinoline-3-carboxylic acid ethyl ester.

Using a similar procedure to that described in **Intermediate 1a,** a mixture of 1,4-dihydro-1-cyclopropyl-6-iodo-4-oxo-quinoline-3-carboxylic acid ethyl ester (S. Turner; J. Strohbach; S. Thaisrivongs; V. Vaillancourt; M. Schnute and J. Tucker, WO 00/40561) (0.647 g, 1.69 mmol) and *N*-*t*-butoxycarbonylpropargylamine (0.393 g, 2.53 mmol) in acetonitrile (15 mL) at 50°C gave the title compound as a cream solid; ESMS *m*/*z* 411 [M+H]⁺.

### b) 6-(3-t-Butoxycarbonylaminopropyl)-1,4-dihydro-1-cyclopropyl-4-oxo-quinoline-3-carboxylic acid ethyl ester.

Using a similar procedure to that described in **Intermediate 1b, Intermediate 33a** (0.450 g, 1.10 mmol) in dichloromethane (20 mL) gave the title compound as a cream solid; ESMS *m*/*z* 415 [M+H]⁺.

### c) 6-(3-t-Butoxycarbonylaminopropyl)-1,4-dihydro-1-cyclopropyl-4-oxo-quinoline-3-carboxylic acid.

Using a similar procedure to that described in **Intermediate 32b, Intermediate 33b** (0.447 g, 1.08 mmol) gave the title compound as a cream solid; ESMS *m*/*z* 387 [M+H]⁺.

### d) 6-(3-Aminopropyl)-1,4-dihydro-1-cyclopropyl-4-oxo-quinoline-3-carboxylic acid trifluoroacetate salt.

Using a similar procedure to that described in **Intermediate 32c, Intermediate 33c** (0.392 g, 1.01 mmol) gave the title compound as a cream solid; ESMS *m*/*z* 287 [M+H]⁺.

### Intermediate 34: 6-Prop-2-ynyloxy-hexanoic acid ethyl ester

To a solution of 6-hydroxy-hexanoic acid ethyl ester (0.5 mL, 3.1 mmol) in THF (5 mL) was added tetrabutylammonium iodide (57.2 mg, 0.155 mmol), sodium iodide (69.7 mg, 0.465 mmol), 3-bromo-propyne (518 µl, 4.65 mmol) and potassium hydroxide (173.9 mg, 3.1 mmol) and the mixture was stirred for 5 hours at room temperature. The solvent was evaporated and the residue extracted with EtOAc and water (2x20 mL). The organic layer was washed with NaCl (2x20 mL), dried over K₂CO₃ and evaporated *in vacuo* yielding (0.347 g) of the title product.
MS (ES) m/z: [MH]⁺ 199.27.
¹H NMR (500 MHz, DMSO) δ ppm: 4.09 (2H, CH₂), 4.04 (2H, CH₂), 3.40 (2H, CH₂), 3.37 (H, C≡CH), 2.27 (2H, CH₂), 1.48 (4H, 2xCH₂), 1.30 (2H, CH₂), 1.17 (3H, CH₃).
¹³C NMR (300 MHz, DMSO) δ ppm: 177.40, 85.14, 81.44, 73.57, 64.26, 61.89, 37.99, 33.21, 29.80, 28.88, 18.74.

### Intermediate 35: 6-[3-(5-Ethoxycarbonyl-pentyloxy)-prop-1-ynyl]1-ethyl-4-oxo-1,4-dihydro-qunoline-3-carboxylic acid ethyl ester

**Intermediate 2a** (312 mg, 0.84 mmol), copper(I) iodide (16 mg, 0.084 mmol) and triethylamine (4.072 mL, 29.4 mmol) were suspended in dry acetonitrile (10 mL). The suspension was heated to 50°C and N₂ bubbled through. After 20 min, dichlorobis(triphenylphosphine) palladium (II) (18 mg, 0.0252 mmol) and **Intermediate 34** (347 mg, 1.75 mmol) were added and the suspension was stirred at 50 °C for 4 hours. The solvent was evaporated and the residue was extracted with EtOAc and water (2x50 mL). The organic layer was washed with NaCl and NaHCO₃ (2x50 mL), dried over K₂CO₃ and evaporated *in vacuo* yielding (476 mg) of the title product.
MS (ES) m/z: [MH]⁺ 442.25.
¹H NMR (500 MHz, DMSO) δ ppm: 8.70 (1H, Q), 8.23 (1H, Q), 7.82 (2x1H, Q), 4.43 (2H, Q-N-CH₂-CH₃), 4.40 (2H, CH₂), 4.23 (2H,Q-CO₂-CH₂CH₃), 4.03 (2H, CH₂), 3.52 (2H, CH₂), 2.29 (2H, CH₂), 1.55 (4H, 2xCH₂), 1.36 (3H, Q-N-CH₂-CH₃), 1.34 (2H, CH₂), 1.29 (Q-CO2-CH₂-CH₃), 1.16 (3H, CH₃).
¹³C NMR (300 MHz, DMSO) δ ppm: 172.73, 171.87, 164.33, 149.20, 138.32, 134.88, 129.34, 128.15, 118.20, 117.94, 110.55, 87.26, 84.29, 69.11, 59.74, 59.54, 57.82, 47.93, 33.37, 28.57, 25.10, 24.17, 14.23, 14.02.

### Intermediate 36: 6-[3-(5-Carboxy-pentyloxy)-prop-1-ynyl]-1-ethyl-4-oxo-1,4-dihydroquinoline-3-carboxylic acid

To a solution of **Intermediate 35** (476 mg, 1.08 mmol) in THF (5.55 mL) was added a solution of sodium hydroxide (185 mg, 4.62 mmol) in water (5.5 mL) and the mixture was stirred for 2 hours at 80°C and for 12 hours at room temperature. The reaction mixture was extracted with EtOAc and water (2x20 mL). The pH value of water layer was adjusted from 9.8 to 5.2 by adding of 2N HCl and the layer was extracted with DCM. The organic layer was evaporated *in vacuo* yielding (184 mg) of the title product.
MS (ES) m/z: [MH]⁺ 386.19.

### Intermediate 37: 2'-Acetyl-4"-O-{6-[3-(3-carboxy-1-ethyl-4-oxo-1,4-dihydro-guinolin-6-yl)-prop-2-ynyloxy]-hexanoyl}-azithromycin

To a solution of **Intermediate 36** (184 mg, 0.48 mmol) in dry DMF (6 mL), which was cooled to 0°C and N₂ bubbled through, was added 1-(3-dimethylaminopropyl)-3-ethylcarbodiimide hydrochloride (131.8 mg, 0.69 mmol) and a solution of 2'OAc-azithromycin (237.3 mg, 0.3 mmol) in dry DCM (3 mL) was added dropwise. DMAP (61.6 mg, 0.504 mmol) was then added to the reaction mixture. The suspension was stirred at first for 3 hours at 0°C and then gradually up to room temperature for 24 hours. The solvent was evaporated and residue was extracted with EtOAc and water (2x20 mL). The organic layer was dried over K₂CO₃ and evaporated *in vacuo* yielding (325 mg) of the title product. MS (ES) m/z: [MH]⁺ 1158.63.

### Intermediate 38: 4"-O-{6-[3-(3-Carboxy-1-ethyl-4-oxo-1,4-dihydro-quinolin-6-yl)-Prop-2-ynyloxy]-hexanoyl}-azithromycin

A solution of **Intermediate 37** (325 mg, 0.28 mmol) in methanol (40 mL) was heated to 55 °C for 12 hours. The solvent was evaporated and the residue was purified by column chromatography (DCM: MeOH: NH₃= 90:5:0.5) yielding (106 mg) crude yellow product.
MS (ES) m/z: [MH]⁺ 1117.08.

### Intermediate 39: 2'-Acetyl-4"-O-{6-[3-(3-carboxy-1-ethyl-4-oxo-1,4-dihydro-quinolin-6-yl)-prop-2-ynyloxy]-hexanoyl}-clarithromycln

Using a similar procedure to that described in **Intermediate 37, Intermediate 36** (148 mg, 0.38 mmol) and 2'OAc-clarithromycin (233 mg, 0.3 mmol) in dry DCM (3 mL) gave the title product (302 mg).

### Intermediate 40: 4"-O-{6-[3-(3-Carboxy-1-ethyl-4-oxo-1,4-dihydro-quinolin-6-yl)-prop-2-ynyloxy]-hexanoyl}-clarithromycin

Using a similar procedure to that described in **Intermediate 38, Intermediate 39** (302 mg, 0.26 mmol) gave the title product as a yellow solid (78mg).
MS (ES) m/z: [MH]⁺ 1115.55.

### Intermediate 41: 2'-Acetyl-4"-O-{6-[3-(3-carboxy-1-ethyl-4-oxo-1,4-dihydro-quinolin-6-yl)-prop-2-ynyloxy]-hexanoyl}-11-O-Me-azlthromycin

Using a similar procedure to that described in **Intermediate 37, Intermediate 36** (280 mg, 0.73 mmol) and 2'OAc-11-O-Me-azithromycin (Kobrehel *et al., J. Antibiotics,* **1982,** 45, 527) (489 mg, 0.61 mmol) in dry DCM (5 mL) gave the title product (250 mg).

### Intermediate 42: 4"-O-{6-[3-(3-Carboxy-1-ethyl-4-oxo-1,4-dihydro-quinolin-6-yl)-prop-2-ynyloxy]-hexanoyl}-11-O-Me-azithromycin

Using a similar procedure to that described in **Intermediate 38, Intermediate 41** (250 mg, 0.21 mmol) gave the title product as a yellow solid (141 mg).
MS (ES) m/z: [MH]⁺ 1130.56.

### Example 1: 4"-O-{3-[3-(3-Carboxy-1,4-dihydro-1-ethyl-6-fluoro-4-oxo-7-guinolinyl) propylamino]propionyl}-6-O-methylerythromycin A

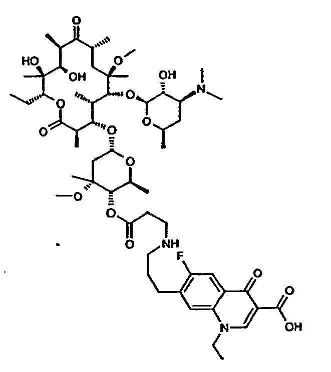

### a) 2'-O-Acetyl-4"-O-{3-[3-(3-carboxy-1,4-dihydro-1-ethyl-6-fluoro-4-oxo-7-quinolinyl) propylamino]propionyl}-6-O-methylerythromycin A.

A mixture of **Intermediate 6** (0.089 g, 0.1 mmol) and **Intermediate 1** trifluoroacetate salt (0.1 g, 0.25 mmol) in DMSO (3 mL), water (5 drops) and triethylamine (0.127 g, 1.25 mmol) was heated at 80°C. After 3 days additional **Intermediate 6** (0.089 g, 0.1 mmol) was added and the mixture heated for a further 2 days. The mixture was cooled, partitioned between dichloromethane and water and the organic phase dried and concentrated. The residue was chromatographed over silica gel eluting with 0-2.5% (9:1 MeOH/20 M NH₃) in dichloromethane to yield the title compound as a white solid; ESMS m/z 1136 [M+H]⁺.

### b) 4"-O-{3-[3-(3-Carboxy-1,4-dihydro-1-ethyl-6-fluoro-4-oxo-7-quinolinyl) propylamino]propionyl}-6-O-methylerythromycin A.

**Example 1a** (0.04 g, 0.035 mmol) was dissolved in methanol (5 mL) and heated to 50°C for 24 h. The reaction mixture was concentrated to provide the title compound as a beige solid; ESMS m/z 1094 [M+H]⁺.

### Example 2: 4"-O-{3-[3-(3-Carboxy-1,4-dihydro-1-ethyl-6-fluoro-4-oxo-7-quinolinyl) propylamino]propionyl}-azithromycin tris trifluoroacetate salt

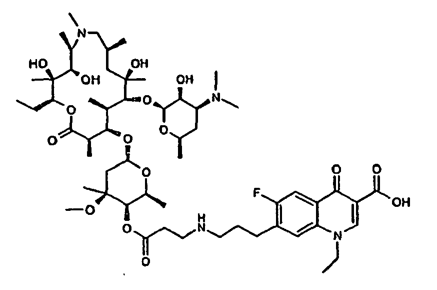

**Intermediate 1** (0.117 g, 0.34 mmol) and **Intermediate 5** (0.273 g, 0.34 mmol) in methanol (3 mL) were heated at 64°C overnight. The reaction mixture was chromatographed over silica gel eluting with 0-10% (9:1 MeOH/20 M NH₃) in dichloromethane followed by reverse phase HPLC purification to yield the title compound as a white solid; ESMS m/z 1095 [M+H]⁺.

### Example 3: 4"-O-{3-[3-(3-Carboxy-1,4-dihydro-1-ethyl-6-fluoro-4-oxo-7-quinolinyl propylamino]propionyl}-azithromycin-11,12-carbonate

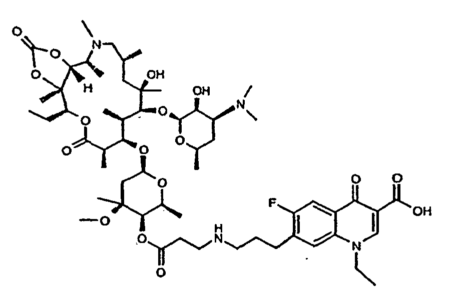

**Intermediate 4** (0.282 g, 0.34 mmol) and **Intermediate 1** (0.117 g, 0.34 mmol) in isopropanol (4 mL), water (1 drop) and triethylamine (0.069 g, 0.68 mmol) were heated at 80°C. After 3 days DMSO (2 mL) was added and the mixture heated overnight, the reaction mixture was diluted with methanol and purified by reverse phase HPLC followed by chromatography over silica gel eluting with 0-5% (9:1 MeOH/20 M NH₃) in dichloromethane to yield the title compound as a white solid; ESMS m/z 1121 [M+H]⁺.

### Example 4: 4"-O-{3-[3-(3-Carboxy-1,4-dihydro-1-ethyl-4-oxo-6-quinolinyl) propylamino]propionyl}-6-O-methylerythromycin A

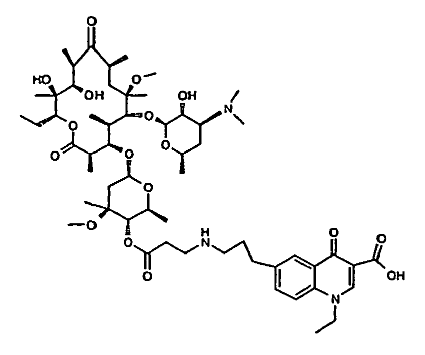

Using a similar procedure to that described in **Example 1a, Intermediate 9** (0.120 g, 0.15 mmol) and **Intermediate 2** (0.058g, 0.15 mmol) gave the title compound as a white solid; ¹H NMR δ (CDCl₃) *inter alia* 4.99 (1H, d, J = 5.0 Hz), 5.06 (1H, d, J = 8.9 Hz), 7.57 (1H, d, J = 8.8 Hz), 7.69 (1H, d x d, J = 2.1 & 8.7 Hz), 8.37 (1H, d, J = 2.0 Hz), 8.77 (1H, s); ESMS m/z 1076 [M+H]⁺.

### Example 5: 4"-O-{3-[3-(3-Carboxy-1,4-dihydro-1-ethyl-4-oxo-6-quinolinyl) propylamino]propionyl}-azithromycin

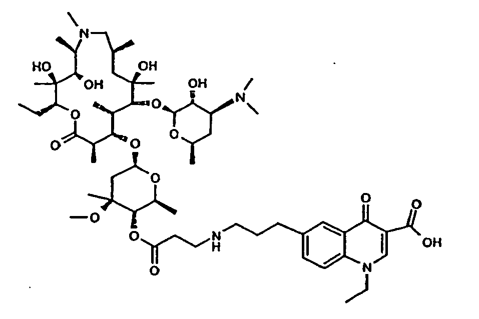

Using a similar procedure to that described in **Example 1a, Intermediate 5** and **Intermediate 2e** (0.082 g, 0.26 mmol) gave the title compound as a white solid; ESMS m/z 1077 [M+H]⁺.

### Example 6: 4"-O-{3-[3-(3-Carboxy-1,4-dihydro-1-ethyl-4-oxo-6-quinolinyl) propylamino]propionyl}-azithromycin-11,12-carbonate

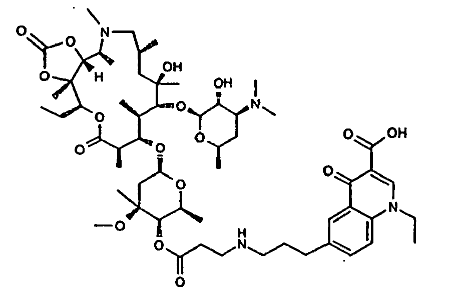

Using a similar procedure to that described in **Example 1a, Intermediate 4** (0.216 g, 0.26 mmol) and **Intermediate 2e** (0.082 g, 0.26 mmol) gave the title compound as a white solid; ¹H NMR δ (CD₃OD) *inter alia* 4.85 (1H, d, J = 6.0 Hz), 5.09 (1H, d, J = 4.4 Hz), 7.78 (1H, d, J = 8.8 Hz), 7.86 (1H, d, J = 8.8 Hz), 8.30 (1H, d, J = 1.6 Hz), 8.84 (1H, s); ESMS m/z 1103 [M+H]⁺.

### Example 7: 4"-O-{3-[3-(3-Carboxy-1,4-dihydro-1-ethyl-4-oxo-6-quinolinyl) propylamino]propionyl}-6-O-methyl-11-desoxv-11-(R)-amino-erythromycin A 11,12-carbamate diformate salt

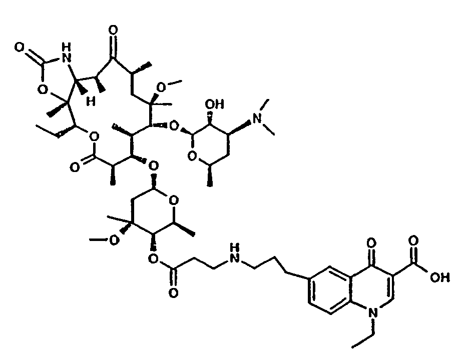

### a) 4"-O-{3-[3-(3-Carboxy-1,4-dihydro-1-ethyl-4-oxo-6-quinolinyl)propylamino] propionyt-2'-O-acetyl-6-O-methyl-11-desoxy-11-(R)-amino-erythromycin A 11,12-carbamate.

Using the procedure described in **Example 1a, Intermediate 8** (0.108 g, 0.125 mmol) and **Intermediate 2e** gave, after chromatography, the title compound as white solid; ESMS m/z 1143 [M+H]⁺. Also isolated was 4"-*O*-{3-[3-(3-carboxy-1,4-dihydro-1-ethyl-4-oxo-6-quinolinyl)propylamino]propionyl-2'-*O*-acetyl-6-*O*-methyl-11-desoxy-11-(R)-amino-erythromycin A 11,12-carbamate methyl ester, obtained as a white solid; ESMS m/z 1157 [M+H]⁺.

### b) 4"-O-{3-[3-(3-Carboxy-1,4-dihydro-1-ethyl-4-oxo-6-quinolinyl)propylamino] propionyl-6-O-methyl-11-desoxy-11-(R)-amino-erythromycin A 11,12-carbamate diformate.

Using the procedure described in **Example 1b, Example 7a** was converted to the title compound. Purification by reverse phase HPLC gave a white solid; ¹H NMR δ (CD₃OD) *inter alia* 4.98 (1H, d, J = 4.8 Hz), 5.05 (1H, d, J = 8.4 Hz), 7.84 (1H, d x d, J = 2.0 & 9.2 Hz), 7.95 (1H, d, J = 8.8 Hz), 8.36 (1H, d, J = 1.6 Hz), 8.97 (1H, s); ESMS m/z 1102 [M+H]⁺.

### Example 8: 4"-O-{3-[3-(3-Carboxy-1,4-dihydro-1-ethyl-4-oxo-6-quinolinyl) propylamino]propionyl}-6-O-methyl-11-desoxy-11-(R)-amino-erythromycin A 11,12-carbamate methyl ester diformate salt

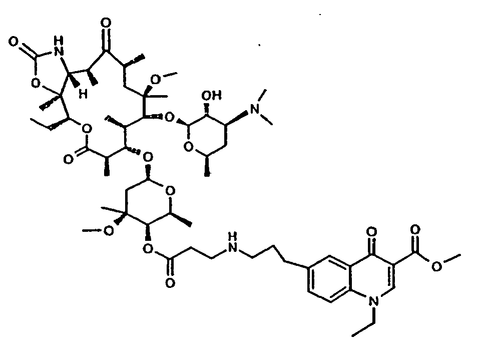

Using the procedure described in **Example 1b,** 4"-O-{3-[3-(3-carboxy-1,4-dihydro-1-ethyl-4-oxo-6-quinolinyl)propylamino]propionyl-2'-*O*-acetyl-6-*O*-methyl-11-desoxy-11-(R)-amino-erythromycin A 11,12-carbamate methyl ester obtained in **Example 7a** was converted to the title compound. Purification by reverse phase HPLC gave a white solid; ESMS m/z 1116[M+H]⁺.

### Example 9: 4"-O-[3-[4-(3-Carboxy-1-ethyl-1,4-dihydro-4-oxo-6-quinolinyl) propylamino]propionyl]-(9E)-O-methoximino erythromycin A

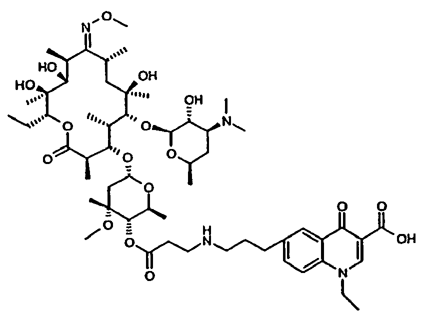

Using a similar procedure to that described for the preparation of **Example 1b, Intermediate 12** (0.14 g, 0.12 mmol) gave the title compound as a white solid. ESMS *m*/*z* 1091 [MH]⁺.

### Example 10: 4"-O-[3-[4-(2-Carboxy-6,7-dihydro-1H,5H-pyrido[3,2,1-ij]-1-oxo-9-quinolinyl)propylamino]propionyl]-6-O-methyl erythromycin A

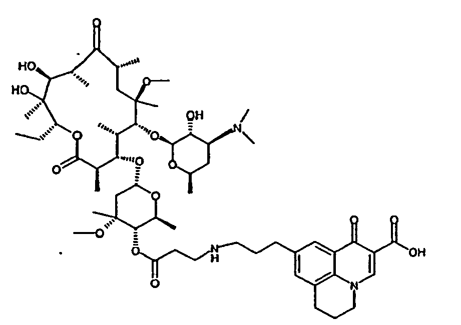

A mixture of **Intermediate 9** (0.116 g, 0.145 mmol) and **Intermediate 19** (0.116 g, 0.29 mmol) in DMSO (1 mL), water (1 drop) and triethylamine (0.13 µL, 0.9 mmol) was heated at 80°C. After 2 days the mixture was cooled and submitted to Mass Directed Auto Prep purification followed by chromatography over silica gel eluting with 0-5% (9:1 MeOH/20 M NH₃) in dichloromethane to yield the title compound as a white solid. ESMS m/z 1089 (MH⁺). NMR (MeOD-*d₄*) δ 8.70 (s, 1H), 8.07 (bs, 1H), 7.55 (bs, 1H), 5.13 (dd, 1H), *inter alia.*

### Example 11: 4"-O-[3-[4-(2-Carboxy-6,7-dihydro-1H,5H-pyrido[3,2,1-ij]-1-oxo-9-guinolinyl)propylamino]propionyl]azithromycin tris formate salt

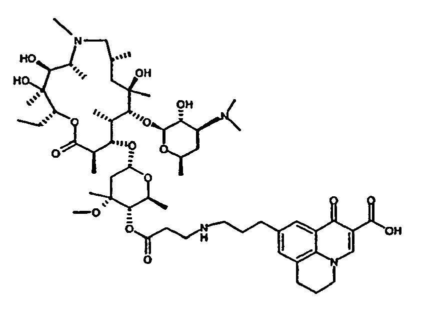

A mixture of **Intermediate 5** (0.088 g, 0.11 mmol) and **Intermediate 19** (0.066 g, 0.165 mmol) in DMSO (1 mL), water (1 drop) and triethylamine (0.072 µL, 0.49 mmol) was heated at 80°C overnight. The mixture was cooled and submitted to Mass Directed Auto Prep purification to yield the title compound as a white solid. ESMS m/z 1090 (MH⁺). NMR (MeOD-*d₄*) δ 8.82 (s, 1H), 8.45 (s, 3H), 8.15 (bs, 1H), 7.63 (bs, 1H), 5.10 (d, 1H), *inter alia.*

### Example 12: 4"-O-[3-[4-(2-Carboxy-6,7-dihydro-1H,5H-pyrido[3,2,1-ij]-1-oxo-9-quinolinyl) propylamino]propionyl]azithromycin-11,12-carbonate

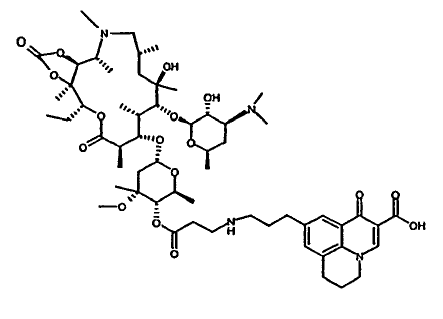

A mixture of **Intermediate 4** (0.120 g, 0.145 mmol) and **Intermediate 19** (0.116 g, 0.29 mmol) in DMSO (1 mL), water (1 drop) and triethylamine (0.13 µL, 0.9 mmol) was heated at 80°C. After 2 days the mixture was cooled and submitted to Mass Directed Auto Prep purification followed by chromatography over silica gel eluting with 0-5% (9:1 MeOH/20 M NH₃) in dichloromethane to yield the title compound as a white solid. ESMS m/z 1116 (MH⁺). NMR (CDCl₃) δ 8.65 (s, 1H), 8.15 (d, 1H), 7.44 (d, 1H), 5.10 (d, 1H), *inter alia.*

### Example 13: 4"-O-[3-[4-(5-Carboxy-1,2-dihydro-6H-pyrrolo[3,2,1-ij]-6-oxo-8-quinolinyl)propylamino]propionyl]-6-O-methyl erythromycin A bis formate salt

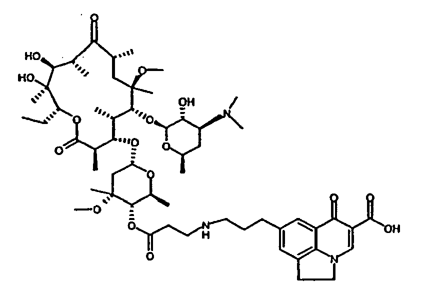

Using a similar procedure to that described in **Example 10** a mixture of **Intermediate 9** (0.160 g, 0.2 mmol) and **Intermediate 27** (0.094 g, 0.24 mmol), DMSO (1 mL), water (1 drop) and triethylamine (0.104 µL, 0.72 mmol) provided after Mass Directed Auto Prep purification the title compound as a beige solid. ESMS m/z 1075 (MH⁺). NMR (MeOD-*d₄*) δ 9.01 (s, 1H), 8.37 (s, 2H), 7.91 (bs, 1H), 7.67 (bs, 1H), 5.15 (dd, 1H), *inter alia.*

### Example 14: 4"-O-[3-[4-(5-Carboxy-1,2-dihydro-6H-pyrrolo[3,2,1-ij]-6-oxo-8-quinolinyl) propylamino]propionyl]azithromycin tris formate salt

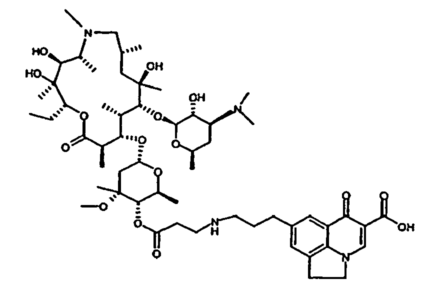

Using a similar procedure to that described in **Example 11** a mixture of **Intermediate 5** (0.051 g, 0.063 mmol), **Intermediate 27** (0.039 g, 0.1 mmol), DMSO (2 mL), water (1 drop) and triethylamine (0.300 µL, 2.04 mmol) provided after Mass Directed Auto Prep purification the title compound as a white solid. ESMS m/z 1076 (MH⁺). NMR (MeOD-*d₄*) δ 9.0 (s, 1H), 8.39 (s, 3H), 7.91 (bs, 1H), 7.67 (bs, 1H), 5.10 (d, 1H), *inter alia.*

### Example 15: 4"-O-[3-[4-(5-Carboxy-1,2-dihydro-6H-pyrrolo[3,2,1-ij]-6-oxo-8-quinolinyl)propylamino]propionyl]azithromycin-11,12-carbonate tris formate salt

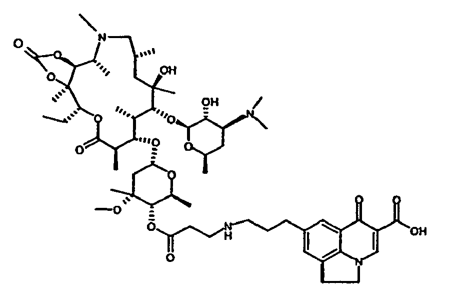

Using a similar procedure to that described in **Example 12** a mixture of **Intermediate 4** (0.1 g, 0.2 mmol), **Intermediate 27** (0.094 g, 0.24 mmol), DMSO (1 mL), water (1 drop) and triethylamine (0.104 µL, 0.72 mmol) provided after Mass Directed Auto Prep purification the title compound as a beige solid. ESMS m/z 1102 (MH⁺). NMR (MeOD-*d₄*) δ 8.93 (s, 1H), 8.87 (s, 1H), 8.39 (s, 3H), 7.66 (s, 1H), 5.09 (d, 1H), *inter alia.*

### Example 16: 4"-O-[3-[4-(2-Carboxy-6,7-dihydro-1H,5H-pyrido[3,2,1-ii]-1-1-oxo-9-quinolinyl) propylamino]propionyl]-(9E)-O-methoximino erythromycin A bisformate

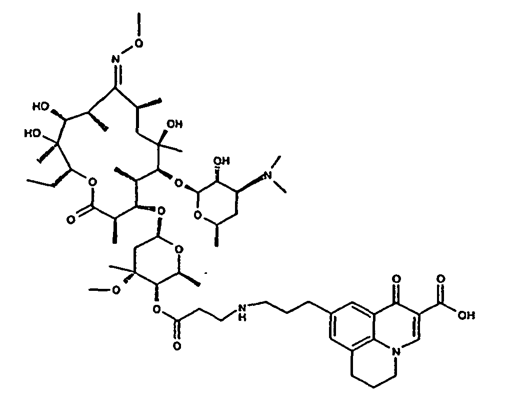

Using a similar procedure to that described in **Example 1a, Intermediate 10c** and **Intermediate 19** gave the title compound as a white solid; ESMS 1104 m/z [M+H]⁺.

### Example 17: 4"-O-[3-[4-(3-Carboxy-1-ethyl-1,4-dihydro-4-oxo-6-quinolinyl) propylamino]propionyl]-(9E)-O-({[2-(methyloxy)ethyl]oxy}methanoximino erythromycin A bisformate

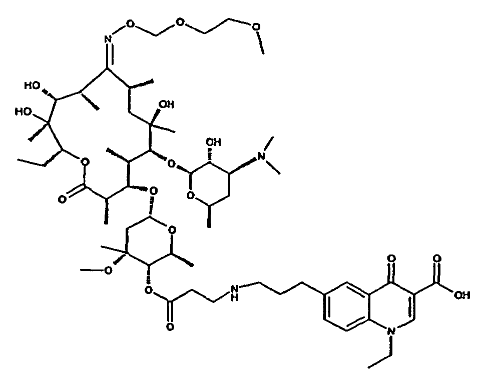

Using a similar procedure to that described in **Example 1a, Intermediate 28c** and **Intermediate 2** gave the title compound as a white solid; ESMS m/z 1166 [M+H]⁺.

### Example 18: 4"-O-[3-[4-(2-Carboxy-6,7-dihydro-1H,5H-pyrido[3,2,1-ij]-1-oxo-9-quinolinyl)propylamino]propionyl]-(9E)-O-({[2-(methyloxy)ethyl]oxy}methanoximino erythromycin A bisformate

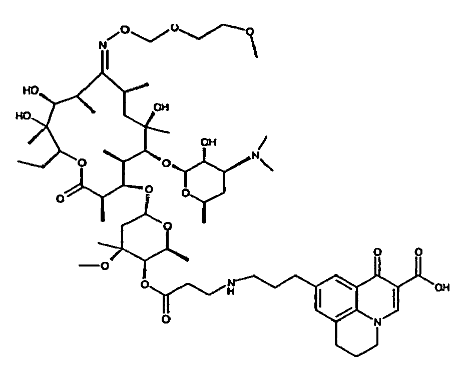

Using a similar procedure to that described in **Example 1a, Intermediate 28c** and **Intermediate 19** gave the title compound as a white solid; ESMS m/z 1178 [M+H]⁺.

### Example 19: 4"-O-[3-[4-(3-Carboxy-1-ethyl-1,4-dihydro-4-oxo-6-quinolinyl) propylamino]propionyl]-(9E)-O-hydroximino erythromycin A

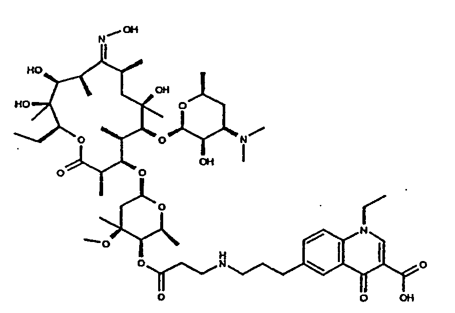

Using a similar procedure to that described in **Example 1a, Intermediate 29b** and **Intermediate 2** gave the title compound as a white solid; ESMS m/z 1078 [M+H]⁺.

### Example 20: 4"-O-[3-[4-(2-Carboxy-6,7-dihydro-1H,5H-pyrido[3,2,1-ij]-1-oxo-9-quinolinyl)propylamino]propionyl]-(9E)-O-hydroximino erythromycin A

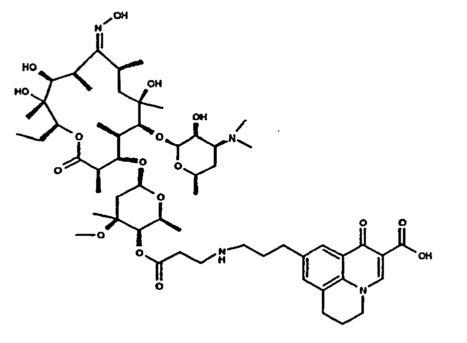

Using a similar procedure to that described in **Example 1a, Intermediate 29c** and **Intermediate 19** gave the title compound as a white solid; ESMS m/z 1090 [M+H]⁺.

### General Procedure for the preparation of Esters of Example 4

The title compounds were prepared as described in **Example 1a** from **Intermediate 9** (0.54 g, 0.64 mmol) and the requisite quinolone 3-carboxylic ester, **Intermediate 30(a-p)** (1.28 mmol). Once complete, as determined by LC/MS, the reaction was cooled and partitioned between water and dichloromethane. The organic layer was separated, dried and evaporated to yield the crude product. Chromatography over silica gel eluting with dichloromethane containing an increasing concentration of methanol/ammonium hydroxide (0 to 10 %) gave the compounds described below.

### Example 21: 4"-O-{3-[3-(3-Carboxy-1,4-dihydro-1-ethyl-4-oxo-6-quinolinyl) propylamino]propionyl}-6-O-methylerythromycin A ethyl ester

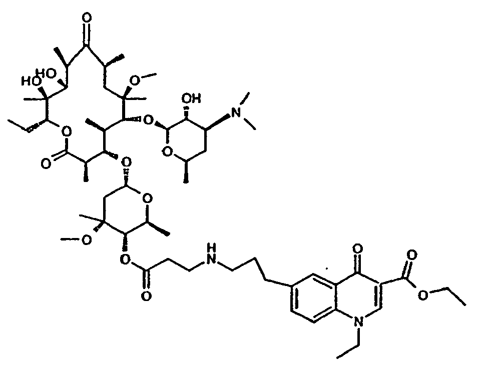

ESMS *m*/*z* 1105 [MH⁺].

### Example 22: 4"-O-{3-[3-(3-Carboxy-1,4-dihydro-1-ethyl-4-oxo-6-quinolinyl) propylamino]propionyl}-6-O-methylerythromycin A n-butyl ester

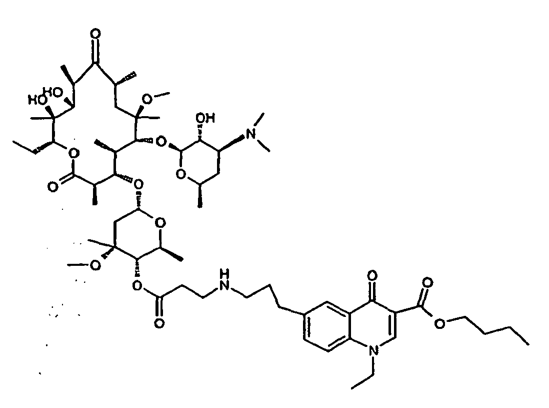

ESMS *m*/*z* 1133 [MH⁺].

### Example 23: 4"-O-{3-[3-(3-Carboxy-1,4-dihydro-1-ethyl-4-oxo-6-quinolinyl) propylamino]propionyl}-6-O-methylerythromycin A pivaloyloxymethyl ester

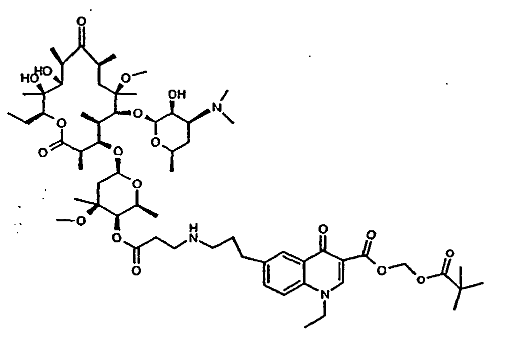

ESMS *m*/*z* 1191 [MH⁺].

### Example 24: 4"-O-{3-[3-(3-Carboxy-1,4-dihydro-1-ethyl-4-oxo-6-quinolinyl) propylamino]propionyl}-6-O-methylerythromycin A 2-(1-N-piperidinyl)ethyl ester

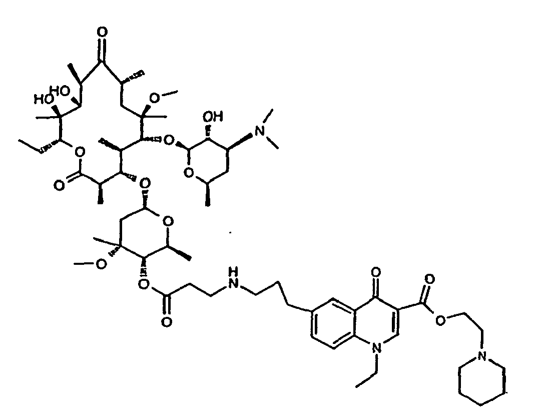

ESMS *m*/*z* 1188 [MH⁺].

### Example 25: 4"-O-{3-[3-(3-Carboxy-1,4-dihydro-1-ethyl-4-oxo-6-quinollnyl) propylamino]propionyl}-6-O-methylerythromycin A 2-methoxyethyl ester

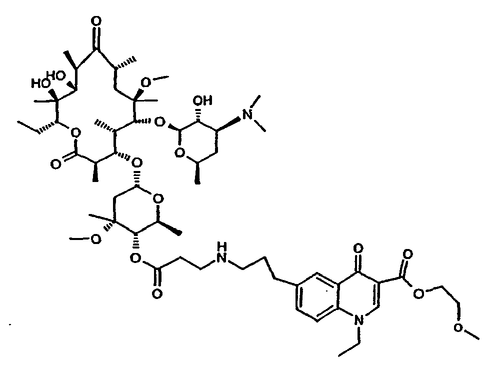

ESMS *m*/*z* 1135 [MH⁺].

### Example 26: 4"-O-{3-[3-(3-Carboxy-1,4-dihydro-1-ethyl-4-oxo-6-quinolinyl) propylamino]propionyl}-6-O-methylerythromycin A 2-(N,N dimethlyaminocarbonyl)methyl ester

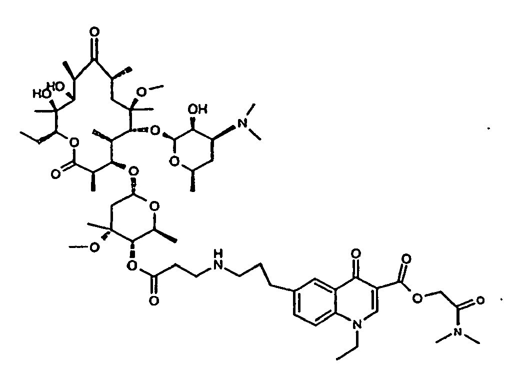

ESMS *m*/*z* 1162 [MH⁺].

### Example 27: 4"-O-{3-[3-(3-Carboxy-1,4-dihydro-1-ethyl-4-oxo-6-quinolinyl) propylamino]propionyl}-6-O-methylervthromycln A 2-(1-N-morphilino)ethyl ester

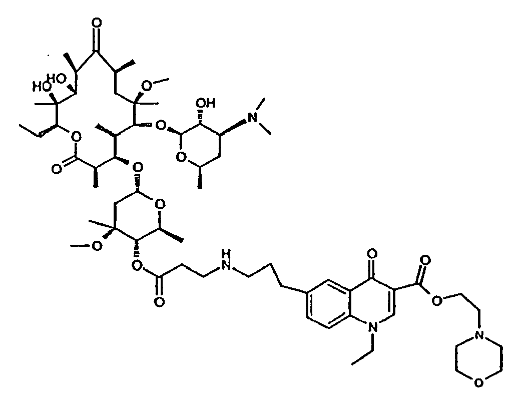

ESMS *m*/*z* 1140 [MH⁺].

### Example 28: 4"-O-{3-[3-(3-Carboxy-1,4-dihydro-1-ethyl-4-oxo-6-quinolinyl) propylamino]propionyl}-6-O-methylerythromycin A 2-(2-(ethoxy)-2-oxoethylaminocarbonyl)ethyl ester

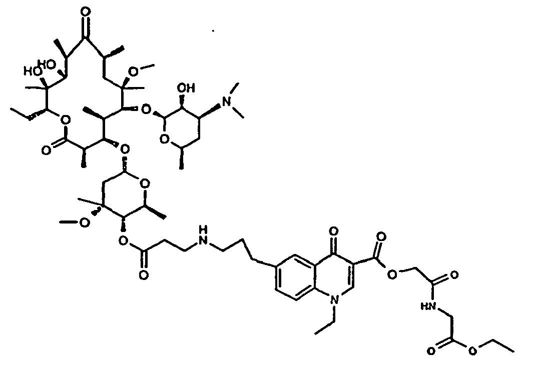

ESMS *m*/*z* 1220 [MH⁺].

### Example 29: 4"-O-{3-[3-(3-Carboxy-1,4-dihydro-1-ethyl-4-oxo-6-quinolinyl) propylamino]propionyl}-6-O-methylerythromycin A i-propyl ester

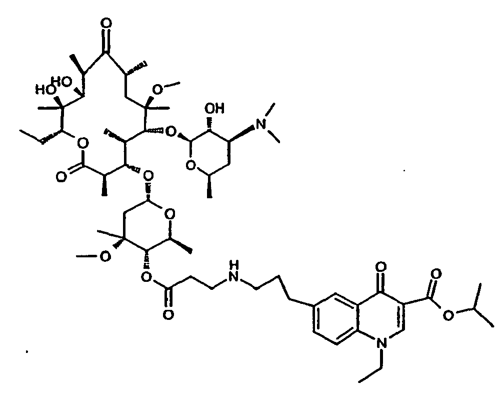

ESMS *m*/*z* 1119 [MH⁺].

### Example 30: 4"-O-{3-[3-(3-Carboxy-1,4-dihvdro-1-ethyl-4-oxo-6-quinolinyl) propylamino]propionyl}-6-O-methylerythromycin A i-butyl ester

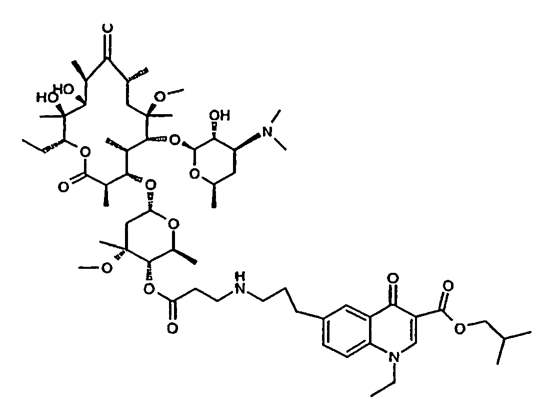

ESMS *m*/*z* 1134 [MH⁺].

### Example 31: 4"-O-{3-[3-(3-Carboxy-1,4-dihydro-1-ethyl-4-oxo-6-quinolinyl) propylamino]propionyl}-6-O-methylerythromycin A allyl ester

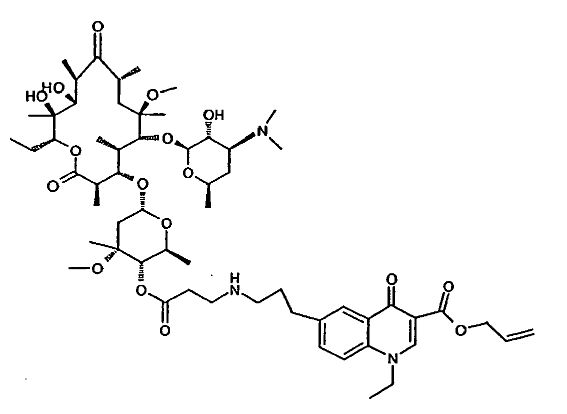

ESMS *m*/*z* 1.117 [MH⁺].

### Example 32: 4"-O-{3-[3-(3-Carboxy-1,4-dihydro-1-ethyl-4-oxo-6-quinolinyl) propylamino]propionyl}-6-O-methylerythromycin A cyclopropylmethyl ester

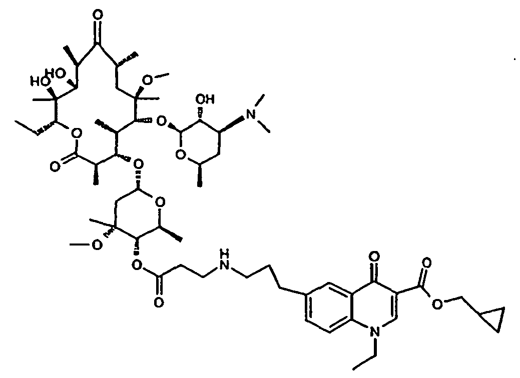

ESMS *m*/*z* 1131 [MH⁺].

### Example 33: 4"-O-{3-[3-(3-Carboxy-1,4-dihydro-1-ethyl-4-oxo-6-quinolinyl) propylamino]propionyl}-6-O-methylerythromycin A 3-butenyl ester

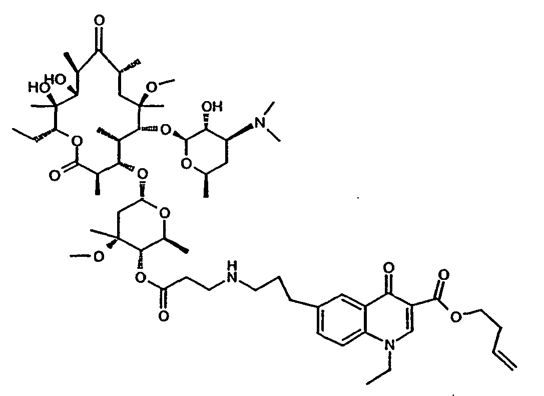

ESMS *m*/*z* 1131 [MH⁺].

### Example 34: 4"-O-{3-[3-(3-Carboxy-1,4-dihydro-1-ethyl-4-oxo-6-quinolinyl) propylamino]propionyl}-6-O-methylerythromycin A 2-butynyl ester

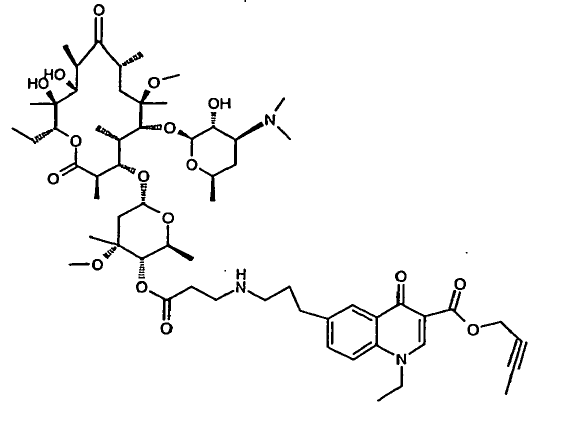

ESMS *m*/*z* 1129 [MH⁺].

### Example 35: 4"-O-{3-[3-(3-Carboxy-1,4-dihydro-1-ethyl-4-oxo-6-quinolinyl) propylamino]propionyl}-6-O-methyl-11-desoxy-11-(R)-aminomethyl-erythromycin A 11,12-carbamate diformate salt

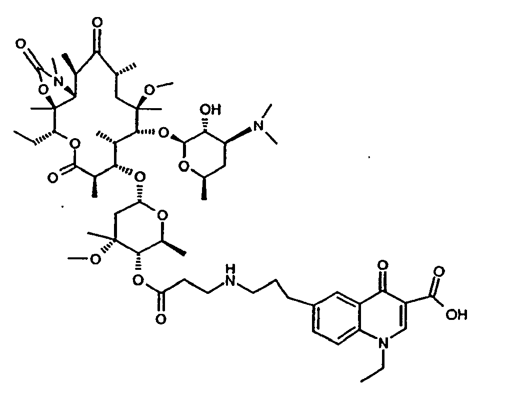

Using a similar procedure to that described in **Example 1a, Intermediate 2** (0.090 g, 0.23 mmol) and **Intermediate 31** (S. Alihodzic *et al.,* WO 03/042228) (0.103 g, 0.23 mmol) gave, after chromatography, methanolysis of the 2'OAc, followed by chromatography gave the title compound as a cream solid; ESMS *m*/*z* 1116 [MH⁺].

### Example 36: 4"-O-[3-[3-(3-Carboxy-1,4-dihydro-1-methyl-4-oxo-6-quinolinyl) propylamino]propionyl]-azithromycin-11,12-carbonate

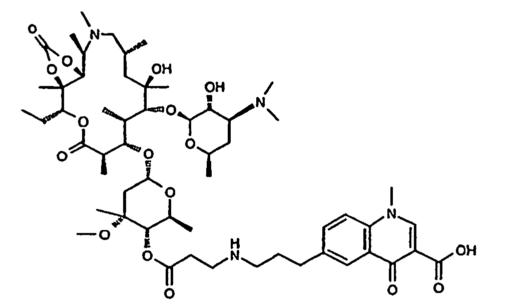

Using a similar procedure to that described in **Example 1a, Intermediate 31e** and **Intermediate 4** gave the title compound as a white solid; ESMS 1090 m/z [M+H]⁺.

### Example 37: 4"-O-[3-[3-(3-Carboxy-1,4-dihydro-1-methyl-4-oxo-6-quinolinyl) propylamino]propionyl]-6-O-methylerythromycin A

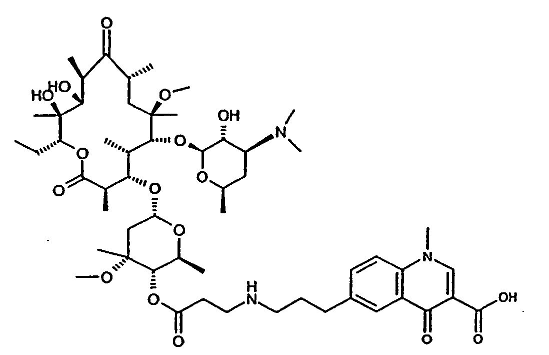

Using a similar procedure to that described in **Example 1a, Intermediate 32c** and **Intermediate 9** gave the title compound as a white solid; ESMS 1063 m/z [M+H]⁺.

### Example 38: 4"-O-[3-[3-(3-Carboxy-1,4-dihydro-1-(2-methoxyethyl)-4-oxo-6-quinolinyl) propylamino]propionyl]-6-O-methylerythromycin A

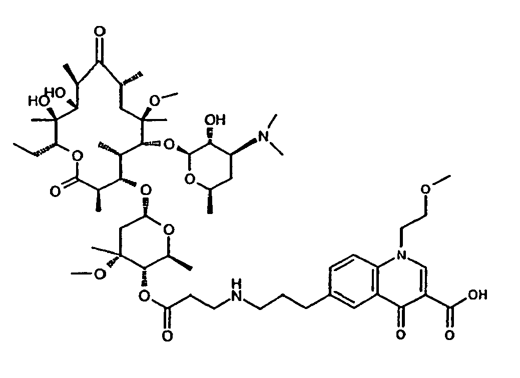

Using a similar procedure to that described in **Example 1a, Intermediate 32c** and **Intermediate 9** gave the title compound as a white solid; ESMS 1107 m/z [M+H]⁺.

### Example 39: 4"-O-[3-[3-(3-Carboxy-1,4-dihydro-1-(2-methoxyethyl)-4-oxo-6-quinolinyl) propylamino]propionyl]-azithromycin-11,12-carbonate

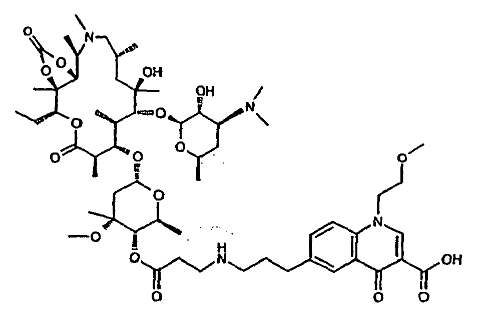

Using a similar procedure to that described in **Example 1a, Intermediate 32c** and **Intermediate 4** gave the title compound as a white solid; ESMS 1134 m/z [M+H]⁺.

### Example 40: 4"-O-[3-[3-(3-Carboxy-1,4-dihydro-1-cyclopropyl-4-oxo-6-quinolinyl) propylamino]propionyl]-azithromycin-11,12-carbonate triformate salt

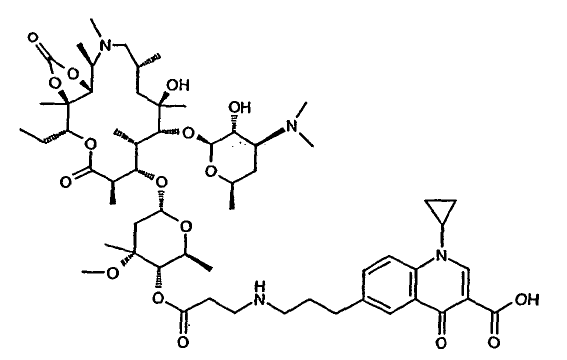

Using a similar procedure to that described in **Example 1a, Intermediate 33d** and **Intermediate 4** gave, after chromatography, the title compound as a cream solid; ESMS 1116 m/z [M+H]⁺.

### Example 41: 4"-O-[3-[3-(3-Carboxy-1,4-dihydro-1-cyclopropyl-4-oxo-6-quinolinyl) propylamino]propionyl]-6-O-methylerythromycin A diformate

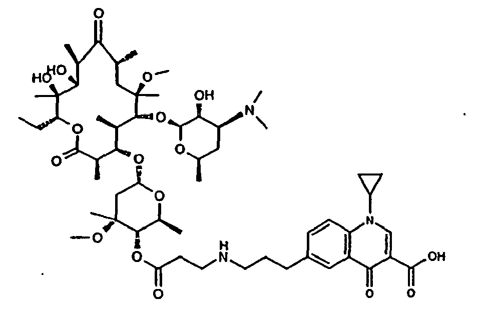

Using a similar procedure to that described in **Example 1a, Intermediate 33d** and **Intermediate 9** gave, after chromatography, the title compound as a tan solid; ESMS 1089 m/z [M+H]⁺.

### Example 42: 4"-O-{6-[3-(3-Carboxy-1-ethyl-4-oxo-1,4-dihydro-quinolin-6-yl)-propoxy]-hexanoyl}-azithromycin

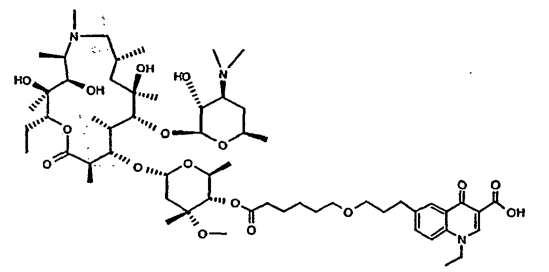

Hydrogenation of **Intermediate 38** (106 mg, 0.095 mmol) in ethanol (15 mL) with 10 % Pd/C (14 mg) in Parr apparatus at 5 bar for 20 hours gave the title product (51 mg).
MS (ES) m/z: [MH]⁺ 1120.68.

### Example 43: 4"-O-{6-[3-(3-Carboxy-1-ethyl-4-oxo-1,4-dihydro-quinolin-6-yl)-propoxy]-hexanoyl}-clarithromycin

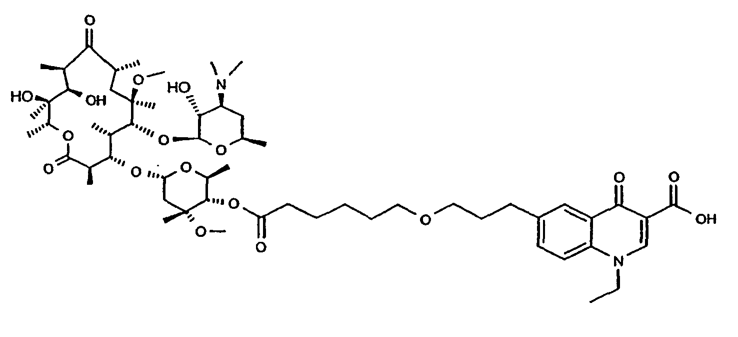

Hydrogenation of **Intermediate 40** (44 mg, 0.04 mmol) in ethanol (15 mL) with 10 % Pd/C (20 mg) in Parr apparatus at 5 bar for 20 hours gave the title product (41 mg).
MS (ES) m/z: [MH]⁺ 1119.5.

### Example 44: 4"-O-{6-[3-(3-Carboxy-1-ethyl-4-oxo-1,4-dihydro-quinolin-6-yl)-propoxy]-hexano yl}-11-O-methyl-azithromycin

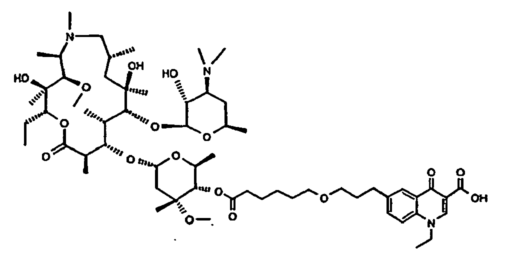

Hydrogenation of **Intermediate 42** (55 mg, 0.05 mmol) in ethanol (20 mL) with 10 % Pd/C (25 mg) in Parr apparatus at 5 bar for 20 hours gave the title product (50 mg).
MS (ES) m/z: [MH]⁺ 1134.51.

### Biological Data

Using a standard broth dilution method in microtitre, compounds were tested for antibacterial activity. The compounds in the above examples gave minimum inhibitory concentrations (MICs) less than 1 microgram per millilitre against erythromycin-sensitive and erythromycin-resistant strains of *Streptococcus pneumoniae* and *Streptococcus pyogenes.*

In addition, the MIC (µg/mL) of test compounds against various organisms was determined including:
*S. aureus* Smith ATCC 13709, *S. pneumoniae* SP030, *S. pyogenes* 3565, *E. faecalis* ATCC 29212, *H. influenzae* ATCC 49247, *M. catarrhalis* ATCC 23246.

Examples 1-4, 6 and 8 have an MIC ≤1 µg/mL against *S. aureus* Smith ATCC 13709, *S. pneumoniae* SP030, *S. pyogenes* 3565 and *E. faecalis* ATCC 29212.

Examples 3, 4, 6 and 7 have an MIC ≤2 µg/mL against *H. influenzae* ATCC 49247 and *M. catarrhalis* ATCC 23246.

Examples 4-8 have an MIC ≤0.25 µg/mL against erythromycin resistant strains of *Streptococcus pneumoniae* and *Streptococcus pyogenes.*

## Claims

1. A compound of formula (I) 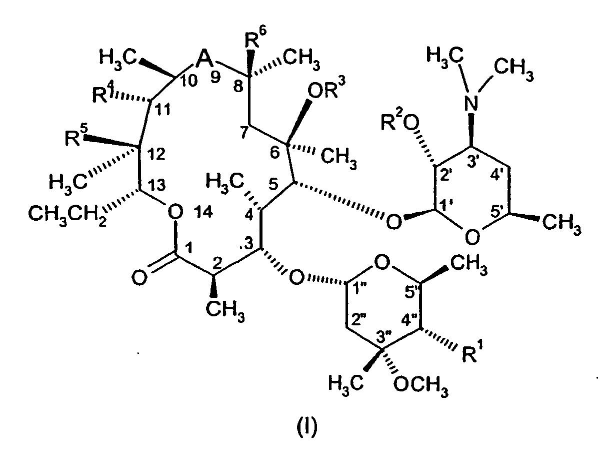 wherein
A is a bivalent radical selected from -C(O)-, -C(O)NH-, -NHC(O)-, -N(R⁷)-CH₂-, -CH₂-N(R⁷)-, -CH(NR⁸R⁹)- and -C(=NR¹⁰)-;
R¹ is -OC(O)(CH₂)_{d}XR¹¹;
R² is hydrogen or a hydroxyl protecting group;
R³ is hydrogen, C₁₋₄alkyl, or C₃₋₆alkenyl optionally substituted by 9 to 10 membered fused bicyclic heteroaryl;
R⁴ is hydroxy, C₃₋₆alkenyloxy optionally substituted by 9 to 10 membered fused bicyclic heteroaryl, or C₁₋₆alkoxy optionally substituted by C₁₋₆alkoxy or -O(CH₂)ₑNR⁷R¹², R⁵ is hydroxy, or
R⁴ and R⁵ taken together with the intervening atoms form a cyclic group having the following structure: 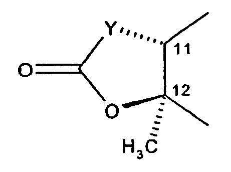 wherein Y is a bivalent radical selected from -CH₂-, -CH(CN)-, -O-, -N(R¹³)- and-CH(SR¹³)-;
R⁶ is hydrogen or fluorine;
R⁷ is hydrogen or C₁₋₆alkyl;
R⁸ and R⁹ are each independently hydrogen, C₁₋₆alkyl, -C(=NR¹⁰)NR¹⁴R¹⁵ or-C(O)R¹⁴, or
R⁸ and R⁹ together form =CH(CR¹⁴R¹⁵)_{f}aryl, =CH(CR¹⁴R¹⁵)_{f}heterocyclyl, =CR¹⁴R¹⁵ or =C(R¹⁴)C(O)OR¹⁴, wherein the alkyl, aryl and heterocyclyl groups are optionally substituted by up to three groups independently selected from R¹⁶;
R¹⁰ is -OR¹⁷, C₁₋₆alkyl, -(CH₂)garyl, -(CH₂)gheterocyclyl or -(CH₂)ₕO(CH₂)ᵢOR⁷, wherein each R¹⁰ group is optionally substituted by up to three groups independently selected from R¹⁶;
R¹¹ is a heterocyclic group having the following structure: 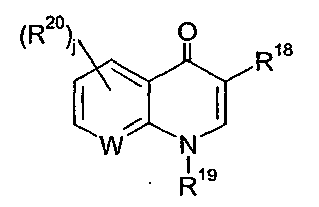 or 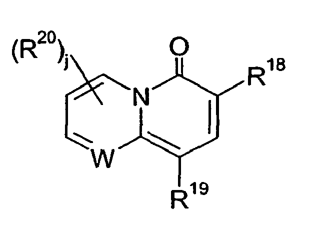 R¹² is hydrogen or C₁₋₆alkyl;
R¹³ is hydrogen or C₁₋₄alkyl optionally substituted by a group selected from optionally substituted phenyl, optionally substituted 5 or 6 membered heteroaryl and optionally substituted 9 to 10 membered fused bicyclic heteroaryl;
R¹⁴ and R¹⁵ are each independently hydrogen or C₁₋₆alkyl;
R¹⁶ is halogen, cyano, nitro, trifluoromethyl, azido, -C(O)R²¹, -C(O)OR²¹, -OC(O)R²¹,-OC(O)OR²¹, -NR²²C(O)R²³, -C(O)NR²²R²³, -NR²²R²³, hydroxy, C₁₋₆alkyl, -S(O)ₖC₁₋₆alkyl, C₁₋₆alkoxy, -(CH₂)ₘaryl or -(CH₂)ₘheteroaryl, wherein the alkoxy group is optionally substituted by up to three groups independently selected from -NR¹⁴R¹⁵, halogen and -OR¹⁴, and the aryl and heteroaryl groups are optionally substituted by up to five groups independently selected from halogen, cyano, nitro, trifluoromethyl, azido,-C(O)R²⁴, -C(O)OR²⁴, -OC(O)OR²⁴, -NR²⁵C(O)R²⁶, -C(O)NR²⁵R²⁶, -NR²⁵R²⁶, hydroxy, C₁₋₆alkyl and C₁₋₆alkoxy;
R¹⁷ is hydrogen, C₁₋₆alkyl, C₃₋₇cycloalkyl, C₃₋₆alkenyl or a 5 or 6 membered heterocyclic group, wherein the alkyl, cycloalkyl, alkenyl and heterocyclic groups are optionally substituted by up to three substituents independently selected from optionally substituted 5 or 6 membered heterocyclic group, optionally substituted 5 or 6 membered heteroaryl, -OR²⁷, -S(O)ₙR²⁷, -NR²⁷R²⁸, -CONR²⁷R²⁸, halogen and cyano;
R¹⁸ is hydrogen, -C(O)OR²⁹, -C(O)NHR²⁹, -C(O)CH₂NO₂ or -C(O)CH₂SO₂R⁷;
R¹⁹ is hydrogen, C₁₋₄alkyl optionally substituted by hydroxy or C₁₋₄alkoxy, C₃₋₇cycloalkyl, or optionally substituted phenyl or benzyl;
R²⁰ is halogen, C₁₋₄alkyl, C₁₋₄thioalkyl, C₁₋₄alkoxy, -NH₂, -NH(C₁₋₄alkyl) or -N(C₁₋₄alkyl)₂;
R²¹ is hydrogen, C₁₋₁₀alkyl, -(CH₂)paryl or -(CH₂)pheteroaryl;
R²² and R²³ are each independently hydrogen, -OR¹⁴, C₁₋₆alkyl, -(CH₂)_{q}aryl or-(CH₂)_{q}heterocyclyl;
R²⁴ is hydrogen, C₁₋₁₀alkyl, -(CH₂)ᵣaryl or -(CH₂)ᵣheteroaryl;
R²⁵ and R²⁶ are each independently hydrogen, -OR¹⁴, C₁₋₆alkyl, -(CH₂)ₛaryl or-(CH₂)ₛheterocyclyl;
R²⁷ and R²⁸ are each independently hydrogen, C₁₋₄alkyl or C₁₋₄alkoxyC₁₋₄alkyl;
R²⁹ is hydrogen,
C₁₋₆alkyl optionally substituted by up to three groups independently selected from halogen, cyano, C₁₋₄alkoxy optionally substituted by phenyl or C₁₋₄alkoxy,-C(O)C₁₋₆alkyl, -C(O)OC₁₋₆alkyl, -OC(O)C₁₋₆alkyl, -OC(O)OC₁₋₆alkyl,-C(O)NR³²R³³, -NR³²R³³ and phenyl optionally substituted by nitro or -C(O)OC₁₋₆alkyl,
-(CH₂)_{W}C₃₋₇cycloalkyl,
-(CH₂)_{W}heterocyclyl,
-(CH₂)_{W}heteroaryl,
-(CH₂)_{W}aryl,
C₃₋₆alkenyl, or
C₃₋₆alkynyl;
R³⁰ is hydrogen, C₁₋₄alkyl, C₃₋₇cycloalkyl, optionally substituted phenyl or benzyl, acetyl or benzoyl;
R³¹ is hydrogen or R²⁰, or R³¹ and R¹⁹ are linked to form the bivalent radical -O(CH₂)₂- or -(CH₂)ₜ-;
R³² and R³³ are each independently hydrogen or C₁₋₆alkyl optionally substituted by phenyl or -C(O)OC₁₋₆alkyl, or
R³² and R³³, together with the nitrogen atom to which they are bound, form a 5 or 6 membered heterocyclic group optionally containing one additional heteroatom selected from oxygen, nitrogen and sulfur;
X is -U(CH₂)ᵥ-;
U is a divalent radical selected from -N(R³⁰)-, -O-, -S(O)_{z}-, -N(R³⁰)C(O)-, -C(O)N(R³⁰)- and -N[C(O)R³⁰]-;
W is -C(R³¹)- or a nitrogen atom;
d is an integer from 1 to 5;
e is an integer from 2 to 4;
f, g, h, m, p, q, r, s and w are each independently integers from 0 to 4;
i is an integer from 1 to 6;
j, k, n and z are each independently integers from 0 to 2;
t is 2 or 3;
v is an integer from 1 to 8;
or a pharmaceutically acceptable derivative thereof.

2. A compound according to claim 1 wherein A is -C(O)-.

3. A compound according to claim 1 or claim 2 wherein X is -N(R³⁰)(CH₂)ᵥ-.

4. A compound according to any one of the preceding claims wherein d is 2.

5. A compound according to any one of the preceding claims wherein R¹¹ is a heterocyclic group of the following formula: 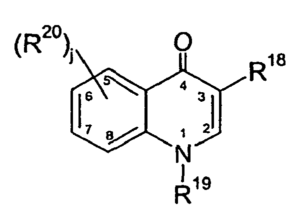 wherein the heterocyclic is linked in the 6 or 7 position and j, R¹⁸, R¹⁹ and R²⁰ are as defined in claim 1.

6. A compound according to any one of claims 1 to 4 wherein R¹¹ is a heterocyclic group of the following formula: 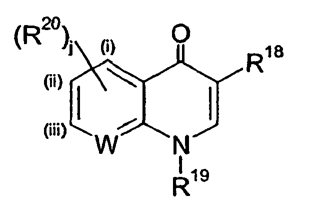 wherein W is -C(R³¹)- where R³¹ and R¹⁹ are linked to form the bivalent radical-O(CH₂)₂- or -(CH₂)ₜ- and said heterocyclic is linked in the (i), (ii) or (iii) position and j, R¹⁸ and R²⁰ are as defined in claim 1.

7. A compound according to claim 1 as defined in any one of Examples 1 to 44, or a pharmaceutically acceptable derivative thereof.

8. A compound selected from:
4"-O-{3-[3-(3-carboxy-1,4-dihydro-1-ethyl-4-oxo-6-quinolinyl)propylamino]propionyl}-6-O-methylerythromycin A;
4"-*O*-{3-[3-(3-carboxy-1,4-dihydro-1-ethyl-4-oxo-6-quinolinyl) propylamino]propionyl}-azithromycin-11,12-carbonate;
4"-*O*-{3-[3-(3-carboxy-1,4-dihydro-1-ethyl-4-oxo-6-quinolinyl) propylamino]propionyl}-6-*O*-methyl-11-desoxy-11-(R)-amino-erythromycin A 11,12-carbamate;
4"-O-[3-[4-(2-carboxy-6,7-dihydro-1*H*,5*H*-pyrido[3,2,1-ij]-1-oxo-9-quinolinyl) propylamino]propionyl]-6-O-methyl erythromycin A;
4"-*O*-[3-[4-(3-carboxy-1-ethyl-1,4-dihydro-4-oxo-6-quinolinyl)propylamino]propionyl]-(9E)-*O*-({[2-(methyloxy)ethyl]oxy}methanoximino erythromycin A;
4"-*O*-[3-[4-(3-carboxy-1-ethyl-1,4-dihydro-4-oxo-6-quinolinyl)propylamino]propionyl]-(9E)-*O*-hydroximino erythromycin A;
4"-O-[3-[4-(2-carboxy-6,7-dihydro-1*H*,5*H*-pyrido[3,2,1-ij]-1-oxo-9-quinolinyl) propylamino]propionyl]-(9E)-*O-*hydroximino erythromycin A;
4"-*O*-{6-[3-(3-carboxy-1-ethyl-4-oxo-1,4-dihydro-quinolin-6-yl)-propoxy]-hexanoyl}-azithromycin; and
4"-*O*-{6-[3-(3-carboxy-1-ethyl-4-oxo-1,4-dihydro-quinolin-6-yl)-propoxy]-hexanoyl}-clarithromycin;
or a pharmaceutically acceptable derivative thereof.

9. A process for the preparation of a compound as claimed in claim 1, or a pharmaceutically acceptable derivative thereof, which comprises:
a) reacting a compound of formula (II) 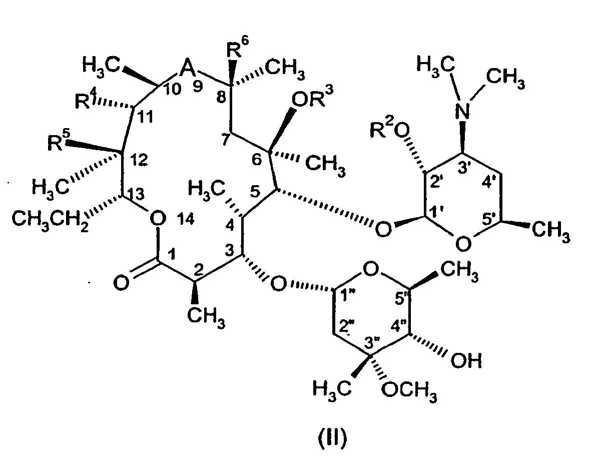
HOC(O)(CH₂)_{d}X^{a}R^{11a} (III)
with a suitable activated derivative of the acid (III), wherein X^{a} and R^{11a} are X and R¹¹ as defined in claim 1 or groups convertible to X and R¹¹;
b) reacting a compound of formula (IV) 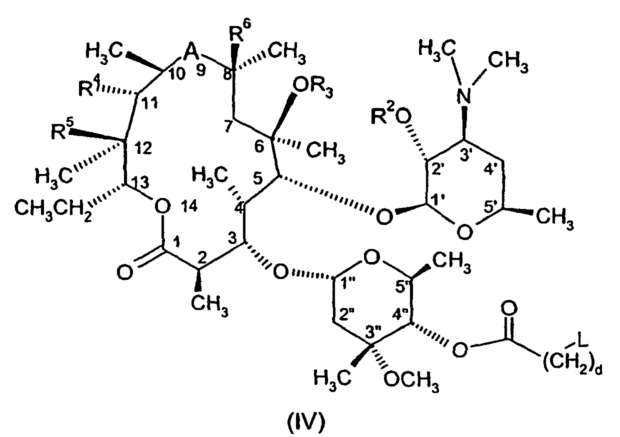 with a compound of formula X^{a}R^{11a} (V), wherein R^{11a} is R¹¹ as defined in claim 1 or a group convertible to R¹¹ and X^{a} is -U(CH₂)ᵥ- or a group convertible to -U(CH₂)ᵥ- in which U is a group selected from -N(R³⁰)- and -S-, and L is suitable leaving group, to produce a compound of formula (I) wherein U is a group selected from -N(R³⁰)- and -S-; or
c) reacting a compound of formula (VII), with a compound of formula X^{a}R¹¹a (V), 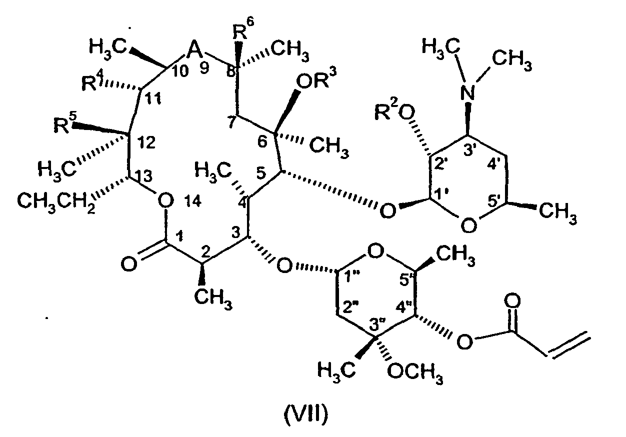
wherein R^{11a} is R¹¹ as defined in claim 1 or a group convertible to R¹¹ and X^{a} is-U(CH₂)ᵥ- or a group convertible to -U(CH₂)ᵥ- in which U is a group selected from-N(R³⁰)- and -S-, to produce a compound of formula (I) wherein d is 2 and U is a group selected from -N(R³⁰)- and -S-, and thereafter, if required, subjecting the resulting compound to one or more of the following operations:
i) removal of the protecting group R²,
ii) conversion of X^{a}R^{11a} to XR¹¹, and
iii) conversion of the resultant compound of formula (I) into a pharmaceutically acceptable derivative thereof.

10. A compound as claimed in any one of claims 1 to 8, or a pharmaceutically acceptable derivative thereof, for use in therapy.

11. The use of a compound as claimed in any one of claims 1 to 8, or a pharmaceutically acceptable derivative thereof, in the manufacture of a medicament for use in the treatment or prophylaxis of systemic or topical microbial infections in a human or animal body.

12. A pharmaceutical composition comprising at least one compound as claimed in any one of claims 1 to 8, or a pharmaceutically acceptable derivative thereof, in association with a pharmaceutically acceptable excipient, diluent and/or carrier.

13. A compound of formula (IA): 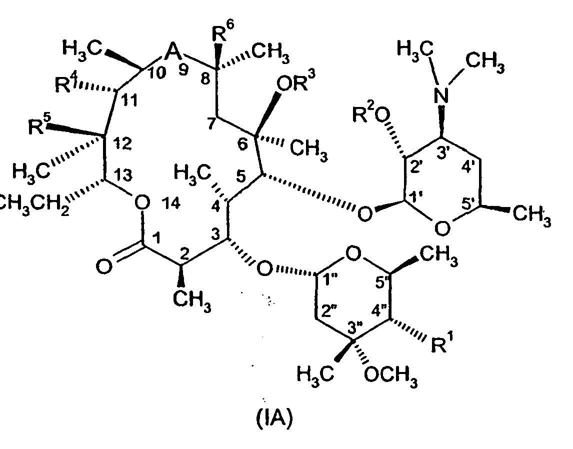 wherein
A is a bivalent radical selected from -C(O)-, -C(O)NH-, -NHC(O)-, -N(R⁷)-CH₂-, -CH₂-N(R⁷)-, -CH(NR⁸R⁹)- and -C(=NR¹⁰)-;
R¹ is -OC(O)(CH₂)_{d}XR¹¹;
R² is hydrogen or a hydroxyl protecting group;
R³ is hydrogen, C₁₋₄alkyl, or C₃₋₆alkenyl optionally substituted by 9 to 10 membered fused bicyclic heteroaryl;
R⁴ is hydroxy, C₃₋₆alkenyloxy optionally substituted by 9 to 10 membered fused bicyclic heteroaryl, or C₁₋₆alkoxy optionally substituted by C₁₋₆alkoxy or -O(CH₂)ₑNR⁷R¹², R⁵ is hydroxy, or
R⁴ and R⁵ taken together with the intervening atoms form a cyclic group having the following structure: 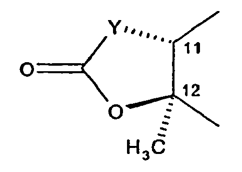 wherein Y is a bivalent radical selected from -CH₂-, -CH(CN)-, -O-, -N(R¹³)- and-CH(SR¹³)-;
R⁶ is hydrogen or fluorine;
R⁷ is hydrogen or C₁₋₆alkyl;
R⁸ and R⁹ are each independently hydrogen, C₁₋₆alkyl, -C(=NR¹⁰)NR¹⁴R¹⁵ or-C(O)R¹⁴, or
R⁸ and R⁹ together form =CH(CR¹⁴R¹⁵)_{f}aryl, =CH(CR¹⁴R¹⁵)_{f}heterocyclyl, =CR¹⁴R¹⁵ or =C(R¹⁴)C(O)OR¹⁴, wherein the alkyl, aryl and heterocyclyl groups are optionally substituted by up to three groups independently selected from R¹⁶;
R¹⁰ is -OR¹⁷, C₁₋₆alkyl, -(CH₂)garyl, -(CH₂)gheterocyclyl or -(CH₂)ₕO(CH₂)ᵢOR⁷, wherein each R¹⁰ group is optionally substituted by up to three groups independently selected from R¹⁶;
R¹¹ is a heterocyclic group having the following structure: 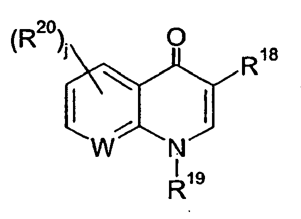 or 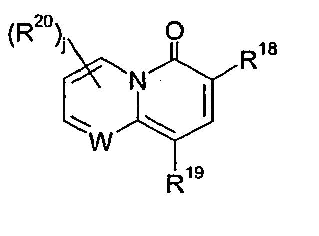 R¹² is hydrogen or C₁₋₆alkyl;
R¹³ is hydrogen or C₁₋₄alkyl optionally substituted by a group selected from optionally substituted phenyl, optionally substituted 5 or 6 membered heteroaryl and optionally substituted 9 to 10 membered fused bicyclic heteroaryl;
R¹⁴ and R¹⁵ are each independently hydrogen or C₁₋₆alkyl;
R¹⁶ is halogen, cyano, nitro, trifluoromethyl, azido, -C(O)R²¹, -C(O)OR²¹, -OC(O)R²¹,-OC(O)OR²¹, -NR²²C(O)R²³, -C(O)NR²²R²³, -NR²²R²³, hydroxy, C₁₋₆alkyl, -S(O)ₖC₁₋₆alkyl, C₁₋₆alkoxy, -(CH₂)ₘaryl or -(CH₂)ₘheteroaryl, wherein the alkoxy group is optionally substituted by up to three groups independently selected from -NR¹⁴R¹⁵, halogen and -OR¹⁴, and the aryl and heteroaryl groups are optionally substituted by up to five groups independently selected from halogen, cyano, nitro, trifluoromethyl, azido,-C(O)R²⁴, -C(O)OR²⁴, -OC(O)OR²⁴, -NR²⁵C(O)R²⁶, -C(O)NR²⁵R²⁶, -NR²⁵R²⁶, hydroxy, C₁₋₆alkyl and C₁₋₆alkoxy;
R¹⁷ is hydrogen, C₁₋₆alkyl, C₃₋₇cycloalkyl, C₃₋₆alkenyl or a 5 or 6 membered heterocyclic group, wherein the alkyl, cycloalkyl, alkenyl and heterocyclic groups are optionally substituted by up to three substituents independently selected from optionally substituted 5 or 6 membered heterocyclic group, optionally substituted 5 or 6 membered heteroaryl, -OR²⁷, -S(O)ₙR²⁷, -NR²⁷R²⁸, -CONR²⁷R²⁸, halogen and cyano;
R¹⁸ is hydrogen, -C(O)OR²⁹, -C(O)NHR²⁹ or -C(O)CH₂NO₂;
R¹⁹ is hydrogen, C₁₋₄alkyl optionally substituted by hydroxy or C₁₋₄alkoxy, C₃₋₇cycloalkyl, or optionally substituted phenyl or benzyl;
R²⁰ is halogen, C₁₋₄alkyl, C₁₋₄thioalkyl, C₁₋₄alkoxy, -NH₂, -NH(C₁₋₄alkyl) or -N(C₁₋₄alkyl)₂;
R²¹ is hydrogen, C₁₋₁₀alkyl, -(CH₂)paryl or -(CH₂)ₚheteroaryl;
R²² and R²³ are each independently hydrogen, -OR¹⁴, C₁₋₆alkyl, -(CH₂)_{q}aryl or-(CH₂)_{q}heterocyclyl;
R²⁴ is hydrogen, C₁₋₁₀alkyl, -(CH₂)ᵣaryl or -(CH₂)ᵣheteroaryl;
R²⁵ and R²⁶ are each independently hydrogen, -OR¹⁴, C₁₋₆alkyl, -(CH₂)ₛaryl or-(CH₂)ₛheterocyclyl;
R²⁷ and R²⁸ are each independently hydrogen, C₁₋₄alkyl or C₁₋₄alkoxyC₁₋₄alkyl;
R²⁹ is hydrogen, C₁₋₆alkyl optionally substituted by up to three groups independently selected from halogen, C₁₋₄alkoxy, -OC(O)C₁₋₆alkyl, -OC(O)OC₁₋₆alkyl, -C(O)NR³²R³³ and -NR³²R³³, -(CH₂)_{w}C₃₋₇cycloalkyl, C₃₋₆alkenyl or C₃₋₆alkynyl;
R³⁰ is hydrogen, C₁₋₄alkyl, C₃₋₇cycloalkyl, optionally substituted phenyl or benzyl, acetyl or benzoyl;
R³¹ is hydrogen or R²⁰, or R³¹ and R¹⁹ are linked to form the bivalent radical -O(CH₂)₂- or -(CH₂)ₜ-;
R³² and R³³ are each independently hydrogen or C₁₋₆alkyl optionally substituted by-C(O)OC₁₋₆alkyl, or
R³² and R³³, together with the nitrogen atom to which they are bound, form a 5 or 6 membered heterocyclic group optionally containing one additional heteroatom selected from oxygen, nitrogen and sulfur;
X is -U(CH₂)ᵥ-;
U is a divalent radical selected from -N(R³⁰)-, -O-. -S(O)_{z}-, -N(R³⁰)C(O)-, -C(O)N(R³⁰)- and -N[C(O)R³⁰]-;
W is -C(R³¹)- or a nitrogen atom;
d is an integer from 1 to 5;
e is an integer from 2 to 4;
f, g, h, m, p, q, r, s and w are each independently integers from 0 to 4;
i is an integer from 1 to 6;
j, k, n and z are each independently integers from 0 to 2;
t is 2 or 3;
v is an integer from 2 to 8;
or a pharmaceutically acceptable derivative thereof.

14. A compound of formula (IB) 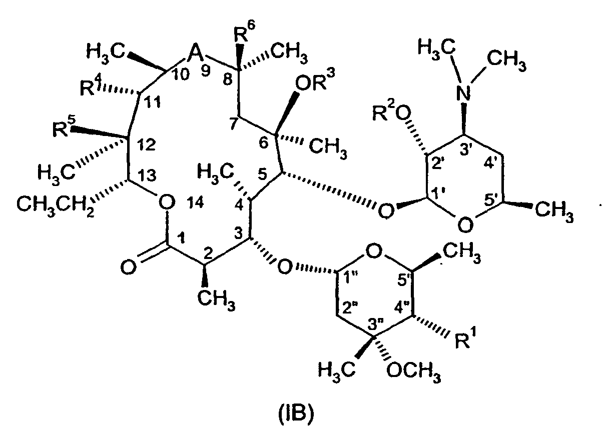 wherein
A is a bivalent radical selected from -C(O)-, -C(O)NH-, -NHC(O)-, -N(R⁷)-CH₂-, -CH₂-N(R⁷)-, -CH(NR⁸R⁹)- and -C(=NR¹⁰)-;
R¹ is -OC(O)(CH₂)_{d}XR¹¹;
R² is hydrogen or a hydroxyl protecting group;
R³ is hydrogen, C₁₋₄alkyl, or C₃₋₆alkenyl optionally substituted by 9 to 10 membered fused bicyclic heteroaryl;
R⁴ is hydroxy, C₃₋₆alkenyloxy optionally substituted by 9 to 10 membered fused bicyclic heteroaryl, or C₁₋₆alkoxy optionally substituted by C₁₋₆alkoxy or -O(CH₂)ₑNR⁷R¹², R⁵ is hydroxy, or
R⁴ and R⁵ taken together with the intervening atoms form a cyclic group having the following structure: 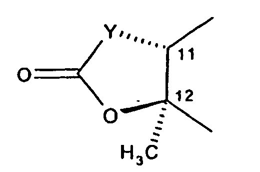 wherein Y is a bivalent radical selected from -CH₂-, -CH(CN)-, -O-, -N(R¹³)- and-CH(SR¹³)-;
R⁶ is hydrogen or fluorine;
R⁷ is hydrogen or C₁₋₆alkyl;
R⁸ and R⁹ are each independently hydrogen, C₁₋₆alkyl, -C(=NR¹⁰)NR¹⁴R¹⁵ or-C(O)R¹⁴, or
R⁸ and R⁹ together form =CH(CR¹⁴R¹⁵)_{f}aryl, =CH(CR¹⁴R¹⁵)_{f}heterocyclyl, =CR¹⁴R¹⁵ or =C(R¹⁴)C(O)OR¹⁴, wherein the alkyl, aryl and heterocyclyl groups are optionally substituted by up to three groups independently selected from R¹⁶;
R¹⁰ is -OR¹⁷, C₁₋₆alkyl, -(CH₂)garyl, -(CH₂)gheterocyclyl or -(CH₂)ₕO(CH₂)ᵢOR⁷, wherein each R¹⁰ group is optionally substituted by up to three groups independently selected from R¹⁶;
R¹¹ is a heterocyclic group having the following structure: 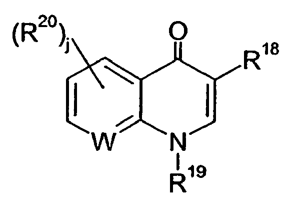 or 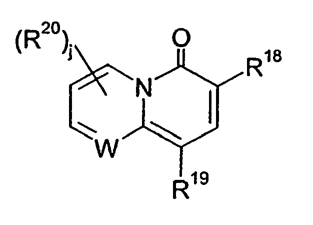 R¹² is hydrogen or C₁₋₆alkyl;
R¹³ is hydrogen or C₁₋₄alkyl substituted by a group selected from optionally substituted phenyl, optionally substituted 5 or 6 membered heteroaryl and optionally substituted 9 to 10 membered fused bicyclic heteroaryl;
R¹⁴ and R¹⁵ are each independently hydrogen or C₁₋₆alkyl;
R¹⁶ is halogen, cyano, nitro, trifluoromethyl, azido, -C(O)R²¹, -C(O)OR²¹, -OC(O)R²¹,-OC(O)OR²¹, -NR²²C(O)R²³, -C(O)NR²²R²³, -NR²²R²³, hydroxy, C₁₋₆alkyl, -S(O)ₖC₁₋₆-alkyl, C₁₋₆alkoxy, -(CH₂)ₘaryl or -(CH₂)ₘheteroaryl, wherein the alkoxy group is optionally substituted by up to three groups independently selected from -NR¹⁴R¹⁵, halogen and -OR¹⁴, and the aryl and heteroaryl groups are optionally substituted by up to five groups independently selected from halogen, cyano, nitro, trifluoromethyl, azido,-C(O)R²⁴, -C(O)OR²⁴, -OC(O)OR²⁴, -NR²⁵C(O)R²⁶, -C(O)NR²⁵R²⁶, -NR²⁵R²⁶, hydroxy, C₁₋₆alkyl and C₁₋₆alkoxy;
R¹⁷ is hydrogen, C₁₋₆alkyl, C₃₋₇cycloalkyl, C₃₋₆alkenyl or a 5 or 6 membered heterocyclic group, wherein the alkyl, cycloalkyl, alkenyl and heterocyclic groups are optionally substituted by up to three substituents independently selected from optionally substituted 5 or 6 membered heterocyclic group, optionally substituted 5 or 6 membered heteroaryl, -OR²⁷, -S(O)ₙR²⁷, -NR²⁷R²⁸, -CONR²⁷R²⁸, halogen and cyano;
R¹⁸ is hydrogen, -C(O)OR²⁹, -C(O)NHR²⁹ or -C(O)CH₂NO₂;
R¹⁹ is hydrogen, C₁₋₄alkyl optionally substituted by hydroxy or C₁₋₄alkoxy, C₃₋₇cycloalkyl, or optionally substituted phenyl or benzyl;
R²⁰ is halogen, C₁₋₄alkyl, C₁₋₄thioalkyl, C₁₋₄alkoxy, -NH₂, -NH(C₁₋₄alkyl) or -N(C₁₋₄alkyl)₂;
R²¹ is hydrogen, C₁₋₁₀alkyl, -(CH₂)ₚaryl or -(CH₂)ₚheteroaryl;
R²² and R²³ are each independently hydrogen, -OR¹⁴, C₁₋₆alkyl, -(CH₂)_{q}aryl or-(CH₂)_{q}heterocyclyl;
R²⁴ is hydrogen, C₁₋₁₀alkyl, -(CH₂)ᵣaryl or -(CH₂)ᵣheteroaryl;
R²⁵ and R²⁶ are each independently hydrogen, -OR¹⁴, C₁₋₆alkyl, -(CH₂)ₛaryl or-(CH₂)_{S}heterocyclyl;
R²⁷ and R²⁸ are each independently hydrogen, C₁₋₄alkyl or C₁₋₄alkoxyC₁₋₄alkyl;
R²⁹ is hydrogen or C₁₋₆alkyl optionally substituted by up to three groups independently selected from halogen, C₁₋₄alkoxy, -OC(O)C₁₋₆alkyl and -OC(O)OC₁₋₆alkyl;
R³⁰ is hydrogen, C₁₋₄alkyl, C₃₋₇cycloalkyl, optionally substituted phenyl or benzyl, acetyl or benzoyl;
R³¹ is hydrogen or R²⁰, or R³¹ and R¹⁹ are linked to form the bivalent radical -O(CH₂)₂- or -(CH₂)ₜ-;
X is -U(CH₂)ᵥ-;
U is a divalent radical selected from -N(R³⁰)-, -O-, -S(O)_{z}-, -N(R³⁰)C(O)-, -C(O)N(R³⁰)- and -N[C(O)R³⁰]-;
W is -C(R³¹)- or a nitrogen atom;
d is an integer from 1 to 5;
e is an integer from 2 to 4;
f, g, h, m, p, q, r and s are each independently integers from 0 to 4;
i is an integer from 1 to 6;
j, k, n and z are each independently integers from 0 to 2;
t is 2 or 3;
v is an integer from 2 to 8;
or a pharmaceutically acceptable derivative thereof.

## Patentansprüche

1. Verbindung der Formel (I), 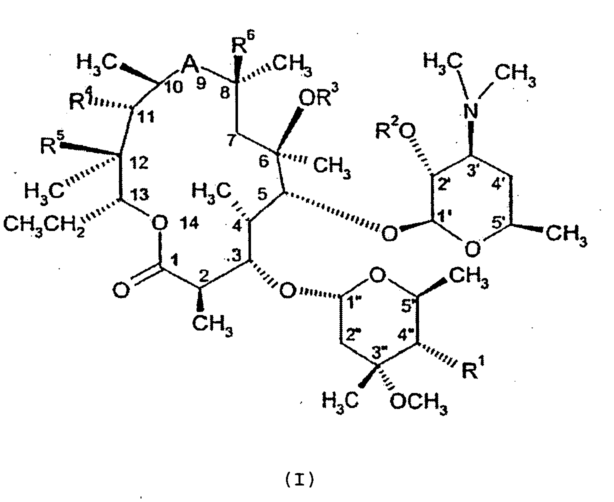 worin
A ein bivalenter Rest ist, ausgewählt aus -C(O)-, -C(O)NH-, -NHC(O)-, -N(R⁷)-CH₂-, -CH₂-N(R⁷)-, -CH(NR⁸R⁹)- und -C(=NR¹⁰)-;
R¹ -OC(O)(CH₂)_{d}XR¹¹ ist;
R² Wasserstoff oder eine Hydroxylschutzgruppe ist;
R³ Wasserstoff, C₁₋₄-Alkyl oder C₃₋₆-Alkenyl ist, gegebenenfalls substituiert durch ein 9- bis 10-gliedriges fusioniertes bizyklisches Heteroaryl; R⁴ Hydroxy, C₃₋₆-Alkenyloxy, gegebenenfalls substituiert durch ein 9-10-gliedriges fusioniertes bizyklisches Heteroaryl, oder C₁₋₆-Alkoxy, gegebenenfalls substituiert durch C₁₋₆-Alkoxy oder -O(CH₂)ₑNR⁷R¹² ist,
R⁵ Hydroxy ist, oder
R⁴ und R⁵ zusammen mit den dazwischenliegenden Atomen eine zyklische Gruppe mit der folgenden Struktur bilden: 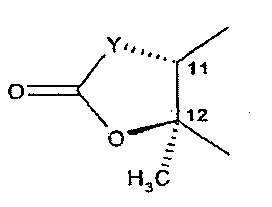 worin Y ein bivalenter Rest ist, ausgewählt aus -CH₂-, -CH(CN)-, -O-, -N(R¹³)- und -CH(SR¹³)-;
R⁶ Wasserstoff oder Fluor ist;
R⁷ Wasserstoff oder C₁₋₆-Alkyl ist;
R⁸ und R⁹ jeweils unabhängig voneinander Wasserstoff, C₁₋₆-Alkyl, -C(=NR¹⁰)NR¹⁴R¹⁵ oder -C(O)R¹⁴ ist, oder
R⁸ und R⁹ zusammen =CH(CR¹⁴R¹⁵)_{f}-Aryl, =CH(CR¹⁴R¹⁵)_{f}-Heterocyclyl, =CR¹⁴R¹⁵ oder =C(R¹⁴)C(O)OR¹⁴ bilden, worin die Alkyl-, Aryl- und
Heterocyclylgruppen gegebenenfalls durch bis zu drei Gruppen substituiert sind, die unabhängig voneinander ausgewählt sind aus R¹⁶;
R¹⁰ ist -OR¹⁷, C₁₋₆-Alkyl, -(CH₂)_{g}-Aryl, -(CH₂)_{g}-Heterocyclyl oder
-(CH₂)ₕO(CH₂)ᵢOR⁷, worin jede R¹⁰-Gruppe gegebenenfalls substituiert ist durch bis zu drei Gruppen unabhängig ausgewählt aus R¹⁶;
R¹¹ ist eine heterozyklische Gruppe mit der folgenden Struktur: 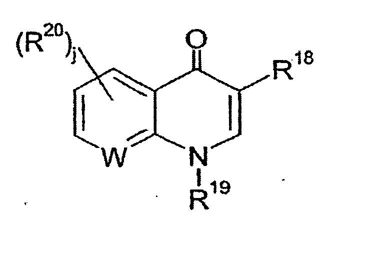 oder 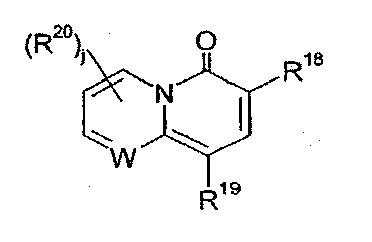 R¹² ist Wasserstoff oder C₁₋₆-Alkyl;
R¹³ ist Wasserstoff oder C₁₋₄-Alkyl, gegebenenfalls substituiert durch eine Gruppe, ausgewählt aus gegebenenfalls substituiertem Phenyl, gegebenenfalls substituiertem 5- oder 6-gliedrigen Heteroaryl und gegebenenfalls substituiertem 9- oder 10-gliedrigen fusionierten bizyklischen Heteroaryl; R¹⁴ und R¹⁵ sind jeweils unabhängig voneinander Wasserstoff oder C₁₋₆-Alkyl;
R¹⁶ ist Halogen, Cyano, Nitro, Trifluormethyl, Azido, -C(O)R²¹, -C(O)OR²¹, -OC(O)R²¹, -OC(O)OR²¹, -NR²²C(O)R²³, -C(O)NR²²R²³, - NR²²R²³, Hydroxy, C₁₋₆-Alkyl, -S(O)ₖC₁₋₆-Alkyl, C₁₋₆-Alkoxy, -(CH₂)ₘ-Aryl oder -(CH₂)ₘ-Heteroaryl, worin die Alkoxygruppe gegebenenfalls substituiert ist mit bis zu drei Gruppen, die unabhängig voneinander ausgewählt sind aus -N¹⁴R¹⁵, Halogen und -OR¹⁴, und die Aryl- und Heteroarylgruppen gegebenenfalls substituiert sind mit bis zu fünf Gruppen, die unabhängig voneinander ausgewählt sind aus Halogen, Cyano, Nitro, Trifluormethyl, Azido, C(O)R²⁴, -C(O)OR²⁴, -OC(O)OR²⁴, -NR²⁵C(O)R²⁶, -C(O)NR²⁵R²⁶, -NR²⁵R²⁶, Hydroxy, C₁₋₆-Alkyl und C₁₋₆-Alkoxy;
R¹⁷ ist Wasserstoff, C₁₋₆-Alkyl, C₃₋₇-Cycloalkyl, C₃₋₆-Alkenyl oder eine 5- oder 6-gliedrige heterozyklische Gruppe, worin die Alkyl-, Cycloalkyl-, Alkenyl- und heterozyklischen Gruppen gegebenenfalls mit bis zu drei Substituenten substituiert sind, die unabhängig voneinander ausgewählt sind aus gegebenenfalls substituierten 5- oder 6-gliedrigen heterozyklischen Gruppen, gegebenenfalls substituiertem 5- oder 6-gliedrigen Heteroaryl, -OR²⁷, -S(O)ₙR²⁷, -NR²⁷R²⁸, -CONR²⁷R²⁸, Halogen und Cyano;
R¹⁸ ist Wasserstoff, -C(O)OR²⁹, -C(O)NHR²⁹, -C(O)CH₂NO₂ oder -C(O)CH₂SO₂R⁷;
R¹⁹ ist Wasserstoff, C₁₋₄-Alkyl, gegebenenfalls substituiert mit Hydroxy oder C₁₋₄-Alkoxy, C₃₋₇-Cycloalkyl, oder gegebenenfalls substituiertem Phenyl oder Benzyl;
R²⁰ ist Halogen, C₁₋₄-Alkyl, C₁₋₄-Thioalkyl, C₁₋₄-Alkoxy, -NH₂, -NH(C₁₋₄-Alkyl) oder -N(C₁₋₄-Alkyl)₂;
R²¹ ist Wasserstoff, C₁₋₁₀-Alkyl, -(CH₂)ₚ-Aryl oder -(CH₂)ₚ-Heteroaryl; R²² und R²³ sind jeweils unabhängig voneinander Wasserstoff, -OR¹⁴, C₁₋₆-Alkyl, -(CH₂)_{q}-Aryl oder -(CH₂)_{q}-Heterocyclyl;
R₂₄ ist Wasserstoff, C₁₋₁₀-Alkyl, -(CH₂)ᵣ-Aryl oder -(CH₂)ᵣ-Heteroaryl;
R²⁵ und R²⁶ sind jeweils unabhängig voneinander Wasserstoff, -OR¹⁴, C₁₋₆-Alkyl, -(CH₂)ₛ-Aryl oder -(CH₂)ₛ-Heterocyclyl;
R²⁷ und R²⁸ sind jeweils unabhängig voneinander Wasserstoff, C₁₋₄-Alkyl oder C₁₋₄-Alkoxy-C₁₋₄-alkyl;
R²⁹ ist Wasserstoff,
C₁₋₆-Alkyl, gegebenenfalls substituiert mit bis zu drei Gruppen, die unabhängig voneinander ausgewählt sind aus Halogen, Cyano, C₁₋₄-Alkoxy, gegebenenfalls substituiert mit Phenyl oder C₁₋₄-Alkoxy, -C(O)C₁₋₆-Alkyl, -C(O)OC₁₋₆-Alkyl, -OC(O)C₁₋₆-Alkyl, -OC(O)OC₁₋₆-Alkyl, -C(O)NR³²R³³, -NR³²R³³, und Phenyl, gegebenenfalls substituiert mit Nitro oder -C(O)OC₁₋₆-Alkyl,
-(CH₂)_{w}C₃₋₇-Cycloalkyl,
-(CH₂)_{w}Heterocyclyl,
-(CH₂)_{w}Heteroaryl,
-(CH₂)_{w}Aryl,
C₃₋₆-Alkenyl oder
C₃₋₆-Alkynyl;
R³⁰ ist Wasserstoff, C₁₋₄-Alkyl, C₃₋₇-Cycloalkyl, gegebenenfalls substituiert mit Phenyl oder Benzyl, Acetyl oder Benzoyl;
R³¹ ist Wasserstoff oder R²⁰, oder R³¹ und R¹⁹ sind verbunden, um den bivalenten Rest -O(CH₂)₂- oder -(CH₂)ₜ- zu bilden;
R³² und R³³ sind jeweils unabhängig voneinander Wasserstoff oder C₁₋₆-Alkyl, gegebenenfalls substituiert mit Phenyl oder -C(O)OC₁₋₆-Alkyl, oder R³² und R³³ bilden zusammen mit dem Stickstoffatom, an das sie gebunden sind, eine 5- oder 6-gliedrige heterozyklische Gruppe, die gegebenenfalls ein zusätzliches Heteroatom enthält, das ausgewählt wird aus Sauerstoff, Stickstoff und Schwefel;
X ist -U(CH₂)ᵥ-;
U ist ein divalenter Rest, ausgewählt aus -N(R³⁰)-, -O-, -S(O)_{z}-, -N(R³⁰)C(O)-, -C(O)N(R³⁰)- und -N[C(O)R³⁰]-;
W ist -C(R³¹)- oder ein Stickstoffatom;
d ist eine ganze Zahl von 1 bis 5,
e ist eine ganze Zahl von 2 bis 4,
f, g, h, m, p, q, r, s und w sind jeweils unabhängig voneinander ganze Zahlen von 0 bis 4;
i ist eine ganze Zahl von 1 bis 6;
j, k, n und z sind jeweils unabhängig voneinander ganze Zahlen von 0 bis 2; t ist 2 oder 3;
v ist eine ganze Zahl von 1 bis 8;
oder ein pharmazeutisch annehmbares Derivat davon.

2. Verbindung gemäß Anspruch 1, worin A -C(O)- ist.

3. Verbindung gemäß Anspruch 1 oder Anspruch 2, worin X -N(R³⁰) (CH₂)ᵥ- ist.

4. Verbindung gemäß irgendeinem der vorhergehenden Ansprüche, worin d 2 ist.

5. Verbindung gemäß irgendeinem der vorhergehenden Ansprüche, worin R¹¹ eine heterozyklische Gruppe der folgenden Formel ist: 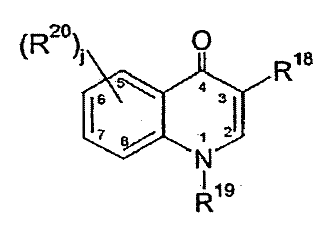 worin der Heterozyklus an die 6- oder 7-Position gebunden ist, und j, R¹⁸, R¹⁹ und R²⁰ wie in Anspruch 1 definiert sind.

6. Verbindung gemäß irgendeinem der Ansprüche 1 bis 4, worin R¹¹ eine heterozyklische Gruppe der folgenden Formel ist: 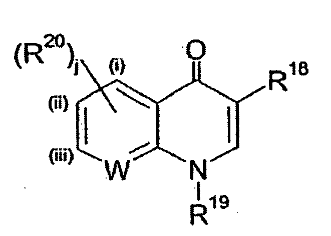 worin W -C(R³¹)- ist, worin R³¹ und R¹⁹ verbunden sind, um den bivalenten Rest -O(CH₂)₂ oder -(CH₂)ₜ- zu bilden, und dieser Heterozyklus an der (i), (ii) oder (iii) Position gebunden ist und j, R¹⁸ und R²⁰ wie in Anspruch 1 definiert sind.

7. Verbindung gemäß Anspruch 1, wie in irgendeinem der Beispiele 1 bis 44 definiert, oder ein pharmazeutisch annehmbares Derivat davon.

8. Verbindung ausgewählt aus:
4"-O-{3-[3-(3-Carboxy-1,4-dihydro-1-ethyl-4-oxo-6-chinolinyl)propylamin]propionyl}-6-O-methylerythromycin A;
4"-O-{3-[3-(3-Carboxy-1,4-dihydro-1-ethyl-4-oxo-6-chinolinyl)propylamin]propionyl}-azithromycin-11,12-carbonat;
4"-O-{3-[3-(3-Carboxy-1,4-dihydro-1-ethyl-4-oxo-6-chinolinyl)propylamin]propionyl}-6-O-methyl-11-desoxy-11-(R)-amino-erythromycin A 11,12-carbamat;
4"-O-{3-[4-(2-Carboxy-6,7-dihydro-1H,5H-pyrid[3,2,1-ij]-1-oxo-9-chinolinyl)propylamin]propionyl]-6-O-methylerythromycin A;
4"-O-[3-[4-(3-Carboxy-1-ethyl-1,4-dihydro-4-oxo-6-chinolinyl)propylamin]propionyl]-(9E)-O-({[2-methyloxy)ethyl]oxy}methanoximinerythromycin A;
4"-O-[3-[4-(3-Carboxy-1-ethyl-1,4-dihydro-4-oxo-6-chinolinyl)propylamin]propionyl]-(9E)-O-hydroximinerythromycin A;
4"-O-[3-[4-(2-Carboxy-6,7-dihydro-1H,5H-pyrid[3,2,1-ij]-1-oxo-9-chinolinyl)propylamin]propionyl]-(9E)-O-hydroximinerythromycin A,
4"-O-{6-[3-(3-Carboxy-1-ethyl-4-oxo-1,4-dihydro-chinolin-6-yl)-propoxy]-hexanoyl}-azithromycin; und
4"-O-{6-[3-(3-Carboxy-1-ethyl-4-oxo-1,4-dihydro-chinolin-6-yl)-propoxy]-hexanoyl}-clarithromycin;
oder ein pharmazeutisch annehmbares Derivat davon.

9. Verfahren zur Herstellung einer Verbindung wie in Anspruch 1 beansprucht, oder ein pharmazeutisch annehmbares Derivat davon, umfassend:
a) Umsetzen einer Verbindung der Formel (II) 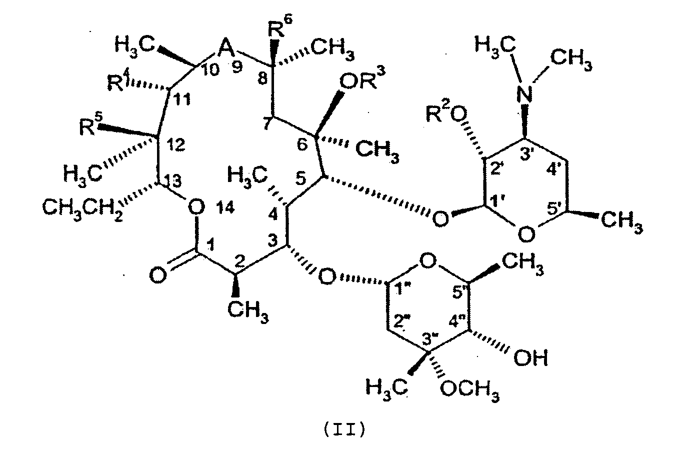
HOC(O)(CH₂)_{d}X^{a}R^{11a} (III)
mit einem in geeigneter Form aktivierten Derivat der Säure (III), worin X^{a} und R^{11a} X und R¹¹ wie in Anspruch 1 definiert sind, oder Gruppen sind, die in X und R¹¹ konvertierbar sind;
b) Umsetzen einer Verbindung der Formel (IV) 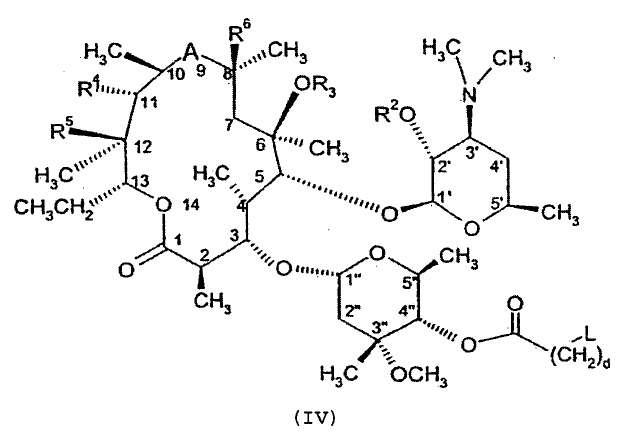 mit einer Verbindung der Formel X^{a}R^{11a}(V), worin R^{11a} R¹¹ ist, wie in Anspruch 1 definiert, oder eine in R¹¹ konvertierbare Gruppe ist, und X^{a} -U(CH₂)ᵥ- ist, oder eine Gruppe, die in -U(CH₂)ᵥ- umwandelbar ist, worin U eine Gruppe ist, die ausgewählt ist aus -N(R³⁰)- und -S-, und L ist eine geeignete Abgangsgruppe, um eine Verbindung der Formel (I) herzustellen, worin U eine Gruppe ist, die ausgewählt ist aus -N(R³⁰)- und -S-; oder
c) Umsetzen einer Verbindung der Formel (VII), mit einer Verbindung der Formel X^{a}R^{11a}(V), 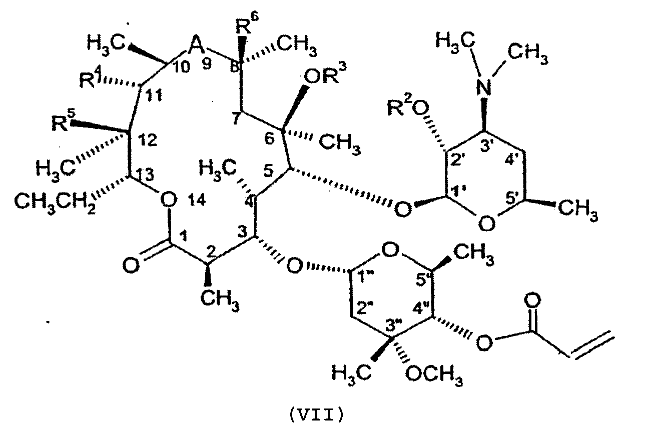
worin R^{11a} R¹¹ ist, wie in Anspruch 1 definiert, oder eine in R¹¹ konvertierbare Gruppe, und X^{a} -U(CH₂)ᵥ- oder eine in X^{a} -U(CH₂)ᵥ- konvertierbare Gruppe ist, worin U eine Gruppe ist, die ausgewählt ist aus -N(R³⁰)- und -S-, um eine Verbindung der Formel (I) herzustellen, worin d 2 ist, und U eine Gruppe ist, die ausgewählt ist aus -N(R³⁰)- und -S-, und danach, falls notwendig, Unterwerfen der erhaltenen Verbindung gegenüber einem oder mehreren der folgenden Arbeitsschritte:
(i) Entfernen der Schutzgruppe R²,
(ii) Umwandeln von X^{a}R^{11a} in XR¹¹, und
(iii) Umwandlung der erhaltenen Verbindung der Formel (I) in ein pharmazeutisch annehmbares Derivat davon.

10. Verbindung wie in irgendeinem der Ansprüche 1 bis 8 beansprucht, oder eines pharmazeutisch annehmbaren Derivats davon, zur Verwendung in der Therapie.

11. Verwendung einer Verbindung wie in irgendeinem der Ansprüche 1 bis 8 beansprucht, oder eines pharmazeutisch annehmbaren Derivats davon, zur Herstellung eines Medikaments zur Verwendung bei der Behandlung oder Prophylaxe systemischer oder topischer microbieller Infektionen des menschlichen oder tierischen Körpers.

12. Pharmazeutische Zusammensetzung, umfassend mindestens eine Verbindung wie in irgendeinem der Ansprüche 1 bis 8 beansprucht, oder ein pharmazeutisch annehmbaren Derivats davon, in Verbindung mit einem pharmazeutisch annehmbaren Excipienten, Verdünnungsmittel und/oder Träger.

13. Verbindung der Formel (IA): 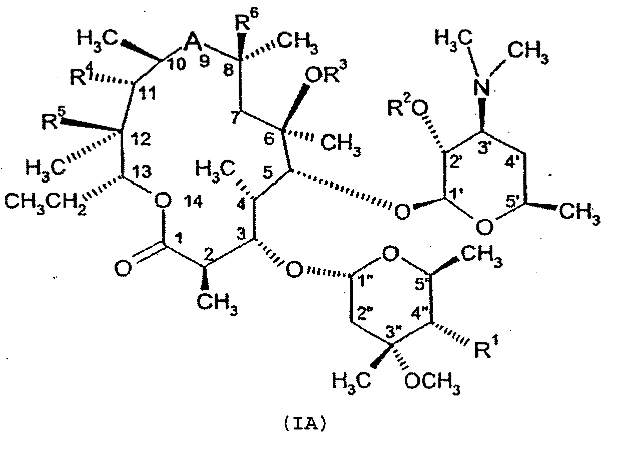 worin
A ein bivalenter Rest ist, ausgewählt aus -C(O)-, -C(O)NH-, -NHC(O)-, -N(R⁷)-CH₂-, -CH₂-N(R⁷)-, -CH(NR⁸R⁹)- und -C(=NR¹⁰)-;
R¹ ist -OC(O)(CH₂)_{d}XR¹¹;
R² ist Wasserstoff oder eine Hydroxyl-Schutzgruppe;
R³ ist Wasserstoff, C₁₋₄-Alkyl oder C₃₋₆-Alkenyl, gegebenenfalls substituiert mit 9- bis 10-gliedrigem fusioniertem bizyklischen Heteroaryl;
R⁴ ist Hydroxy, C₃₋₆-Alkenyloxy, gegebenenfalls substituiert mit einem 9- bis 10-gliedrigen fusionierten bizyklischen Heteroaryl, oder C₁₋₆-Alkoxy, gegebenenfalls substituiert mit C₁₋₆-Alkoxy oder -O(CH₂)ₑNR⁷R¹²,
R⁵ ist Hydroxy, oder
R⁴ und R⁵ bilden zusammen mit den dazwischen liegenden Atomen eine zyklische Gruppe mit der folgenden Struktur: 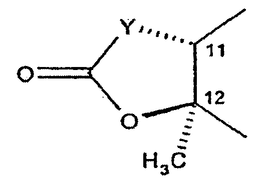 worin Y ein bivalenter Rest ist ausgewählt aus -CH₂-, -CH(CN)-, -O-, -N(R¹³)- und -CH(SR¹³)-;
R⁶ ist Wasserstoff oder Fluor;
R⁷ ist Wasserstoff oder C₁₋₆-Alkyl;
R⁸ und R⁹ sind jeweils unabhängig voneinander Wasserstoff, C₁₋₆-Alkyl, -C(=NR¹⁰)NR¹⁴R¹⁵ oder -C(O)R¹⁴, oder
R⁸ und R⁹ bilden zusammen =CH(CR¹⁴R¹⁵)_{f}-Aryl, =CH(CR¹⁴R¹⁵)_{f}-Heterocyclyl, =CR¹⁴R¹⁵ oder =C(R¹⁴)C(O)OR¹⁴, worin die Alkyl-, Aryl- und
Heterocyclylgruppen gegebenenfalls mit bis zu drei Gruppen substituiert sind, die unabhängig voneinander ausgewählt sind aus R¹⁶;
R¹⁰ ist -OR¹⁷, C₁₋₆-Alkyl, -(CH₂)_{g}-Aryl, -(CH₂)_{g}-Heterocyclyl oder -(CH₂)ₕO(CH₂)ᵢOR⁷, worin jede R¹⁰-Gruppe gegebenenfalls substituiert ist mit bis zu drei Gruppen, die unabhängig ausgewählt sind aus R¹⁶;
R¹¹ ist eine heterozyklische Gruppe mit der folgenden Struktur: 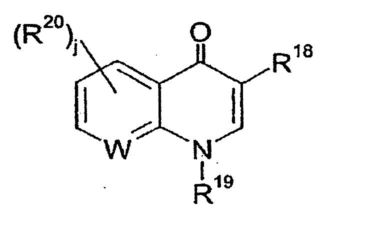 oder 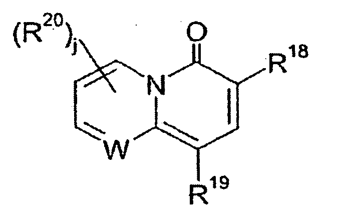 R¹² ist Wasserstoff oder C₁₋₆-Alkyl;
R¹³ ist Wasserstoff oder C₁₋₄-Alkyl, gegebenenfalls substituiert mit einer Gruppe, ausgewählt aus gegebenenfalls substituiertem Phenyl, gegebenenfalls substituierten 5- oder 6-gliedrigen Heteroaryl und gegebenenfalls substituiertem 9- oder 10-gliedrigen fusionierten bizyklischen Heteroaryl; R¹⁴ und R¹⁵ sind jeweils unabhängig voneinander Wasserstoff oder C₁₋₆-Alkyl;
R¹⁶ ist Halogen, Cyano, Nitro, Trifluormethyl, Azido, -C(O)R²¹, -C(O)OR²¹, -OC(O)R²¹, -OC(O)OR²¹, -NR²²C(O)R²³, -C(O)NR²²R²³, - NR²²R²³, Hydroxy, C₁₋₆-Alkyl, -S(O)ₖC₁₋₆-Alkyl, C₁₋₆-Alkoxy, -(CH₂)ₘ-Aryl oder -(CH₂)ₘ-Heteroaryl, worin die Alkoxygruppe gegebenenfalls substituiert ist mit bis zu drei Gruppen, die unabhängig voneinander ausgewählt sind aus -N¹⁴R¹⁵, Halogen und -OR¹⁴, und die Aryl- und Heteroarylgruppen gegebenenfalls substituiert sind mit bis zu fünf Gruppen, die unabhängig voneinander ausgewählt sind aus Halogen, Cyano, Nitro, Trifluormethyl, Azido, C(O)R²⁴, -C(O)OR²⁴, -OC(O)OR²⁴, -NR²⁵C(O)R²⁶, -C(O)NR²⁵R²⁶, -NR²⁵R²⁶, Hydroxy, C₁₋₆-Alkyl und C₁₋₆-Alkoxy;
R¹⁷ ist Wasserstoff, C₁₋₆-Alkyl, C₃₋₇-Cycloalkyl, C₃₋₆-Alkenyl oder eine 5- oder 6-gliedrige heterozyklische Gruppe, worin die Alkyl-, Cycloalkyl-, Alkenyl- und heterozyklischen Gruppen gegebenenfalls substituiert sind mit bis zu drei Substituenten, unabhängig ausgewählt aus gegebenenfalls substituierter 5- oder 6-gliedriger heterozyklischen Gruppe, gegebenenfalls substituiertem 5- oder 6-gliedrigen Heteroaryl, -OR²⁷, -S(O)ₙR²⁷, -NR²⁷R²⁸, -CONR²⁷R²⁸, Halogen und Cyano;
R¹⁸ ist Wasserstoff, -C(O)OR²⁹, -C(O)NHR²⁹, oder -C(O)CH₂NO₂;
R¹⁹ ist Wasserstoff, C₁₋₄-Alkyl, gegebenenfalls substituiert mit Hydroxy oder C₁₋₄-Alkoxy, C₃₋₇-Cycloalkyl, oder gegebenenfalls substituiertem Phenyl oder Benzyl;
R²⁰ ist Halogen, C₁₋₄-Alkyl, C₁₋₄-Thioalkyl, C₁₋₄-Alkoxy, -NH₂, -NH(C₁₋₄-Alkyl) oder -N(C₁₋₄-Alkyl)₂;
R²¹ ist Wasserstoff, C₁₋₁₀-Alkyl, -(CH₂)ₚ-Aryl oder -(CH₂)ₚ-Heteroaryl;
R²² und R²³ sind jeweils unabhängig voneinander Wasserstoff, -OR¹⁴, C₁₋₆-Alkyl, -(CH₂)_{q}-Aryl oder -(CH₂)_{q}-Heterocyclyl;
R₂₄ ist Wasserstoff, C₁₋₁₀-Alkyl, -(CH₂)ᵣ-Aryl oder -(CH₂)ᵣ-Heteroaryl; R²⁵ und R²⁶ sind jeweils unabhängig voneinander Wasserstoff, -OR¹⁴, C₁₋₆-Alkyl, -(CH₂)ₛ-Aryl oder -(CH₂)ₛ-Heterocyclyl;
R²⁷ und R²⁸ sind jeweils unabhängig voneinander Wasserstoff, C₁₋₄-Alkyl oder C₁₋₄-Alkoxy-C₁₋₄-alkyl;
R²⁹ ist Wasserstoff, C₁₋₆-Alkyl, gegebenenfalls substituiert mit bis zu drei Gruppen, die unabhängig voneinander ausgewählt sind aus Halogen, C₁₋₄-Alkoxy, -OC(O)C₁₋₆-Alkyl, -OC(O)OC₁₋₆-Alkyl, -C(O)NR³²R³³, und -NR³²R³³, -(CH₂)_{w}C₃₋₇-Cycloalkyl, C₃₋₆-Alkenyl oder C₃₋₆-Alkynyl;
R³⁰ ist Wasserstoff, C₁₋₄-Alkyl, C₃₋₇-Cycloalkyl, gegebenenfalls substituiertes Phenyl oder Benzyl, Acetyl oder Benzoyl;
R³¹ ist Wasserstoff oder R²⁰, oder R³¹ und R¹⁹ sind verbunden, um den bivalenten Rest -O(CH₂)₂- oder -(CH₂)ₜ- zu bilden;
R³² und R³³ sind jeweils unabhängig voneinander Wasserstoff oder C₁₋₆-Alkyl, gegebenenfalls substituiert mit -C(O)OC₁₋₆-Alkyl, oder
R³² und R³³ bilden zusammen mit dem Stickstoffatom, an das sie gebunden sind, eine 5- oder 6-gliedrige heterozyklische Gruppe, die gegebenenfalls ein zusätzliches Heteroatom enthält, ausgewählt aus Sauerstoff, Stickstoff und Schwefel;
X ist -U(CH₂)ᵥ-;
U ist ein divalenter Rest ausgewählt aus -N(R³⁰)-, -O-, -S(O)_{z}-, -N(R³⁰)C(O)-, -C(O)N(R³⁰)- und -N[C(O)R³⁰]-;
W ist -C(R³¹)- oder ein Stickstoffatom;
d ist eine ganze Zahl von 1 bis 5,
e ist eine ganze Zahl von 2 bis 4,
f, g, h, m, p, q, r, s und w sind jeweils unabhängig voneinander ganze Zahlen von 0 bis 4;
i ist eine ganze Zahl von 1 bis 6;
j, k, n und z sind unabhängig voneinander ganze Zahlen von 0 bis 2;
t ist 2 oder 3;
v ist eine ganze Zahl von 2 bis 8;
oder ein pharmazeutisch annehmbares Derivat davon.

14. Verbindung der Formel (IB) 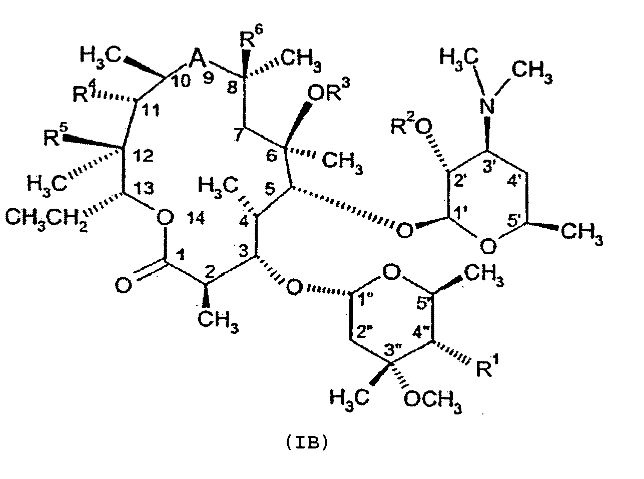 worin
A ein bivalenter Rest ist, ausgewählt aus -C(O)-, -C(O)NH-, -NHC(O)-, -N(R⁷)-CH₂-, -CH₂-N(R⁷)-, -CH(NR⁸R⁹)- und -C(=NR¹⁰)-;
R¹ ist -OC(O) (CH₂)_{d}XR¹¹;
R² ist Wasserstoff oder eine Hydroxyl-Schutzgruppe;
R³ ist Wasserstoff, C₁₋₄-Alkyl oder C₃₋₆-Alkenyl, gegebenenfalls substituiert mit einem 9- bis 10-gliedrigen fusionierten bizyklischen Heteroaryl;
R⁴ ist Hydroxy, C₃₋₆-Alkenyloxy, gegebenenfalls substituiert mit einem 9- bis 10-gliedrigen fusionierten bizyklischen Heteroaryl, oder C₁₋₆-Alkoxy, gegebenenfalls substituiert mit C₁₋₆-Alkoxy oder -O(CH₂)ₑNR⁷R¹²;
R⁵ ist Hydroxy, oder
R⁴ und R⁵ bilden zusammen mit den dazwischen liegenden Atomen eine zyklische Gruppe mit der folgenden Struktur: 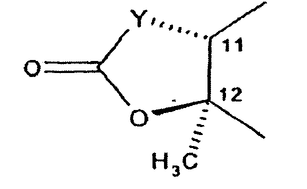 worin Y ein bivalenter Rest ist ausgewählt aus -CH₂-, -CH(CN)-, -O-, -N(R¹³)- und -CH(SR¹³)-;
R⁶ ist Wasserstoff oder Fluor;
R⁷ ist Wasserstoff oder C₁₋₆-Alkyl;
R⁸ und R⁹ sind unabhängig voneinander Wasserstoff, C₁₋₆-Alkyl, -C(=NR¹⁰)NR¹⁴R¹⁵ oder -C(O)R¹⁴, oder
R⁸ und R⁹ bilden zusammen =CH(CR¹⁴R¹⁵)_{f}-Aryl, =CH(CR¹⁴R¹⁵)_{f}-Heterocyclyl, =CR¹⁴R¹⁵ oder =C(R¹⁴)C(O)OR¹⁴, worin die Alkyl-, Aryl- und
Heterocyclylgruppen gegebenenfalls mit drei Gruppen substituiert sind, die unabhängig voneinander ausgewählt sind aus R¹⁶;
R¹⁰ ist -OR¹⁷, C₁₋₆-Alkyl, -(CH₂)_{g}-Aryl, -(CH₂)_{g}-Heterocyclyl oder -(CH₂)ₕO(CH₂)ᵢOR⁷, worin jede R¹⁰-Gruppe gegebenenfalls substituiert ist durch drei Gruppen, die unabhängig voneinander ausgewählt sind aus R¹⁶;
R¹¹ ist eine heterozyklische Gruppe mit der folgenden Struktur: 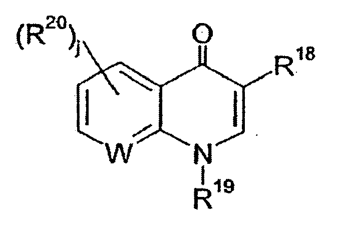 oder 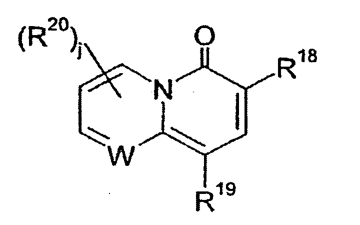 R¹² ist Wasserstoff oder C₁₋₆-Alkyl;
R¹³ ist Wasserstoff oder C₁₋₄-Alkyl, substituiert mit einer Gruppe ausgewählt aus gegebenenfalls substituiertem Phenyl, gegebenenfalls substituiertem 5- oder 6-gliedrigen Heteroaryl und gegebenenfalls substituiertem 9- oder 10-gliedrigen fusionierten bizyklischen Heteroaryl; R¹⁴ und R¹⁵ sind jeweils unabhängig Wasserstoff oder C₁₋₆-Alkyl;
R¹⁶ ist Halogen, Cyano, Nitro, Trifluormethyl, Azido, -C(O)R²¹, -C(O)OR²¹, -OC(O)R²¹, -OC(O)OR²¹, -NR²²C(O)R²³, -C(O)NR²²R²³, - NR²²R²³, Hydroxy, C₁₋₆-Alkyl, -S(O)ₖC₁₋₆-Alkyl, C₁₋₆-Alkoxy, -(CH₂)ₘ-Aryl oder -(CH₂)ₘ-Heteroaryl, worin die Alkoxygruppe gegebenenfalls substituiert ist mit bis zu drei Gruppen, unabhängig voneinander ausgewählt aus -N¹⁴R¹⁵, Halogen und -OR¹⁴, und die Aryl- und Heteroarylgruppen sind gegebenenfalls substituiert mit bis zu fünf Gruppen, unabhängig ausgewählt aus Halogen, Cyano, Nitro, Trifluormethyl, Azido, C(O)R²⁴, -C(O)OR²⁴, -OC(O)OR²⁴, -NR²⁵C(O)R²⁶, -C(O)NR²⁵R²⁶, -NR²⁵R²⁶, Hydroxy, C₁₋₆-Alkyl und C₁₋₆-Alkoxy;
R¹⁷ ist Wasserstoff, C₁₋₆-Alkyl, C₃₋₇-Cycloalkyl, C₃₋₆-Alkenyl oder eine 5- oder 6-gliedrige heterozyklische Gruppe, worin die Alkyl-, Cycloalkyl-, Alkenyl- und heterozyklischen Gruppen gegebenenfalls mit bis zu drei Substituenten substituiert sind, die unabhängig voneinander ausgewählt sind aus gegebenenfalls substituierten 5- oder 6-gliedrigen heterozyklischen Gruppen, gegebenenfalls substituierten 5- oder 6-gliedrigen Heteroaryl, -OR²⁷, -S(O)ₙR²⁷, -NR²⁷R²⁸, -CONR²⁷R²⁸, Halogen und Cyano;
R¹⁸ ist Wasserstoff, -C(O)OR²⁹, -C(O)NHR²⁹ oder -C(O)CH₂NO₂;
R¹⁹ ist Wasserstoff, C₁₋₄-Alkyl, gegebenenfalls substituiert mit Hydroxy oder C₁₋₄-Alkoxy, C₃₋₇-Cycloalkyl, oder gegebenenfalls substituiertem Phenyl oder Benzyl;
R²⁰ ist Halogen, C₁₋₄-Alkyl, C₁₋₄-Thioalkyl, C₁₋₄-Alkoxy, -NH₂, -NH(C₁₋₄-Alkyl) oder -N(C₁₋₄-Alkyl)₂;
R²¹ ist Wasserstoff, C₁₋₁₀-Alkyl, -(CH₂)ₚ-Aryl oder (CH₂)ₚ-Heteroaryl;
R²² und R²³ sind jeweils unabhängig Wasserstoff, -OR¹⁴, C₁₋₆-Alkyl, -(CH₂)_{q}-Aryl oder -(CH₂)_{q}-Heterocyclyl;
R₂₄ ist Wasserstoff, C₁₋₁₀-Alkyl, -(CH₂)ᵣ-Aryl oder -(CH₂)ᵣ-Heteroaryl;
R²⁵ und R²⁶ sind jeweils unabhängig Wasserstoff, -OR¹⁴, C₁₋₆-Alkyl, -(CH₂)ₛ-Aryl oder -(CH₂)ₛ-Heterocyclyl;
R²⁷ und R²⁸ sind jeweils unabhängig Wasserstoff, C₁₋₄-Alkyl oder C₁₋₄-Alkoxy-C₁₋₄-alkyl;
R²⁹ ist Wasserstoff oder C₁₋₆-Alkyl, gegebenenfalls substituiert mit bis zu drei Gruppen, unabhängig ausgewählt aus Halogen, C₁₋₄-Alkoxy, -OC(O)C₁₋₆-Alkyl und -OC(O)OC₁₋₆-Alkyl;
R³⁰ ist Wasserstoff, C₁₋₄-Alkyl, C₃₋₇-Cycloalkyl, gegebenenfalls substituiertem Phenyl oder Benzyl, Acetyl oder Benzoyl;
R³¹ ist Wasserstoff oder R²⁰, oder R³¹ und R¹⁹ sind verbunden, um den bivalenten Rest -O(CH₂)₂- oder -(CH₂)ₜ- zu bilden;
X ist -U(CH₂)ᵥ-;
U ist ein divalenter Rest ausgewählt aus -N(R³⁰)-, -O-, -S(O)₂-, -N(R³⁰)C(O)-, -C(O)N(R³⁰)- und -N[C(O)R³⁰]-;
W ist -C(R³¹)- oder ein Stickstoffatom;
d ist eine ganze Zahl von 1 bis 5,
e ist eine ganze Zahl von 2 bis 4,
f, g, h, m, p, q, r und s sind jeweils unabhängig voneinander ganze Zahlen von 0 bis 4;
i ist eine ganze Zahl von 1 bis 6;
j, k, n und z sind jeweils unabhängig voneinander ganze Zahlen von 0 bis 2;
t ist 2 oder 3;
v ist eine ganze Zahl von 2 bis 8;
oder ein pharmazeutisch annehmbares Derivat davon.

## Revendications

1. Composé de formule (I) : 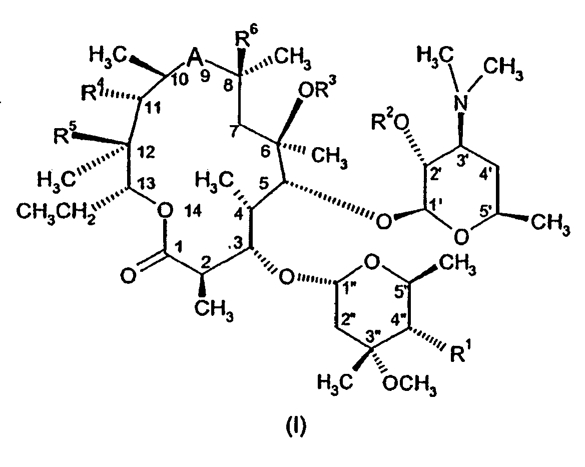 dans laquelle :
A est un radical bivalent choisi parmi -C(O)-, -C(O)NH-, -NHC(O)-, -N(R⁷)-CH₂-, -CH₂-N(R⁷)-, -CH(NR⁸R⁹)- et-C(=NR¹⁰)- ;
R¹ est -OC(O)(CH₂)_{d}XR¹¹ ;
R² est un atome d'hydrogène ou un groupe hydroxy protecteur ;
R³ est un atome d'hydrogène, un groupe alkyle en C₁₋₄, ou alcényle en C₃₋₆ éventuellement substitué par un groupe hétéroaryle bicyclique condensé de 9 à 10 chaînons ;
R⁴ est un groupe hydroxy, alcényloxy en C₃₋₆ éventuellement substitué par un groupe hétéroaryle bicyclique condensé de 9 à 10 chaînons, ou alcoxy en C₁₋₆ éventuellement substitué par un groupe alcoxy en C₁₋₆ ou -O(CH₂)ₑNR⁷R¹²,
R⁵ est un groupe hydroxy, ou bien
R⁴ et R⁵ pris ensemble avec les atomes intermédiaires forment un groupe cyclique ayant la structure suivante : 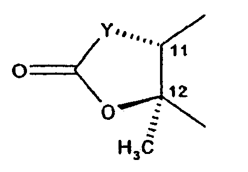 dans laquelle Y est un radical bivalent choisi parmi -CH₂-, -CH(CN), -O-, -N(R¹³)- et -CH(SR¹³)- ;
R⁶ est un atome d'hydrogène ou de fluor ;
R⁷ est un atome d'hydrogène ou un groupe alkyle en C₁₋₆,
R⁸ et R⁹ sont chacun indépendamment un atome d'hydrogène, un groupe alkyle en C₁₋₆, -C(=NR¹⁰)NR¹⁴R¹⁵ ou -C(O)R¹⁴, ou bien
R⁸ et R⁹ forment ensemble =CH(CR¹⁴R¹⁵)_{f}aryle, =CH(CR¹⁴R¹⁵)_{f}hétérocyclyle, =CR¹⁴R¹⁵ ou =C(R¹⁴)C(O)OR¹⁴ dans lesquels les groupes alkyle, aryle et hétérocyclyle sont éventuellement substitués par un maximum de trois groupes choisis indépendamment parmi les groupes R¹⁶ ;
R¹⁰ est -OR¹⁷, alkyle en C₁₋₆, -(CH₂)_{g}aryle, -(CH₂)_{g}hétérocyclyle ou -(CH₂)ₕO(CH₂)ᵢOR⁷ où chaque groupe R¹⁰ est éventuellement substitué par un maximum de trois groupes choisis indépendamment parmi les groupes R¹⁶ ;
R¹¹ est un groupe hétérocyclique ayant la structure suivante : 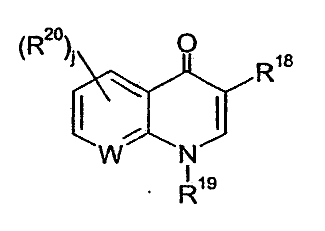 ou 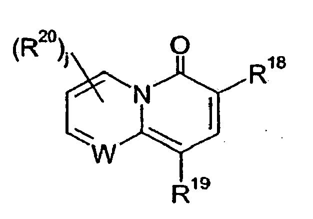
R¹² est un atome d'hydrogène ou un groupe alkyle en C₁₋₆ ;
R¹³ est un atome d'hydrogène ou un groupe alkyle en C₁₋₄, éventuellement substitué par un groupe choisi parmi un groupe phényle éventuellement substitué, hétéroaryle de 5 ou 6 chaînons éventuellement substitué et hétéroaryle bicyclique condensé de 9 à 10 chaînons éventuellement substitué ;
R¹⁴ et R¹⁵ sont chacun indépendamment un atome d'hydrogène ou un groupe alkyle en C₁₋₆ ;
R¹⁶ est un atome d'halogène, un groupe cyano, nitro, trifluorométhyle, azido, -C(O)R²¹, -C(O)OR²¹, -OC(O)R²¹, -OC(O)OR²¹, -NR²²C(O)R²³, -C(O)NR²²R²³, -NR²²R²³, hydroxy, alkyle en C₁₋₆, -S(O)ₖalkyle en C₁₋₆, alcoxy en C₁₋₆, -(CH₂)ₘaryle ou -(CH₂)ₘhétéroaryle dans lequel le groupe alcoxy est éventuellement substitué par un maximum de trois groupes choisis indépendamment parmi -NR¹⁴R¹⁵, halogène et -OR¹⁴ et les groupes aryle et héréroaryle sont éventuellement substitués par un maximum de cinq groupes choisis indépendamment parmi un atome d'halogène, un groupe cyano, nitro, trifluorométhyle, azido, -C(O)R²⁴, -C(O)OR²⁴, -OC(O)OR²⁴, -NR²⁵C(O)R²⁶, -C(O)NR²⁵R²⁶, -NR²⁵R²⁶, hydroxy, alkyle en C₁₋₆ et alcoxy en C₁₋₆ ;
R¹⁷ est un atome d'hydrogène, un groupe alkyle en C₁₋₆, cycloalkyle en C₃₋₇, alcényle en C₃₋₆ ou un groupe hétérocyclique de 5 ou 6 chaînons, dans lequel les groupes alkyle, cycloalkyle, alcényle et hétérocyclique sont éventuellement substitués par un maximum de trois substituants choisis indépendamment parmi un groupe hétérocyclique de 5 ou 6 chaînons éventuellement substitué, hétéroaryle de 5 ou 6 chaînons éventuellement substitué, -OR²⁷, -S(O)ₙR²⁷, -NR²⁷R²⁸, -CONR²⁷R²⁸, halogène et cyano ;
R¹⁸ est un atome d'hydrogène, -C(O)OR²⁹, -C(O)NHR²⁹, -C(O)CH₂NO₂ ou -C(O)CH₂SO₂R⁷ ;
R¹⁹ est un atome d'hydrogène, un groupe alkyle en C₁₋₄, éventuellement substitué par un groupe hydroxy ou alcoxy en C₁₋₄, cycloalkyle en C₃₋₇, ou benzyle ou phényle éventuellement substitué ;
R²⁰ est un atome d'halogène, un groupe alkyle en C₁₋₄, thioalkyle en C₁₋₄, alcoxy en C₁₋₄, -NH₂, -NHalkyle en C₁₋₄ ou -N(alkyle en C₁₋₄)₂ ;
R²¹ est un atome d'hydrogène, un groupe alkyle en C₁₋₁₀, -(CH₂)ₚaryle ou -(CH₂)ₚhétéroaryle ;
R²² et R²³ sont chacun indépendamment un atome d'hydrogène, -OR¹⁴, alkyle en C₁₋₆, -(CH₂)_{q}aryle ou -(CH₂)_{q}hétérocyclyle ;
R²⁴ est un atome d'hydrogène, un groupe alkyle en C₁₋₁₀, -(CH₂)ᵣaryle ou -(CH₂)ᵣhétéroaryle ;
R²⁵ et R²⁶ sont chacun indépendamment un atome d'hydrogène, -OR¹⁴, alkyle en C₁₋₆, -(CH₂)ₛaryle ou -(CH₂)ₛhétérocyclyle ;
R²⁷ et R²⁸ sont chacun indépendamment un atome d'hydrogène, un groupe alkyle en C₁₋₄ ou alcoxy(en C₁₋₄)alkyle en C₁₋₄ ;
R 29 est un atome d'hydrogène, un groupe alkyle en C₁₋₆ éventuellement substitué par un maximum de trois groupes choisis indépendamment parmi un atome d'halogène, un groupe cyano, alcoxy en C₁₋₄ éventuellement substitué par un groupe phényle ou alcoxy en C₁₋₄, -C(O)alkyle en C₁₋₆, -C(O)Oalkyle en C₁₋₆, -OC(O)alkyle en C₁₋₆, -OC(O)Oalkyle en C₁₋₆, -C(O)NR³²R³³, -NR³²R³³ et phényle éventuellement substitué par un groupe nitro ou -C(O)Oalkyle en C₁₋₆, -(CH₂)_{w}cycloalkyle en C₃₋₇, -(CH₂)_{w}hétérocyclyle, -(CH₂)_{w}hétéroaryle, -(CH₂)_{w}aryle, alcényle en C₃₋₆ ou alcynyle en C₃₋₆ ;
R³⁰ est un atome d'hydrogène, un groupe alkyle en C₁₋₄, cycloalkyle en C₃₋₇, benzyle ou phényle éventuellement substitué, acétyle ou benzoyle ;
R³¹ est un atome d'hydrogène ou R²⁰, ou bien R³¹ et R¹⁹ sont reliés pour former le radical bivalent -O(CH₂)₂- ou -(CH₂)ₜ⁻ ;
R³² et R³³ sont chacun indépendamment un atome d'hydrogène ou un groupe alkyle en C₁₋₆ éventuellement substitué par un groupe phényle ou -C(O)Oalkyle en C₁₋₆, ou bien
R³² et R³³ forment avec l'atome d'azote auquel ils sont liés, un groupe hétérocyclique de 5 ou 6 chaînons contenant éventuellement un hétéroatome supplémentaire choisi parmi l'oxygène, l'azote et le soufre ;
X est -U(CH₂)ᵥ-;
U est un radical bivalent choisi parmi -N(R³⁰)-, -O-, -S(O)₂, -N(R³⁰)C(O)-, -C(O)N(R³⁰)- et -N[C(O)R³⁰]- ;
W est -C(R³¹)- ou un atome d'azote ;
d est un entier de 1 à 5 ;
e est un entier de 2 à 4 ;
f, g, h, m, p, q, r, s et w sont chacun indépendamment des entiers de 0 à 4 ;
i est un entier de 1 à 6 ;
j, k, n et z sont chacun indépendamment des entiers de 0 à 2 ;
t est 2 ou 3 ;
v est un entier de 1 à 8 ;
ou dérivé de celui-ci acceptable du point de vue pharmaceutique.

2. Composé selon la revendication 1, dans lequel A est-C(O)-.

3. Composé selon la revendication 1 ou la revendication 2, dans lequel X est -N(R³⁰)(CH₂)ᵥ-.

4. Composé selon l'une quelconque des revendications précédentes, dans lequel d est 2.

5. Composé selon l'une quelconque des revendications précédentes, dans lequel R¹¹ est un groupe hétérocyclique ayant la formule suivante 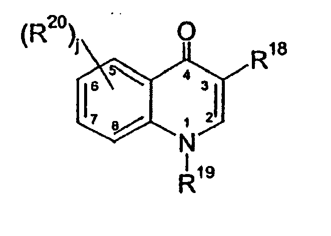 dans laquelle le groupe hétérocyclique est lié en position 6 ou 7 et j, R¹⁸, R¹⁹ et R²⁰ sont tels que définis dans la revendication 1.

6. Composé selon l'une quelconque des revendications 1 à 4, dans lequel R¹¹ est un groupe hétérocyclique ayant la formule suivante : 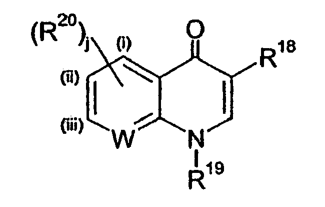 dans laquelle W est -C(R³¹)-, où R³¹ et R¹⁹ sont réunis pour former le radical bivalent -O(CH₂)₂- ou -(CH₂)ₜ- et ledit groupe hétérocyclique est lié en position (i), (ii) ou (iii) et j, R¹⁸ et R²⁰ sont tels que définis dans la revendication 1.

7. Composé selon la revendication 1 tel que défini dans l'un quelconque des exemples 1 à 44, ou dérivé de celui-ci acceptable du point de vue pharmaceutique.

8. Composé choisi parmi les :
4"-O-{3-[3-(3-carboxy-1,4-dihydro-1-éthyl-4-oxo-6-quinoléinyl)propylamino]propionyl}-6-O-méthylérythromycine A ;
4"-O-{3-[3-(3-carboxy-1,4-dihydro-1-éthyl-4-oxo-6-quinoléinyl)propylamino]propionyl}-azithromycine-11,12-carbonate ;
4"-O-{3-[3-(3-carboxy-1,4-dihydro-1-éthyl-4-oxo-6-quinoléinyl)propylamino]propionyl}-6-O-méthyl-11-désoxy-11-(R)-amino-érythromycine A 11,12-carbamate ;
4"-O-[3-[4-(2-carboxy-6,7-dihydro-*1H*,*5H*-pyrido[3,2,1-ij]-1-oxo-9-quinoléinyl)propylamino]propionyl]-6-O-méthyl érythromycine A ;
4"-O-[3-[4-(3-carboxy-1-éthyl-1,4-dihydro-4-oxo-6-quinoléinyl)propylamino]propionyl]-(9E)-O-({[2-(méthyloxy)éthyl]oxy}méthanoximino érythromycine A ;
4"-O-[3-[4-(3-carboxy-1-éthyl-1,4-dihydro-4-oxo-6-quinoléinyl)propylamino]propionyl]-(9E)-O-hydroximino érythromycine A ;
4"-O-[3-[4-(2-carboxy-6,7-dihydro-*1H*,*5H*-pyrido[3,2,1-ij]-1-oxo-9-quinoléinyl)propylamino]propionyl]-(9E)-O-hydroximino érythromycine A ;
4"-O-{6-[3-(3-carboxy-1-éthyl-4-oxo-1,4-dihydro-quinoléin-6-yl)propoxy]hexanoyl}-azithromycine ; et
4"-O-{6-[3-(3-carboxy-1-éthyl-4-oxo-1,4-dihydro-quinoléin-6-yl)propoxy]hexanoyl}-clarithromycine ;
ou un dérivé de ceux-ci acceptable du point de vue pharmaceutique.

9. Procédé de préparation d'un composé selon la revendication 1 ou d'un dérivé de celui-ci acceptable du point de vue pharmaceutique, qui comprend :
a) la réaction d'un composé de formule (II) 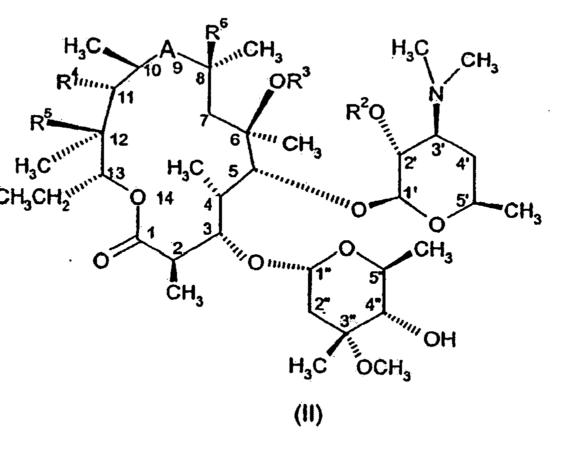
HOC(O)(CH₂)_{d}X^{a}R^{11a} (III)
avec un dérivé activé convenable de l'acide (III), dans lequel X^{a} et R^{11a} sont X et R¹¹ tels que définis dans la revendication 1 ou des groupes pouvant être convertis en X et R¹¹ ;
b) la réaction d'un composé de formule (IV) : 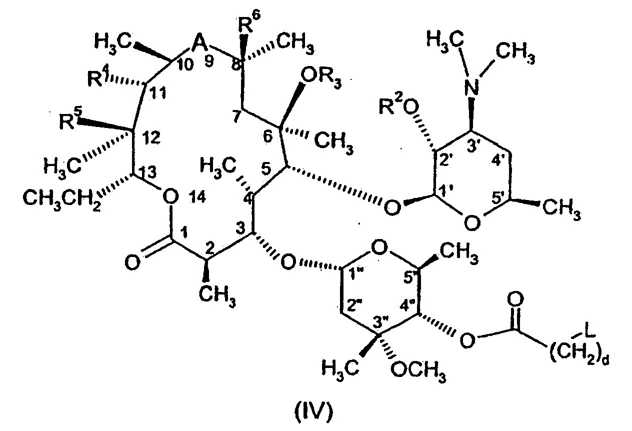 avec un composé de formule X^{a}R^{11a} (V), dans laquelle R^{11a} est R¹¹ tel que défini dans la revendication 1 ou un groupe pouvant être converti en R¹¹ et X^{a} est -U(CH₂)ᵥ- ou un groupe pouvant être converti en-U(CH₂)ᵥ- dans lequel U est un groupe choisi parmi -N(R³⁰)- et -S-, et L est un groupe mobile convenable, pour produire un composé de formule (I) dans lequel U est un groupe choisi parmi -N(R³⁰)- et -S- ; ou bien
c) la réaction d'un composé de formule (VII) : 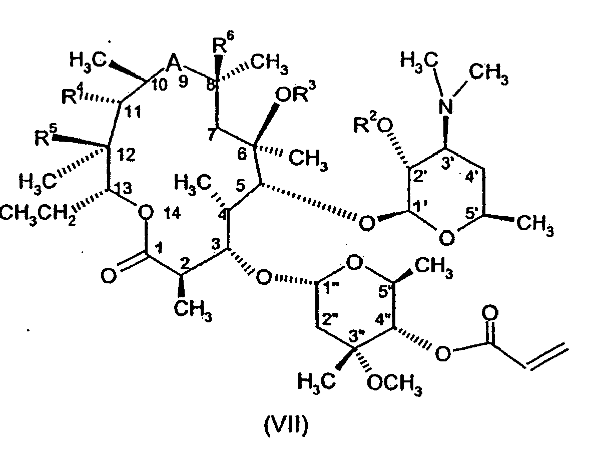 avec un composé de formule X^{a}R^{11a} (V), dans laquelle R^{11a} est R¹¹ tel que défini dans la revendication 1 ou un groupe pouvant être converti en R¹¹ et X^{a} est -U(CH₂)ᵥ- ou un groupe pouvant être converti en-U(CH₂)ᵥ- dans lequel U est un groupe choisi parmi -N(R³⁰)- et -S-, pour produire un composé de formule (I) dans lequel d est 2 et U est un groupe choisi parmi -N(R³⁰)- et -S-, et ensuite, si nécessaire, la soumission du composé résultant à une ou plusieurs des opérations suivantes :
i) élimination du groupe protecteur R²,
ii) conversion de X^{a}R^{11a} en XR¹¹, et
iii) conversion du composé résultant de formule (I) en un dérivé de celui-ci acceptable du point de vue pharmaceutique.

10. Composé selon l'une quelconque des revendications 1 à 8 ou dérivé de celui-ci acceptable du point de vue pharmaceutique, utile en thérapie.

11. Utilisation d'un composé selon l'une quelconque des revendications 1 à 8 ou d'un dérivé de celui-ci acceptable du point de vue pharmaceutique, dans la fabrication d'un médicament destiné au traitement ou à la prophylaxie d'infections microbiennes systémiques ou topiques dans un corps humain ou animal.

12. Composition pharmaceutique comprenant au moins un composé selon l'une quelconque des revendications 1 à 8 ou un dérivé de celui-ci acceptable du point de vue pharmaceutique, en association avec un excipient, diluant et/ou support acceptables du point de vue pharmaceutique.

13. Composé de formule (IA) : 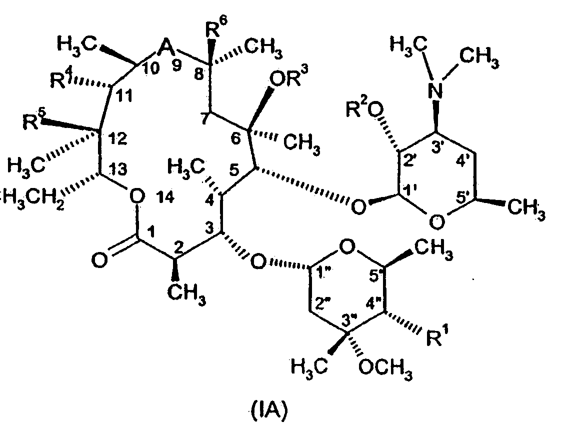 dans laquelle :
A est un radical bivalent choisi parmi -C(O)-, -C(O)NH-, -NHC(O)-, -N(R⁷)-CH₂-, -CH₂-N(R⁷)-, -CH(NR⁸R⁹)- et-C(=NR¹⁰)- ;
R¹ est -OC(O)(CH₂)_{d}XR¹¹ ;
R² est un atome d'hydrogène ou un groupe hydroxy protecteur ;
R³ est un atome d'hydrogène, un groupe alkyle en C₁₋₄ ou alcényle en C₃₋₆ éventuellement substitué par un groupe hétéroaryle bicyclique condensé de 9 à 10 chaînons ;
R⁴ est un groupe hydroxy, alcényloxy en C₃₋₆ éventuellement substitué par un groupe hétéroaryle bicyclique condensé de 9 à 10 chaînons, ou alcoxy en C₁₋₆ éventuellement substitué par un groupe alcoxy en C₁₋₆ ou -O(CH₂)ₑNR⁷R¹²;
R⁵ est un groupe hydroxy, ou bien
R⁴ et R⁵ pris ensemble avec les atomes intermédiaires forment un groupe cyclique ayant la structure suivante : 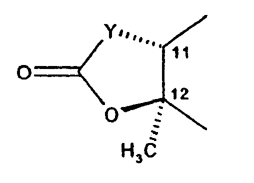 dans laquelle Y est un radical bivalent choisi parmi -CH₂-, -CH(CN), -O-, -N(R¹³)- et -CH(SR¹³)- ;
R⁶ est un atome d'hydrogène ou de fluor ;
R⁷ est un atome d'hydrogène ou un groupe alkyle en C₁₋₆,
R⁸ et R⁹ sont chacun indépendamment un atome d'hydrogène, un groupe alkyle en C₁₋₆, -C(=NR¹⁰)NR¹⁴R¹⁵ ou -C(O)R¹⁴, ou bien
R⁸ et R⁹ forment ensemble =CH(CR¹⁴R¹⁵)_{f}aryle, =CH(CR¹⁴R¹⁵)_{f}hétérocyclyle, =CR¹⁴R¹⁵ ou =C(R¹⁴)C(O)OR¹⁴ dans lesquels les groupes alkyle, aryle et hétérocyclyle sont éventuellement substitués par un maximum de trois groupes choisis indépendamment parmi les groupes R¹⁶ ;
R¹⁰ est -OR¹⁷, alkyle en C₁₋₆, -(CH₂)_{g}aryle, -(CH₂)_{g}hétérocyclyle ou -(CH₂)ₕO(CH₂)ᵢOR⁷ où chaque groupe R¹⁰ est éventuellement substitué par un maximum de trois groupes choisis indépendamment parmi les groupes R¹⁶ ;
R¹¹ est un groupe hétérocyclique ayant la structure suivante : 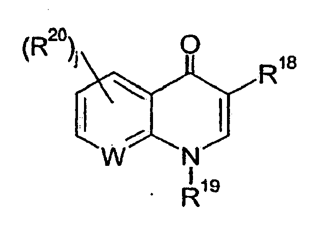 ou 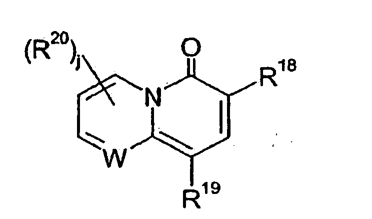
R¹² est un atome d'hydrogène ou un groupe alkyle en C₁₋₆ ;
R¹³ est un atome d'hydrogène ou un groupe alkyle en C₁₋₄, éventuellement substitué par un groupe choisi parmi un groupe phényle éventuellement substitué, hétéroaryle de 5 ou 6 chaînons éventuellement substitué et hétéroaryle bicyclique condensé de 9 à 10 chaînons éventuellement substitué ;
R¹⁴ et R¹⁵ sont chacun individuellement un atome d'hydrogène ou un groupe alkyle en C₁₋₆ ;
R¹⁶ est un atome d'halogène, un groupe cyano, nitro, trifluorométhyle, azido, -C(O)R²¹, -C(O)OR²¹, -OC(O)R²¹, -OC(O)OR²¹, -NR²²C(O)R²³, -C(O)NR²²R²³, -NR²²R²³, hydroxy, alkyle en C₁₋₆, -S(O)ₖalkyle en C₁₋₆, alcoxy en C₁₋₆, -(CH₂)ₘaryle ou -(CH₂)ₘhétéroaryle dans lequel le groupe alcoxy est éventuellement substitué par un maximum de trois groupes choisis indépendamment parmi -NR¹⁴R¹⁵, halogène et -OR¹⁴ et les groupes aryle et héréroaryle sont éventuellement substitués par un maximum de cinq groupes choisis indépendamment parmi un atome d'halogène, un groupe cyano, nitro, trifluorométhyle, azido, -C(O)R²⁴, -C(O)OR²⁴, -OC(O)OR²⁴, -NR²⁵C(O)R²⁶, -C(O)NR²⁵R²⁶, -NR²⁵R²⁶, hydroxy, alkyle en C₁₋₆ et alcoxy en C₁₋₆ ;
R¹⁷ est un atome d'hydrogène, un groupe alkyle en C₁₋₆, cycloalkyle en C₃₋₇, alcényle en C₃₋₆ ou un groupe hétérocyclique de 5 ou 6 chaînons, dans lequel les groupes alkyle, cycloalkyle, alcényle et hétérocyclique sont éventuellement substitués par un maximum de trois substituants choisis indépendamment parmi un groupe hétérocyclique de 5 ou 6 chaînons éventuellement substitué, hétéroaryle de 5 ou 6 chaînons éventuellement substitué, -OR²⁷ -S(O)ₙR²⁷, -NR²⁷R²⁸, -CONR²⁷R²⁸, halogène et cyano ;
R¹⁸ est un atome d'hydrogène, -C(O)OR²⁹, -C(O)NHR²⁹ ou -C(O)CH₂NO₂;
R¹⁹ est un atome d'hydrogène, un groupe alkyle en C₁₋₄ éventuellement substitué par un groupe hydroxy ou alcoxy en C₁₋₄, cycloalkyle en C₃₋₇, ou benzyle ou phényle éventuellement substitué ;
R²⁰ est un atome d'halogène, un groupe alkyle en C₁₋₄, thioalkyle en C₁₋₄, alcoxy en C₁₋₄, -NH₂, -NH(alkyle en C₁₋₄) ou-N(alkyle en C₁₋₄)₂ ;
R²¹ est un atome d'hydrogène, un groupe alkyle en C₁₋₁₀, -(CH₂)ₚaryle ou -(CH₂)ₚhétéroaryle ;
R²² et R²³ sont chacun indépendamment un atome d'hydrogène, -OR¹⁴, alkyle en C₁₋₆, -(CH₂)_{q}aryle ou -(CH₂)_{q}hétérocyclyle ;
R²⁴ est un atome d'hydrogène, un groupe alkyle en C₁₋₁₀, -(CH₂)ᵣaryle ou -(CH₂)ᵣhétéroaryle ;
R²⁵ et R²⁶ sont chacun indépendamment un atome d'hydrogène, -OR¹⁴, alkyle en C₁₋₆, -(CH₂)ₛaryle ou -(CH₂)ₛhétérocyclyle ;
R²⁷ et R²⁸ sont chacun indépendamment un atome d'hydrogène, un groupe alkyle en C₁₋₄ ou alcoxy(en C₁₋₄)alkyle en C₁₋₄ ;
R²⁹ est un atome d'hydrogène, un groupe alkyle en C₁₋₆ éventuellement substitué par un maximum de trois groupes choisis indépendamment parmi un atome d'halogène, un groupe alcoxy en C₁₋₄, -OC(O)alkyle en C₁₋₆, -OC(O)Oalkyle en C₁₋₆, -C(O)NR³²R³³ et -NR³²R³³, -(CH₂)_{w}cycloalkyle en C₃₋₇, alcényle en C₃₋₆ ou alcynyle en C₃₋₆ ;
R³⁰ est un atome d'hydrogène, un groupe alkyle en C₁₋₄, cycloalkyle en C₃₋₇, benzyle ou phényle éventuellement substitué, acétyle ou benzoyle ;
R³¹ est un atome d'hydrogène ou R²⁰, ou bien R³¹ et R¹⁹ sont reliés pour former le radical bivalent -O(CH₂)₂- ou -(CH₂)ₜ⁻ ;
R³² et R³³ sont chacun indépendamment un atome d'hydrogène ou un groupe alkyle en C₁₋₆ éventuellement substitué par un groupe -C(O)Oalkyle en C₁₋₆, ou bien
R³² et R³³ forment avec l'atome d'azote auquel ils sont liés, un groupe hétérocyclique de 5 ou 6 chaînons contenant éventuellement un hétéroatome supplémentaire choisi parmi l'oxygène, l'azote et le soufre ;
X est -U(CH₂)ᵥ -;
U est un radical bivalent choisi parmi -N(R³⁰)-, -O-, -S(O)_{z}⁻, -N(R³⁰)C(O)-, -C(O)N(R³⁰)- et -N[C(O)R³⁰]- ;
W est -C(R³¹)- ou un atome d'azote ;
d est un entier de 1 à 5 ;
e est un entier de 2 à 4 ;
f, g, h, m, p, q, r, s et w sont chacun indépendamment des entiers de 0 à 4 ;
i est un entier de 1 à 6 ;
j, k, n et z sont chacun indépendamment des entiers de 0 à 2 ;
t est 2 ou 3 ;
v est un entier de 2 à 8 ;
ou dérivé de celui-ci acceptable du point de vue pharmaceutique.

14. Composé de formule (IB) : 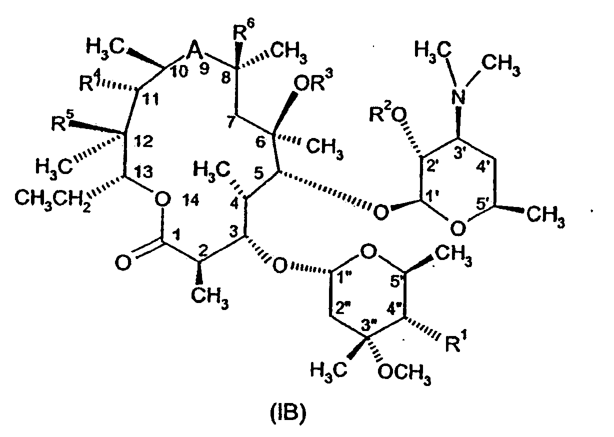 dans laquelle :
A est un radical bivalent choisi parmi -C(O)-, -C(O)NH-, -NHC(O)-, -N(R⁷)-CH₂-, -CH₂-N(R⁷)-, -CH(NR⁸R⁹)- et-C(=NR¹⁰)- ;
R¹ est -OC(O)(CH₂)_{d}XR¹¹ ;
R² est un atome d'hydrogène ou un groupe hydroxy protecteur ;
R³ est un atome d'hydrogène, un groupe alkyle en C₁₋₄, ou alcényle en C₃₋₆ éventuellement substitué par un groupe hétéroaryle bicyclique condensé de 9 à 10 chaînons ;
R⁴ est un groupe hydroxy, alcényloxy en C₃₋₆ éventuellement substitué par un groupe hétéroaryle bicyclique condensé de 9 à 10 chaînons, ou alcoxy en C₁₋₆ éventuellement substitué par un groupe alcoxy en C₁₋₆ ou -O(CH₂)ₑNR⁷R¹²,
R⁵ est un groupe hydroxy, ou bien
R⁴ et R⁵ pris ensemble avec les atomes intermédiaires forment un groupe cyclique ayant la structure suivante : 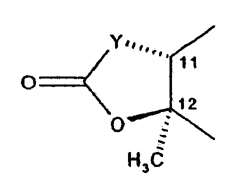 dans laquelle Y est un radical bivalent choisi parmi -CH₂-, -CH(CN), -O-, -N(R¹³)- et -CH(SR¹³)- ;
R⁶ est un atome d'hydrogène ou de fluor ;
R⁷ est un atome d'hydrogène ou un groupe alkyle en C₁₋₆,
R⁸ et R⁹ sont chacun indépendamment un atome d'hydrogène, un groupe alkyle en C₁₋₆, -C(=NR¹⁰)NR¹⁴R¹⁵ ou -C(O)R¹⁴, ou bien
R⁸ et R⁹ forment ensemble =CH(CR¹⁴R¹⁵)_{f}aryle, =CH(CR¹⁴R¹⁵)_{f}hétérocyclyle, =CR¹⁴R¹⁵ ou =C(R¹⁴)C(O)OR¹⁴ dans lesquels les groupes alkyle, aryle et hétérocyclyle sont éventuellement substitués par un maximum de trois groupes choisis indépendamment parmi les groupes R¹⁶ ;
R¹⁰ est -OR¹⁷, alkyle en C₁₋₆, -(CH₂)_{g}aryle, -(CH₂)_{g}hétérocyclyle ou -(CH₂)ₕO(CH₂)ᵢOR⁷ où chaque groupe R¹⁰ est éventuellement substitué par un maximum de trois groupes choisis indépendamment parmi les groupes R¹⁶ ;
R¹¹ est un groupe hétérocyclique ayant la structure suivante : 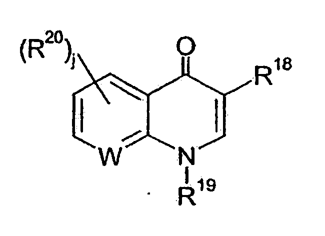 ou 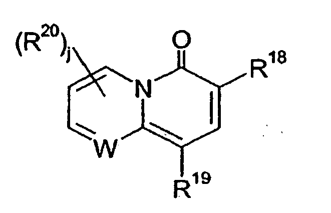
R¹² est un atome d'hydrogène ou un groupe alkyle en C₁₋₆ ;
R¹³ est un atome d'hydrogène ou un groupe alkyle en C₁₋₄ substitué par un groupe choisi parmi un groupe phényle éventuellement substitué, hétéroaryle de 5 ou 6 chaînons éventuellement substitué et hétéroaryle bicyclique condensé de 9 à 10 chaînons éventuellement substitué ;
R¹⁴ et R¹⁵ sont chacun indépendamment un atome d'hydrogène ou un groupe alkyle en C₁₋₆ ;
R¹⁶ est un atome d'halogène, un groupe cyano, nitro, trifluorométhyle, azido, -C(O)R²¹, -C(O)OR²¹, -OC(O)R²¹, -OC(O)OR²¹, -NR²²C(O)R²³, -C(O)NR²²R²³, -NR²²R²³, hydroxy, alkyle en C₁₋₆, -S(O)ₖalkyle en C₁₋₆, alcoxy en C₁₋₆, -(CH₂)ₘaryle ou -(CH₂)ₘhétéroaryle dans lequel le groupe alcoxy est éventuellement substitué par un maximum de trois groupes choisis indépendamment parmi -NR¹⁴R¹⁵, halogène et -OR¹⁴ et les groupes aryle et héréroaryle sont éventuellement substitués par un maximum de cinq groupes choisis indépendamment parmi un atome d'halogène, un groupe cyano, nitro, trifluorométhyle, azido, -C(O)R²⁴, -C(O)OR²⁴, -OC(O)OR²⁴, -NR²⁵C(O)R²⁶, -C(O)NR²⁵R²⁶, -NR²⁵R²⁶, hydroxy, alkyle en C₁₋₆ et alcoxy en C₁₋₆ ;
R¹⁷ est un atome d'hydrogène, un groupe alkyle en C₁₋₆, cycloalkyle en C₃₋₇, alcényle en C₃₋₆ ou un groupe hétérocyclique de 5 ou 6 chaînons, dans lequel les groupes alkyle, cycloalkyle, alcényle et hétérocyclique sont éventuellement substitués par un maximum de trois substituants choisis indépendamment parmi un groupe hétérocyclique de 5 ou 6 chaînons éventuellement substitué, hétéroaryle de 5 ou 6 chaînons éventuellement substitué, -OR²⁷ -S(O)ₙR²⁷, -NR²⁷R²⁸, -CONR²⁷R²⁸, halogène et cyano ;
R¹⁸ est un atome d'hydrogène, -C(O)OR²⁹, -C(O)NHR²⁹ ou -C(O)CH₂NO₂ ;
R¹⁹ est un atome d'hydrogène, un groupe alkyle en C₁₋₄ éventuellement substitué par un groupe hydroxy ou alcoxy en C₁₋₄, cycloalkyle en C₃₋₇, ou benzyle ou phényle éventuellement substitué ;
R²⁰ est un atome d'halogène, un groupe alkyle en C₁₋₄, thioalkyle en C₁₋₄, alcoxy en C₁₋₄, -NH₂, -NH(alkyle en C₁₋₄) ou-N(alkyle en C₁₋₄)₂ ;
R²¹ est un atome d'hydrogène, un groupe alkyle en C₁₋₁₀, -(CH₂)ₚaryle ou -(CH₂)ₚhétéroaryle ;
R 22 et R²³ sont chacun indépendamment un atome d'hydrogène, -OR¹⁴, alkyle en C₁₋₆, -(CH₂)_{q}aryle ou -(CH₂)_{q}hétérocyclyle ;
R²⁴ est un atome d'hydrogène, un groupe alkyle en C₁₋₁₀, -(CH₂)ᵣaryle ou -(CH₂)ᵣhétéroaryle ;
R 25 et R²⁶ sont chacun indépendamment un atome d'hydrogène, -OR¹⁴, alkyle en C₁₋₆, -(CH₂)ₛaryle ou -(CH₂)ₛhétérocyclyle ;
R²⁷ et R²⁸ sont chacun indépendamment un atome d'hydrogène, un groupe alkyle en C₁₋₄ ou alcoxy(en C₁₋₄)alkyle en C₁₋₄ ;
R²⁹ est un atome d'hydrogène ou un groupe alkyle en C₁₋₆ éventuellement substitué par un maximum de trois groupes choisis indépendamment parmi un atome d'halogène, un groupe alcoxy en C₁₋₄, -OC(O)alkyle en C₁₋₆ et -OC(O)Oalkyle en C₁₋₆ ;
R³⁰ est un atome d'hydrogène, un groupe alkyle en C₁₋₄, cycloalkyle en C₃₋₇, benzyle ou phényle éventuellement substitué, acétyle ou benzoyle ;
R³¹ est un atome d'hydrogène ou R²⁰, ou bien R³¹ et R¹⁹ sont reliés pour former le radical bivalent -O(CH₂)₂- ou -(CH₂)ₜ⁻ ;
X est -U(CH₂)ᵥ -;
U est un radical bivalent choisi parmi -N(R³⁰)-, -O-, -S(O)_{z}⁻, -N(R³⁰)C(O)-, -C(O)N(R³⁰)- et -N[C(O)R³⁰]- ;
W est -C(R³¹)- ou un atome d'azote ;
d est un entier de 1 à 5 ;
e est un entier de 2 à 4 ;
f, g, h, m, p, q, r et s sont chacun indépendamment des entiers de 0 à 4 ;
i est un entier de 1 à 6 ;
j, k, n et z sont chacun indépendamment des entiers de 0 à 2 ;
t est 2 ou 3 ;
v est un entier de 2 à 8 ;
ou dérivé de celui-ci acceptable du point de vue pharmaceutique.
